# EUROPEAN PATENT APPLICATION

(11) **EP 4 159 738 A1**
(43) Date of publication of application: **05.04.2023**
(21) Application number: 21813815.4
(22) Date of filing: 21.05.2021
(51) Int. Cl.: C07D 498/08, A61K 31/505, A61P 35/00

(54) **MACROCYCLIC JAK INHIBITOR AND USES THEREOF**

(30) Priority: 27.05.2020 CN 202010460966
(71) Applicant: Biopolar Hongye (Guangdong) Pharmaceutical Co. Ltd, Zhongshan, Guangdong 528437 (CN); Biopolar Hongye (Nantong) Pharmaceutical Co., Ltd, Nantong, Jiangsu 226133 (CN)
(72) Inventor: LV, Zhijian, Nantong, Jiangsu 226133 (CN); LI, Jia, Nantong, Jiangsu 226133 (CN); SU, Mingbo, Nantong, Jiangsu 226133 (CN); GAO, Anhui, Nantong, Jiangsu 226133 (CN)
(74) Representative: Lavoix
(86) International application number: PCT/CN2021/095332
(87) International publication number: WO 2021/238818

(57) **Abstract**

The present invention relates to a macrocyclic JAK inhibitor and the uses thereof. Specifically, the present invention relates to a compound represented by formula I, or a stereoisomer or an optical isomer, a pharmaceutically acceptable salt, or a prodmg or a solvate thereof; and also relates to a pharmaceutical composition of the compound, and the medical uses thereof as a JAK inhibitor and in the preparation of a drug for preventing and/or treating diseases related to JAK, especially JAK3.

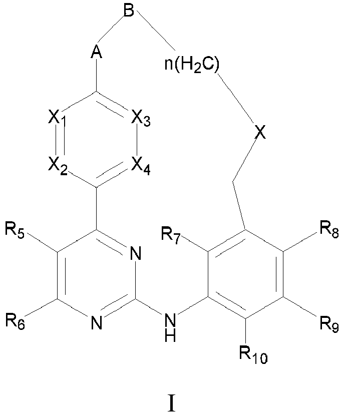

## Description

### TECHNICAL FIELD

The invention belongs to the field of medicinal chemistry, and specifically relates to a macrocyclic JAK inhibitor and use thereof.

### BACKGROUND OF THE INVENTION

Protein kinases (PK) are a group of enzymes that regulate a variety of important biological processes that constitute one of the largest families of enzymes in human. In particular, the biological processes include cellular kinases that catalyze the phosphorylation of proteins, lipids, sugars, nucleosides and other cellular metabolites and play a key role in all aspects of eukaryotic cell physiology. It has been shown that abnormal kinase activity is involved in many human diseases, including cancer, autoimmune diseases and inflammatory diseases.

Janus kinase (JAK) is a cytoplasmic tyrosine kinase that transduces cytokine signals from membrane receptors to STAT transcription factors, and plays an important role in cytokine signaling. The JAK family includes four members JAK1, JAK2, JAK3 and tyrosine kinase 2(TYK2). JAK usually associates with cytokine receptors in pairs as homodimers or heterodimers. Cytokines bind to their receptors, causing dimerization of receptor molecules. Receptor-coupled JAKs approach each other and are activated by phosphorylation of interacting tyrosine residues. JAK family transmits cytokine-mediated signals to cells through JAK-STAT (signal transduction and transcription activation factor) pathway.

Signal Transducer and Activator of Transcription (STAT) are a group of cytoplasmic proteins that can bind to target gene regulatory region DNA. As the downstream substrate of JAKs, STATs can be activated by tyrosine phosphorylation under the stimulation of external signals, and then transferred to nucleus to regulate the transcription of genes. When cytokine bind to its receptor, JAK family members autophosphorylate and/or transphosphorylate each other, followed by STATs phosphorylation, and then migrate into the nucleus to regulate transcription.

Many abnormal immune responses, such as allergies, asthma, (allogeneic) transplant rejection, rheumatoid arthritis, amyotrophic lateral sclerosis and multiple sclerosis and other autoimmune diseases, myelodysplasia, hematologic malignancies such as leukemia and lymphoma, have their regulation associated with the JAK/STAT signaling pathway.

Studies have shown that blocking signal transduction at the level of JAK kinase provides prospects for the development of therapeutic methods for inflammatory diseases, autoimmune diseases, myeloproliferative diseases and cancer. The inhibition of JAK kinase also contributes to the treatment of skin immune diseases such as psoriasis and skin sensitization. Pfizer's Toficitinib has been marketed for the treatment of rheumatoid arthritis; and Incyte's Ruxolitinib for the treatment of myelofibrosis and acute graft-versus-host disease.

However, some of the currently available JAK kinase inhibitors also have some significant toxic side effects. For example, some JAK inhibitors are prone to the following side effects: infections, including pneumonia, viral infections (such as herpes zoster infection), bacterial infections, actinomycotic infections (mycobacterial infections), fungal infections, decreased immunity (such as NK cell reduction), and anemia. In the United States, there are even black box warnings for some serious side effects, such as acute tuberculosis, invasive fungal infections, bacterial infections, and some lymphoma or other tumors. Studies have shown that the existing JAK inhibitors often have inhibitory activity on JAK1 and JAK3. Unlike other JAKs that are widely expressed, JAK3 is only expressed in the hematopoietic system, so it is generally believed that selective inhibition of JAK3 can achieve safe and effective immune effects.

However, studies have shown that JAKs family kinases are responsible for regulating many signaling pathways. Since JAK1 and JAK3 are components of common γ-chain cytokine receptor complexes, it is very difficult to develop inhibitors with high selectivity to JAK3.

Therefore, there is an urgent need in the art to develop inhibitors of Janus kinase or related kinases, especially inhibitors with high selectivity to JAK3.

### SUMMARY OF THE INVENTION

The invention provides an inhibitor of JAK or related kinases, especially an inhibitor with high selectivity to JAK3.

In the first aspect of the present invention, it provides a compound of formula I or a stereoisomer or an optical isomer, a pharmaceutically acceptable salt, a prodrug or a solvate thereof, 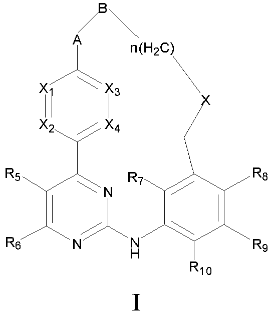 wherein,
X₁, X₂, X₃ and X₄ are each independently selected from the group consisting of N, C and C-R_{d}; and 0, 1, 2 and 3 of X₁, X₂, X₃ and X₄ are N;
or, when X₄ is C-R_{d}, R_{d} is fused with X₂ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or, when X₂ is C-R_{d}, R_{d} is fused with X₁ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or, when X₃ is C-R_{d}, R_{d} is fused with X₄ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or, when X₄ is C-R_{d}, R_{d} is fused with X₃ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
A is selected from the group consisting of -C(=O)N-R_{b}, -C(=O)O-, -SO₂N(R_{b})-, -C(= O)N-R_{b}-SO₂-, O and S; wherein R_{b} is selected from the group consisting of H, and substituted or unsubstituted C1-C6 alkyl;
B is selected from the substituted or unsubstituted group consisting of bond, C(R_{c})₂, C3-C10 cycloalkylene, 3-10-membered heterocyclylene, 5-12-membered heteroarylene, C6-C12 arylene, and (CH₂)m-R'-; R_{c} is each independently selected from the group consisting of H, halogen, amino, nitro, hydroxyl, cyano, carboxyl, sulfonyl, sulfinyl, amido, sulfonamido, ester group, C1-C6 alkyl, C1-C6 alkoxy, 3-10 membered heterocycloalkyl, C3-C10 cycloalkyl, 5-12 membered heteroaryl, and C6-C12 aryl; R' is independently selected from the substituted or unsubstituted group consisting of 5-12 membered heteroarylene, and C6-C12 arylene;
X is selected from the substituted or unsubstituted group consisting of bond, C(R_{c})₂, (CH₂)m-O-, C(= O)O-, O, N-R_{b}, S, SO, SO₂, C3-C10 cycloalkylene, 3-10-membered heterocyclylene, 5-12-membered heteroarylene, and C6-C12 arylene;
R₅, R₆, R₇, R₈, R₉, R₁₀ and R_{d} are each independently selected from the group consisting of H, D, halogen, amino, amine, nitro, hydroxyl, sulfydryl, cyano, carboxyl, sulfonyl, sulfinyl, amido, sulfonamido, ester group, formyl, formamido, C1-C6 alkyl, C2-C6 alkenyl, C2-C6 alkynyl, 3-10-membered heterocyclyl, C3-C10 cycloalkyl, 5-12-membered heteroaryl, C6-C12 aryl and -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
or R₅ and R₆ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₈ and R₉ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₉ and R₁₀ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
the H atom in (CH₂)n and (CH₂)m can be optionally substituted by one or more (e. g. 2, 3, 4, 5) Rₐ;
m is 1, 2, 3, 4 or 5;
n is 0, 1, 2, 3, 4, 5, 6, 7 or 8;
the "substituted" refers to being substituted by one or more (such as 2, 3, 4, 5) groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more (such as 2, 3, 4, 5) Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, the compound of formula I or the stereoisomer or optical isomer, the pharmaceutically acceptable salt, the prodrug or the solvate thereof has a structure shown in formula I': 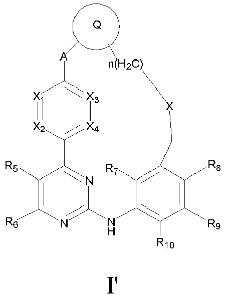 wherein,
Q is selected from the substituted or unsubstituted group consisting of (CH₂)m-R'-, C3-C10 cycloalkylene, 3-10-membered heterocyclylene, 5-12-membered heteroarylene, and C6-C12 arylene; wherein R' is independently selected from the substituted or unsubstituted group consisting of 5-12 membered heteroarylene, and C6-C12 arylene;
the "substituted" refers to being substituted by one or more (such as 2, 3, 4, 5) groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
X, m, n, A, X₁, X₂, X₃, X₄, R₅, R₆, R₇, R₈, R₉, and R₁₀ are defined as above.

In another preferred embodiment, the compound or the stereoisomer or optical isomer, pharmaceutically acceptable salt, prodrug or solvate thereof, X₂ is C-R_{d}, and R_{d} is selected from the group consisting of H, amino, amine, C1-C6 alkyl, hydroxyl, and sulfydryl, the alkyl can be optionally substituted by one or more groups selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, the compound of formula I or the stereoisomer or the optical isomer, the pharmaceutically acceptable salt, the prodrug or the solvate thereof has a structure shown in formula II: 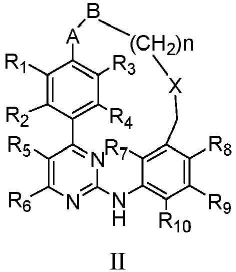 wherein,
R₁, R₂, R₃, R₄, R₅, R₆, R₇, R₈, R₉ and R₁₀ are each independently selected from the group consisting of H, D, halogen, amino, amine, nitro, hydroxyl, sulfydryl, cyano, carboxyl, sulfonyl, sulfinyl, amido, sulfonamido, ester group, formyl, formamido, C1-C6 alkyl, C2-C6 alkenyl, C2-C6 alkynyl, 3-10-membered heterocyclyl, C3-C10 cycloalkyl, 5-12-membered heteroaryl, C6-C12 aryl and -OR₁₁; Wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
or R₁ and R₂ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₃ and R₄ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₅ and R₆ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₈ and R₉ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₉ and R₁₀ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
the H atom in (CH₂)m and (CH₂)n can be optionally substituted by one or more Rₐ;
A, B, m, n and X are defined as above;
the "substituted" refers to being substituted by one or more (such as 2, 3, 4, 5) groups selected from the group consisting of D, halogen, amino, amine, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more (such as 2, 3, 4, 5) Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, the compound of formula I or the stereoisomer or the optical isomer, the pharmaceutically acceptable salt, the prodrug or the solvate thereof has a structure shown in formula III: 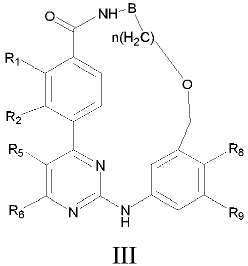 wherein,
B is selected from the substituted or unsubstituted group consisting of bond, (CH₂)m-R'-, C3-C10 cycloalkylene, 3-10-membered heterocyclylene, 5-12-membered heteroarylene, and C6-C12 arylene; wherein R' is independently selected from the substituted or unsubstituted group consisting of 5-12 membered heteroarylene, and C6-C12 arylene;
the "substituted" refers to being substituted by one or more (such as 2, 3, 4, 5) groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
m, n, R₅, R₆, R₈, R₉, R₁, and R₂ are defined as above.

In another preferred embodiment, B is selected from the substituted or unsubstituted group consisting of 5-12-membered heteroarylene, and C6-C12 arylene; wherein, the "substituted" refers to being substituted by one or more (such as 2, 3, 4, 5) groups selected from the group consisting of D, halogen, amino, amine, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl; n is 0 or 1.

In another preferred embodiment, A is selected from the group consisting of -C(=O)N-R_{b}, -C(= O)O-, and -SO₂N(R_{b})-, wherein, R_{b} is selected from the group consisting of H, and substituted or unsubstituted C1-C6 alkyl, wherein, the "substituted" refers to being substituted by one or more (such as 2, 3, 4, 5) groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

In another preferred embodiment, A is -C(= O)N-R_{b}, wherein, R_{b} is selected from the group consisting of H, and substituted or unsubstituted C1-C6 alkyl, wherein, the "substituted" refers to being substituted by one or more (such as 2, 3, 4, 5) groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

In another preferred embodiment, when A is -C(= O)N-R_{b}, the carbonyl moiety is connected to 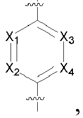 wherein R_{b} is defined as above.

In another preferred embodiment, the compound of formula I or the stereoisomer or optical isomer, the pharmaceutically acceptable salt, the prodrug or the solvate thereof has a structure shown in formula IV 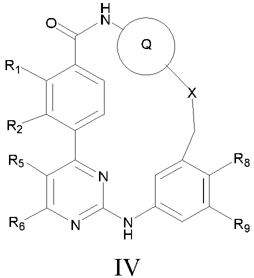 wherein,
Q is selected from the substituted or unsubstituted group consisting of (CH₂)m-R'-, 5-12 membered heteroarylene and C6-C12 arylene; wherein R' is independently selected from the substituted or unsubstituted group consisting of 5-12 membered heteroarylene, and C6-C12 arylene; the "substituted" refers to being substituted by one or more (such as 2, 3, 4, 5) groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
X, m, R₁, R₂, R₅, R₆, R₈, and R₉ are defined as above.

In another preferred embodiment, the compound of formula I or the stereoisomer or optical isomer, the pharmaceutically acceptable salt, the prodrug or the solvate thereof has a structure shown in formula V 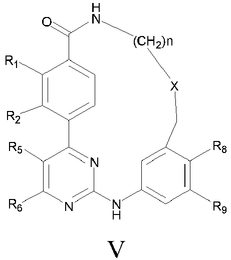 wherein, R₅, R₆, R₈, R₉, X, n, R₁, and R₂ are defined as above.

In another preferred embodiment, X is selected from the group consisting of bond, C(R_{c})₂, (CH₂)m-O-, C(= O)O-, O, N-R_{b}, S, SO, and SO₂; R_{c}, R_{b} and m are defined as above.

In another preferred embodiment, X is C(R_{c})₂, O or S, wherein R_{c} is defined as above.

In another preferred embodiment, the compound of formula I or the stereoisomer or optical isomer, pharmaceutically acceptable salt, prodrug or solvate thereof has a structure of formula VI 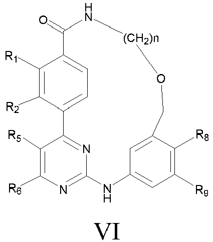 wherein, R₁, R₂, R₅, R₆, R₈, R₉ and n are defined as above.

In another preferred embodiment, the compound of formula I or the stereoisomer or optical isomer, the pharmaceutically acceptable salt, the prodrug or the solvate thereof has a structure shown in formula VII 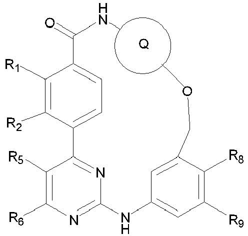 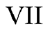 wherein, R₁, R₂, R₅, R₆, R₈, R₉ and Q are defined as above.

In another preferred embodiment, the compound or the stereoisomer or optical isomer, pharmaceutically acceptable salt, prodrug or solvate thereof, R₂ is selected from the group consisting of H, amino, amine, C1-C6 alkyl, hydroxyl, and sulfydryl, the alkyl can be optionally substituted by one or more (such as 2, 3, 4, 5) groups selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, the compound of formula I or the stereoisomer or optical isomer, the pharmaceutically acceptable salt, the prodrug or the solvate thereof has a structure shown in formula VIII 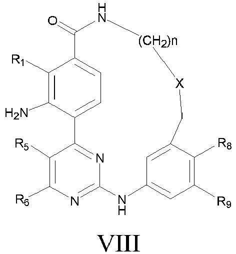 wherein, R₁, R₅, R₆, R₈, R₉, X and n are defined as above.

In another preferred embodiment, the compound or the stereoisomer or optical isomer, pharmaceutically acceptable salt, prodrug or solvate thereof, R₈ is selected from the group consisting of H, halogen, amino, amine, C1-C6 alkyl, C2-C6 alkenyl, C2-C6 alkynyl, 3-10 membered heterocyclyl, C3-C10 cycloalkyl, 5-12 membered heteroaryl, C6-C12 aryl and -OR₁₁;
wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more (such as 2, 3, 4, 5) groups selected from the group consisting of D, halogen, amino, amine, hydroxyl, cyano, amido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
the above alkyl, alkenyl, alkynyl, heterocyclyl, cycloalkyl, heteroaryl, and aryl may be further optionally substituted by one or more (such as 2, 3, 4, 5) Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

In another preferred embodiment, R₈ is selected from the group consisting of C1-C6 alkyl, C3-C10 cycloalkyl, 3-10 membered heterocyclyl, and -OR₁₁;
R₁₁ is selected from the substituted or unsubstituted group consisting of C1-C6 alkyl, 3-10 membered heterocyclyl, and C3-C10 cycloalkyl;
the "substituted" refers to being substituted by one or more (such as 2, 3, 4, 5) groups selected from the group consisting of D, halogen, amino, amine, hydroxyl, cyano, amido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
wherein, the above alkyl, alkenyl, alkynyl, heterocyclyl, cycloalkyl, heteroaryl, and aryl may be further optionally substituted by one or more (such as 2, 3, 4, 5) Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

In another preferred embodiment, the compound of formula I or the stereoisomer or optical isomer, the pharmaceutically acceptable salt, the prodrug or the solvate thereof has a structure shown in formula IX 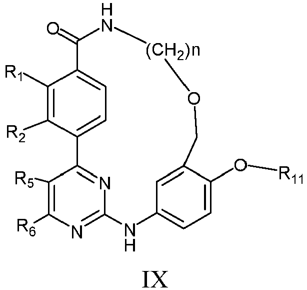
wherein, R₁, R₂, R₅ and R₆ are each independently selected from H, halogen, OH, amino, amine, C1-C3 alkyl, halogenated C1-C3 alkyl, C1-C3 alkoxy, halogenated C1-C3 alkoxy, 3-6-membered heterocyclyl, or C3-C6 cycloalkyl;
R₁₁ and n are defined as above.

In another preferred embodiment, R₁₁ is selected from the substituted or unsubstituted group consisting of C1-C3 alkyl, 3-6-membered heterocyclyl, C3-C6 cycloalkyl, 5-6-membered heteroaryl, and phenyl;
the "substituted" refers to being substituted by one or more groups selected from the group consisting of D, halogen, amino, amine, hydroxyl, cyano, amido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl; wherein, the alkyl, alkenyl, alkynyl, heterocyclyl, cycloalkyl, heteroaryl, and aryl may be further optionally substituted by one or more (such as 2, 3, 4, 5) Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

In another preferred embodiment, the compound of formula I or the stereoisomer or optical isomer, the pharmaceutically acceptable salt, the prodrug or the solvate thereof has a structure shown in formula X 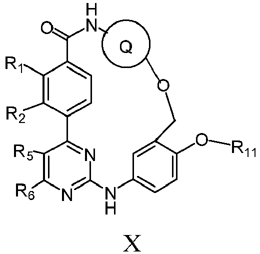
wherein, R₁, R₂, R₅ and R₆ are each independently selected from H, halogen, OH, amino, amine, C1-C3 alkyl, halogenated C1-C3 alkyl, C1-C3 alkoxy, halogenated C1-C3 alkoxy, 3-6-membered heterocyclyl, or C3-C6 cycloalkyl;
R₁₁ and Q are defined as above.

In another preferred embodiment, Q is the substituted or unsubstituted group consisting of phenylene, -phenylene CH₂-, -5-6-membered heteroarylene, and -5-6-membered heteroarylene CH₂-; the "substituted" refers to being substituted by one or more groups selected from the group consisting of D, halogen, amino, amine, hydroxyl, cyano, amido, C1-C6 alkyl, and C1-C6 alkoxy.

In another preferred embodiment, the compound or the stereoisomer or optical isomer, pharmaceutically acceptable salt, prodrug or solvate thereof, R₉ is selected from the group consisting of H, C1-C6 alkyl, C1-C6 alkoxy, 3-10 membered heterocyclyl, and C3-C10 cycloalkyl; wherein, the alkyl, alkoxy, heterocycloalkyl, and cycloalkyl may be further optionally substituted by one or more (such as 2, 3, 4, 5) Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, R₁ is selected from the group consisting of H, and halogen.

In another preferred embodiment, B is bond.

In another preferred embodiment, R₇ is H.

In another preferred embodiment, R₃ is H.

In another preferred embodiment, R₄ is H.

In another preferred embodiment, R₅ is H, halogen, cyano, C1-C6 alkyl, C2-C6 alkenyl, C2-C6 alkynyl, C1-C6 alkoxy, 3-10 membered heterocyclyl, or C3-C10 cycloalkyl, the alkyl, alkoxy, alkenyl, alkynyl, heterocycloalkyl, cycloalkyl, heteroaryl, and aryl may be further optionally substituted by one or more (such as 2, 3, 4, 5) Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, R₆ is H.

In another preferred embodiment, R₁₀ is H.

In another preferred embodiment, the compound of formula I or the stereoisomer or optical isomer, pharmaceutically acceptable salt, prodrug or solvate thereof has one or more characteristics selected from the group consisting of:
A is selected from the group consisting of -C(= O)N-R_{b}, -C(= O)O-, and -SO₂N(R_{b})-, preferably -C(= O)N-R_{b};
B is selected from the substituted or unsubstituted group consisting of bond, 5-12-membered heteroarylene, C6-C12 arylene, and (CH₂)m-R'-; R' is independently selected from the substituted or unsubstituted group consisting of 5-12 membered heteroarylene, and C6-C12 arylene; the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
X₁, X₂, X₃ and X₄ are each independently N or C-R_{d}; preferably, X₂ is CH, C-NH₂, C-N(R_{b})₂, C-OH, C-SH; preferably, X₁ is CH, C-halogen; preferably, X₃ is CH; preferably, X₄ is CH;
R₅ is H, halogen, cyano, C1-C6 alkyl, C2-C6 alkenyl, C2-C6 alkynyl, C1-C6 alkoxy, 3-10 membered heterocyclyl, and C3-C10 cycloalkyl,
R₆ is H;
R₇ is H;
R₈ is selected from the group consisting of H, halogen, amino, amine, C1-C6 alkyl, C2-C6 alkenyl, C2-C6 alkynyl, 3-10 membered heterocyclyl, C3-C10 cycloalkyl, 5-12 membered heteroaryl, C6-C12 aryl and -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
R₉ is selected from the group consisting of H, C1-C6 alkyl, C1-C6 alkoxy, 3-10 membered heterocyclyl, and C3-C10 cycloalkyl;
R₁₀ is H;
X is C(R_{c})₂, (CH₂)m-O-, O or S;
n is 0, 1, 2, 3, 4 or 5;
R_{b} is selected from the group consisting of H, and substituted or unsubstituted C1-C6 alkyl;
each R_{d} is independently selected from the group consisting of H, D, halogen, amino, amine, nitro, hydroxyl, sulfydryl, cyano, carboxyl, sulfonyl, sulfinyl, amido, sulfonamido, ester group, formyl, formamido, C1-C6 alkyl, C2-C6 alkenyl, C2-C6 alkynyl, 3-10-membered heterocyclyl, C3-C10 cycloalkyl, 5-12-membered heteroaryl, C6-C12 aryl and -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, X, n, A, B, X₁, X₂, X₃, X₄, R₅, R₆, R₇, R₈, R₉, and R₁₀ are the specific groups corresponding to each specific compound in the examples.

In another preferred embodiment, the compound or the stereoisomer or optical isomer, pharmaceutically acceptable salt, prodrug or solvate thereof, and the compound is selected from the group consisting of

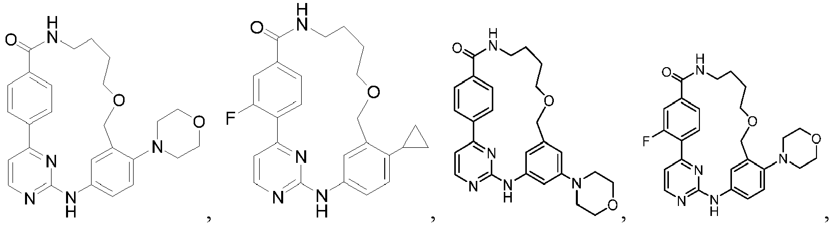

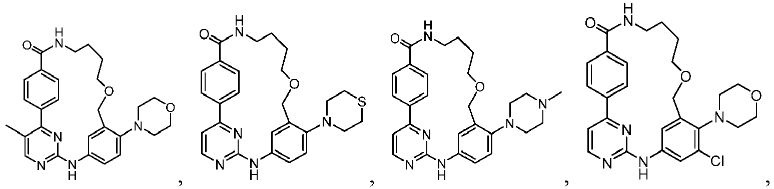

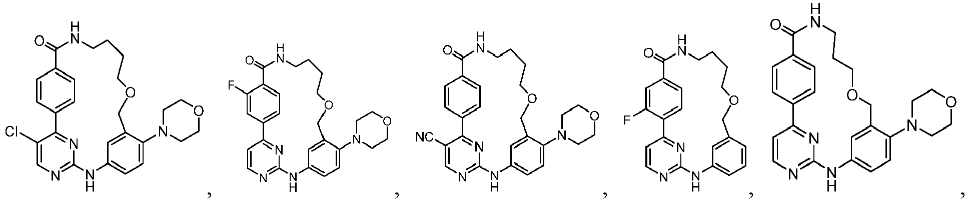

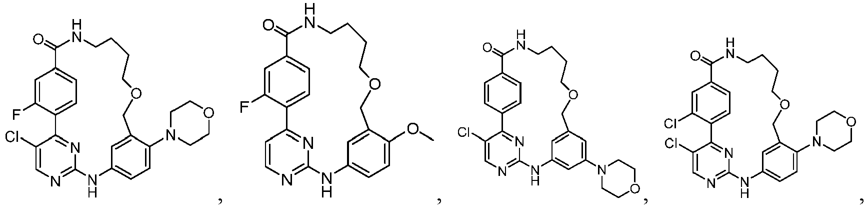

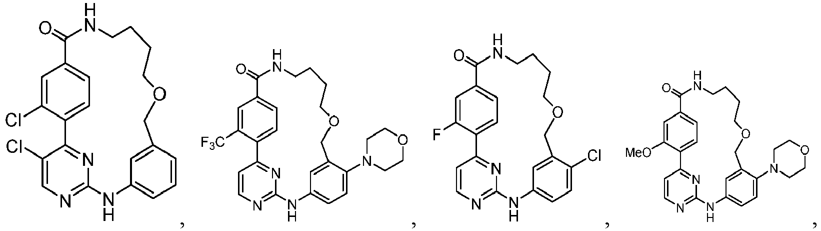

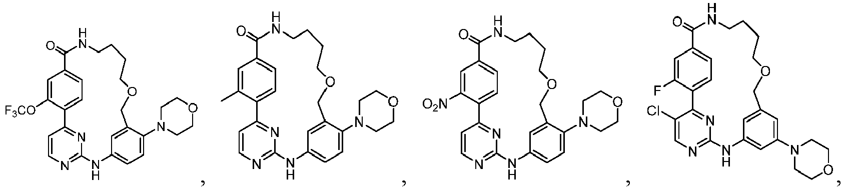

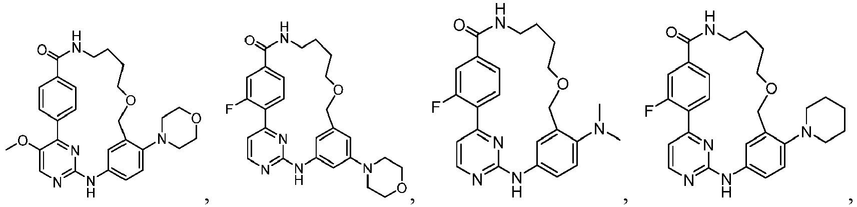

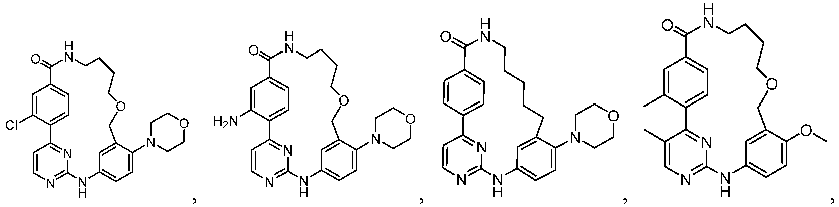

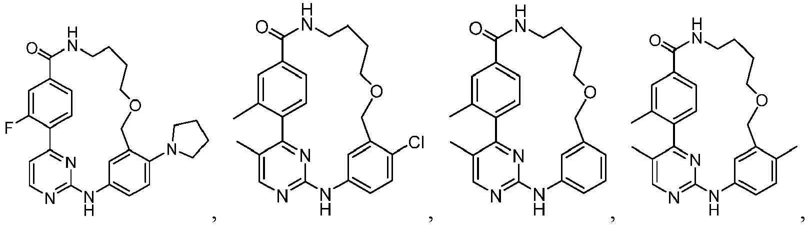

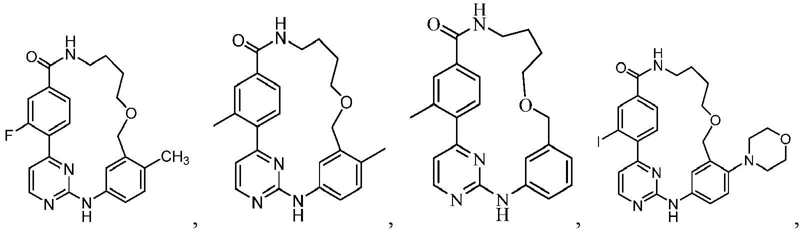

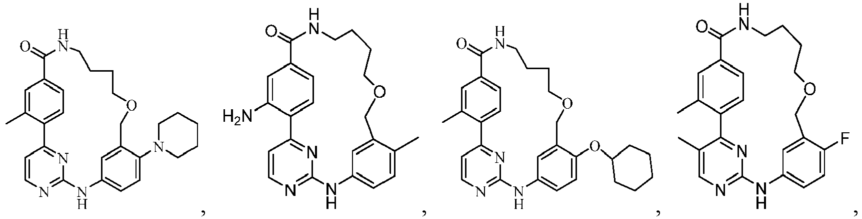

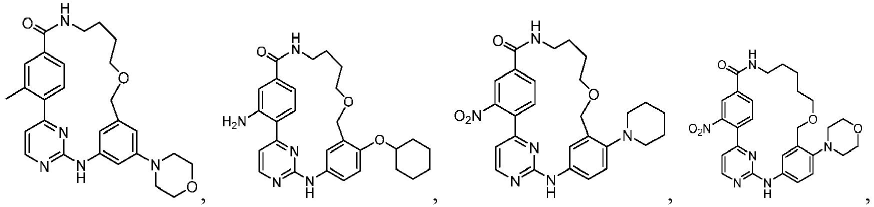

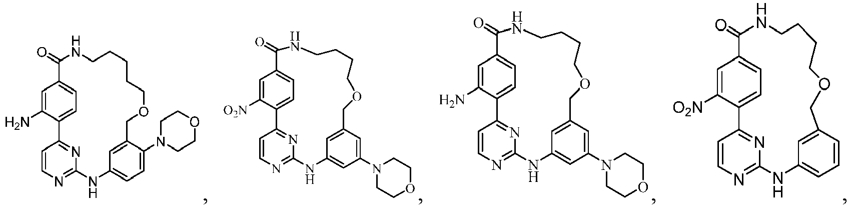

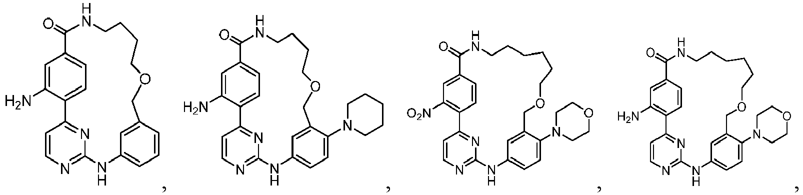

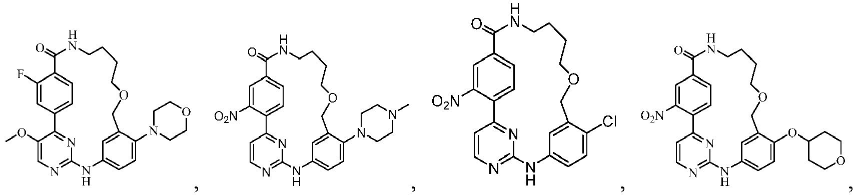

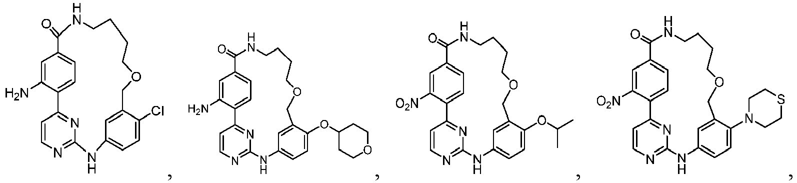

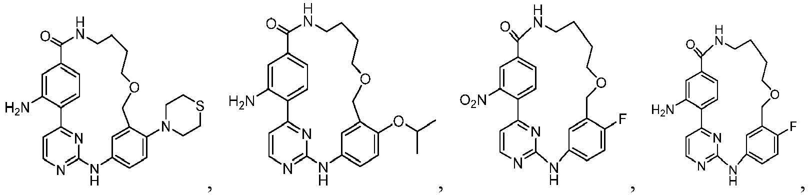

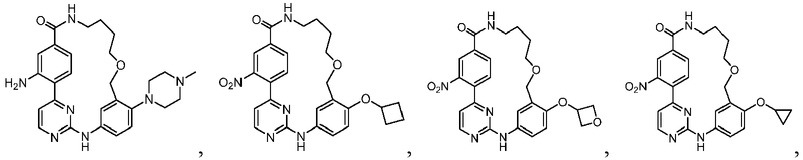

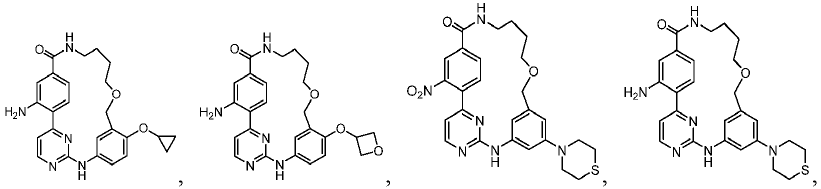

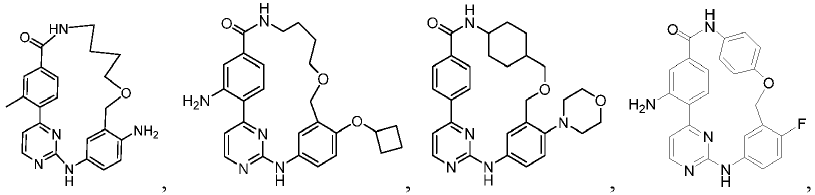

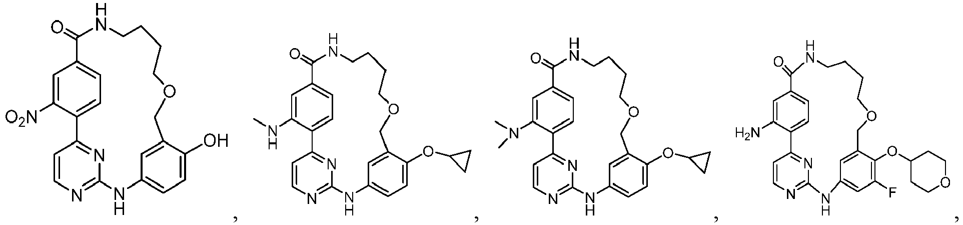

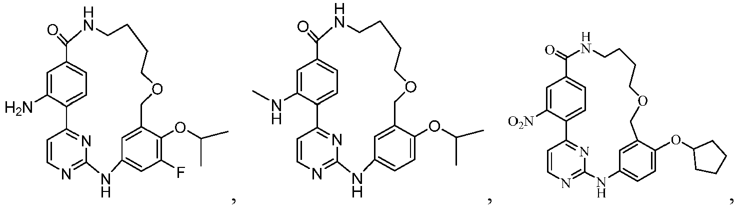

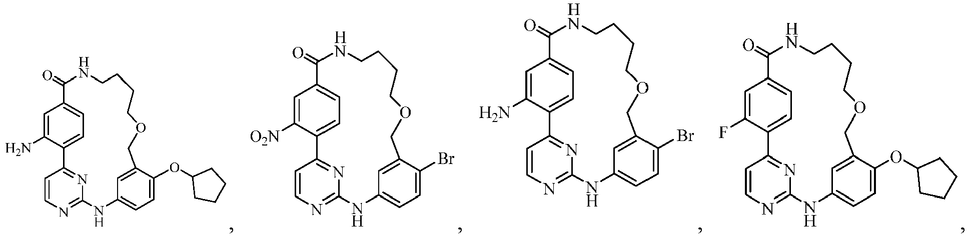

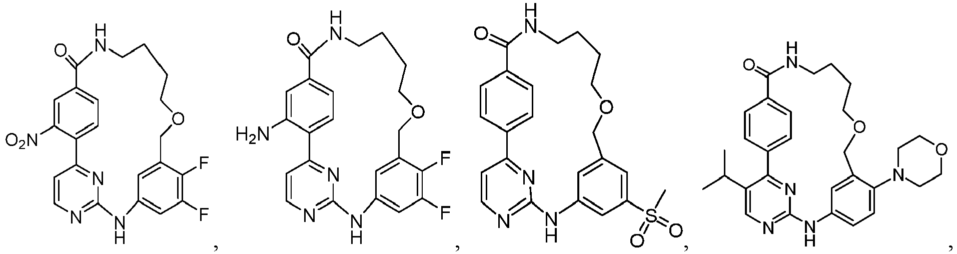

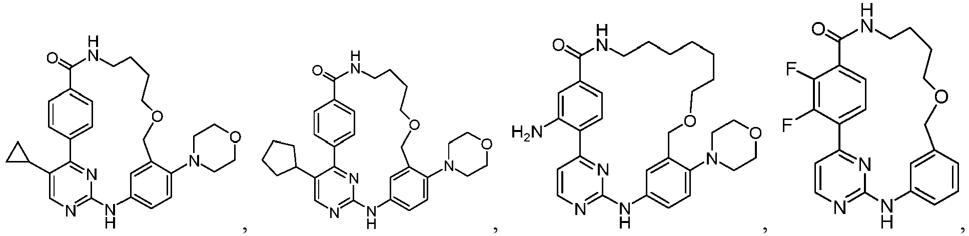

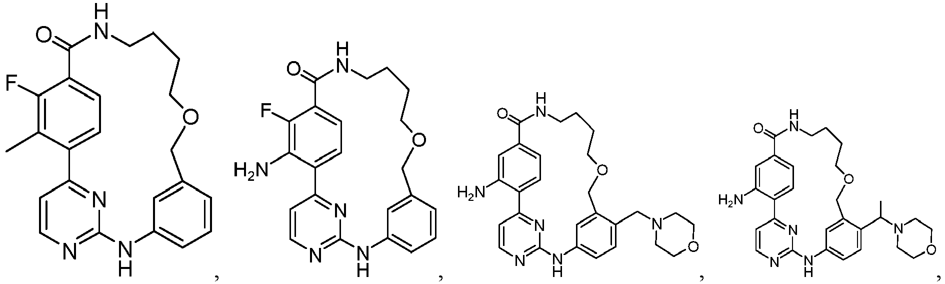

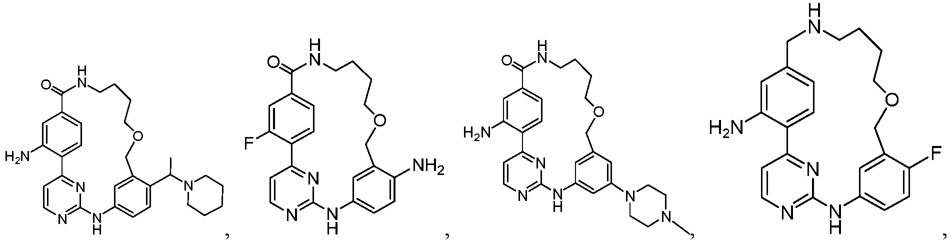

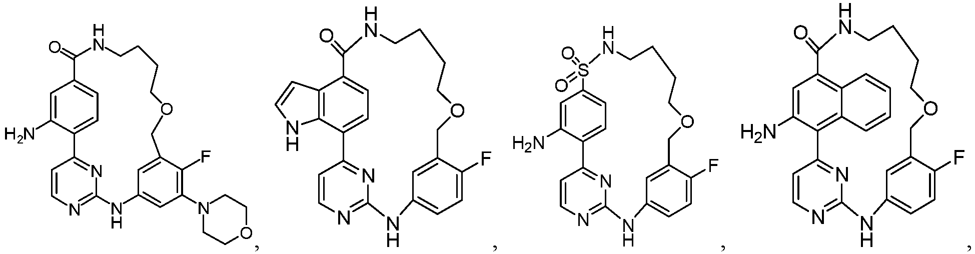

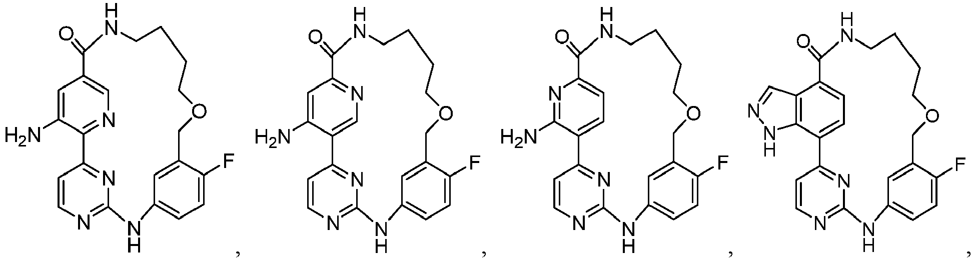

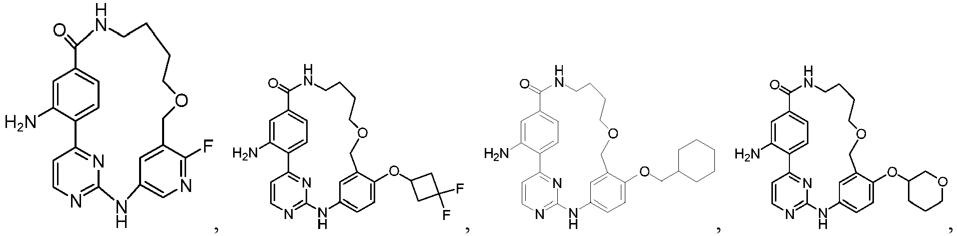

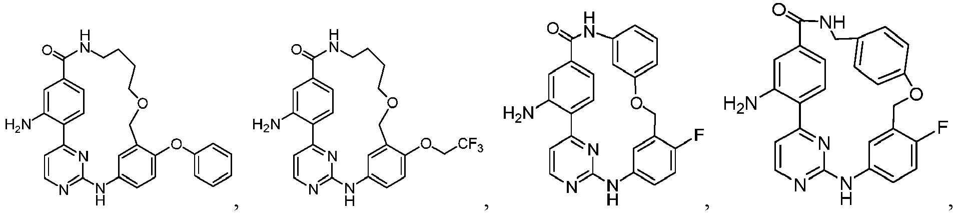

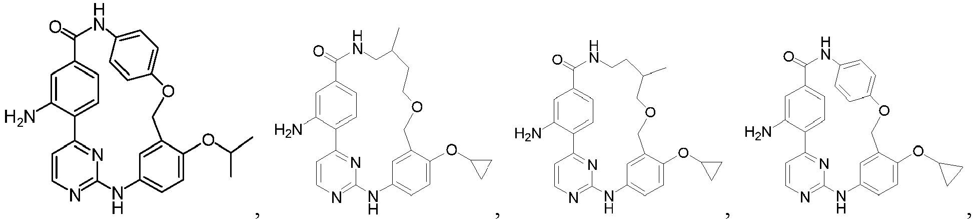

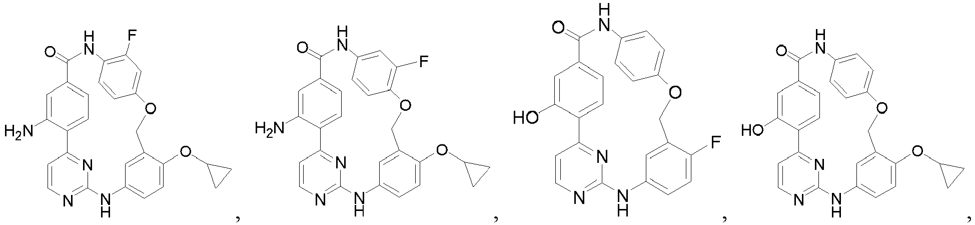

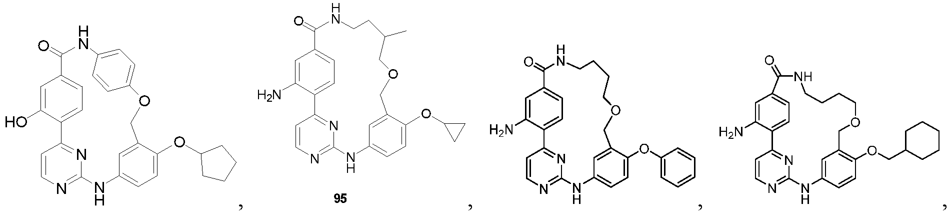

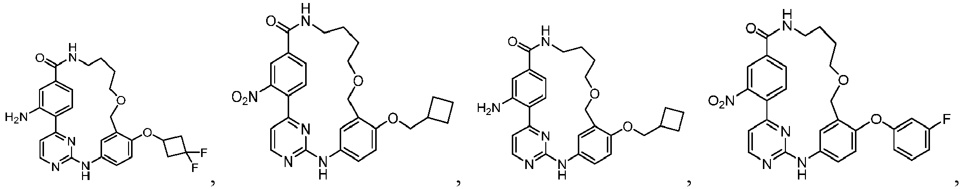

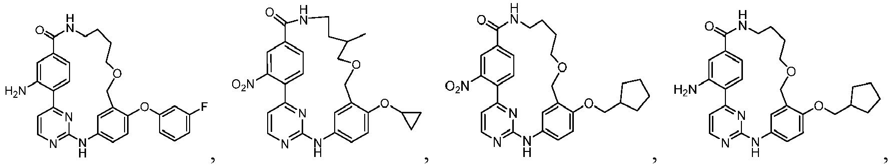

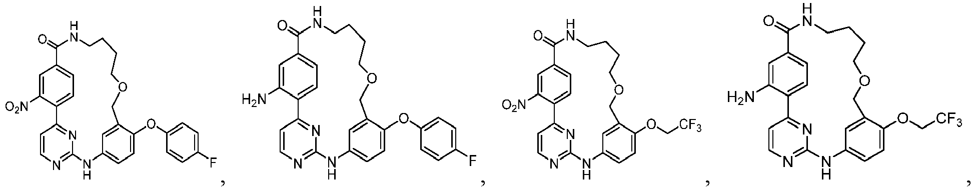

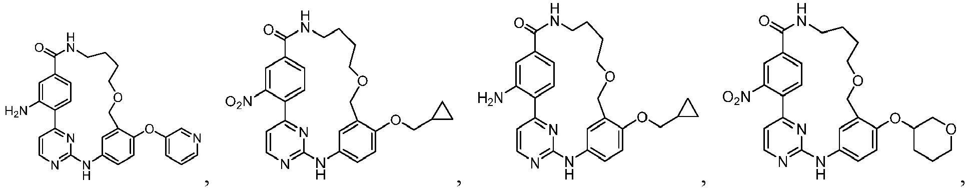

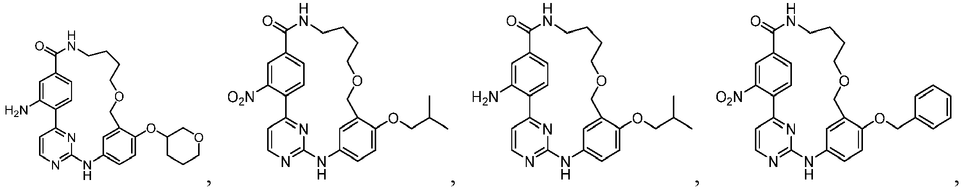

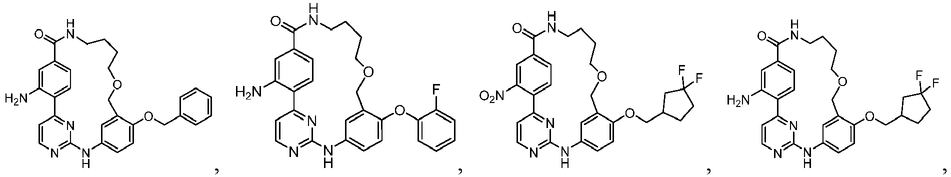

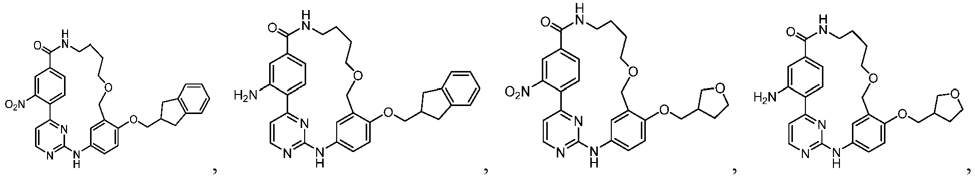

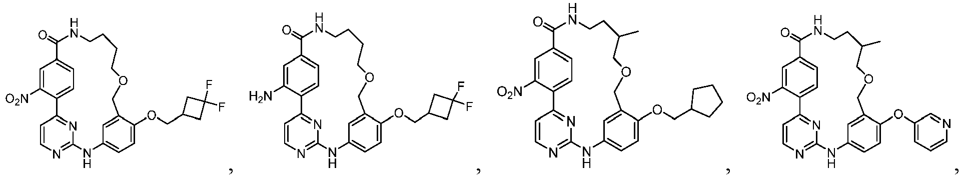

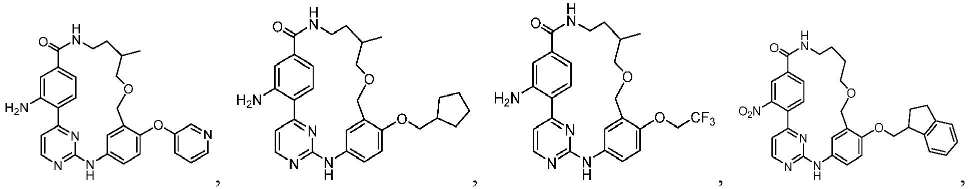

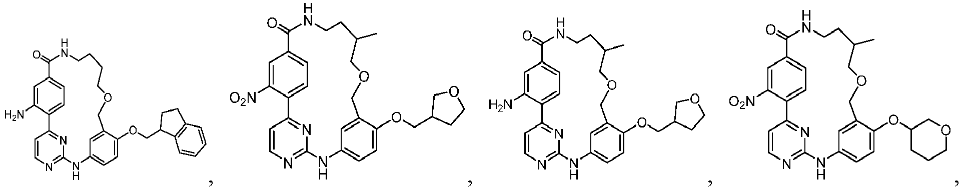

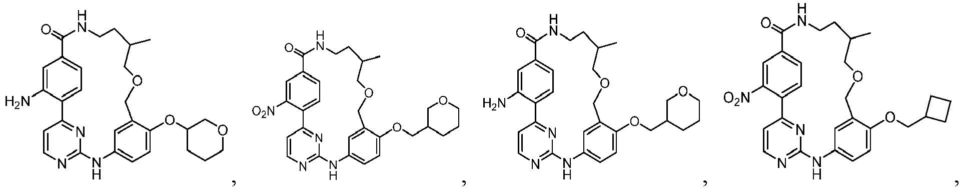

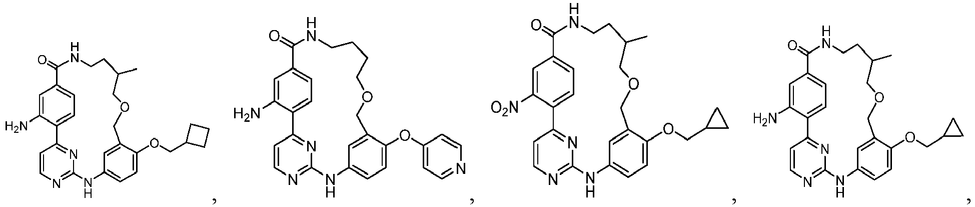

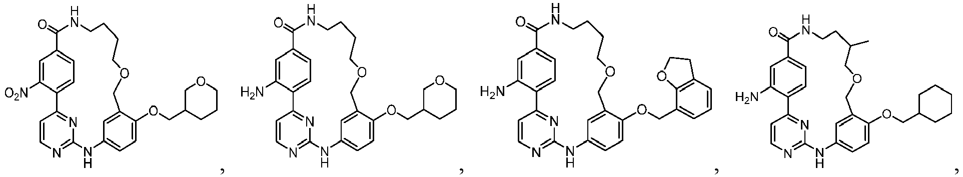

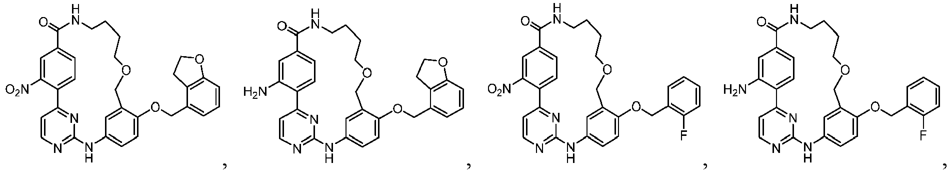

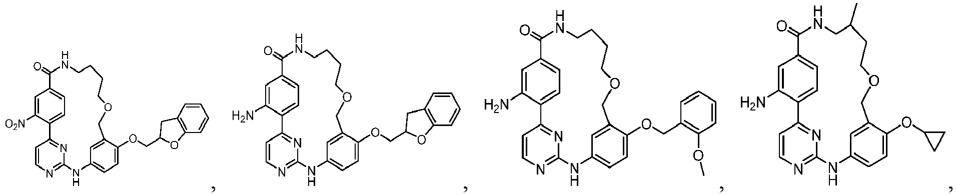

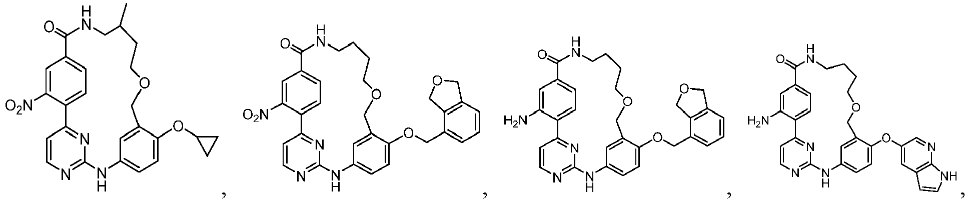

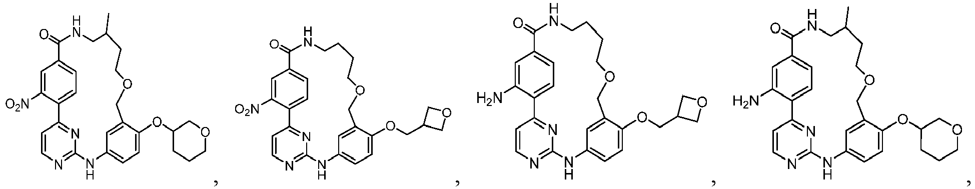

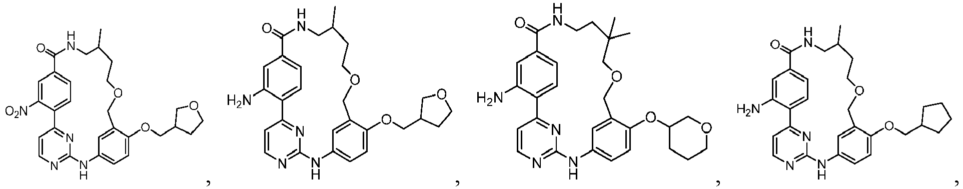

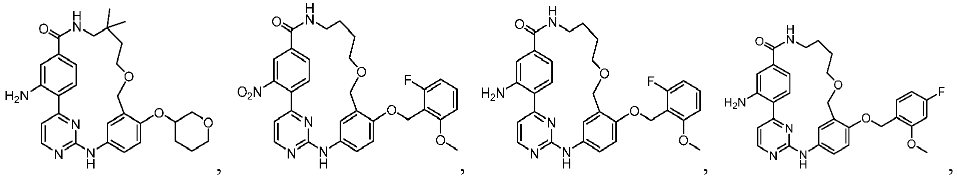

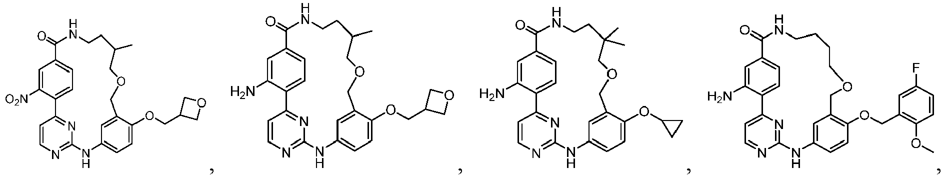

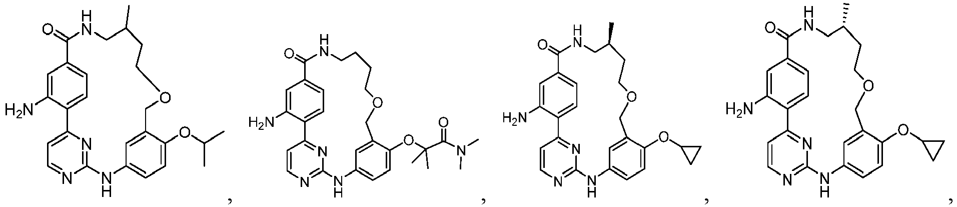

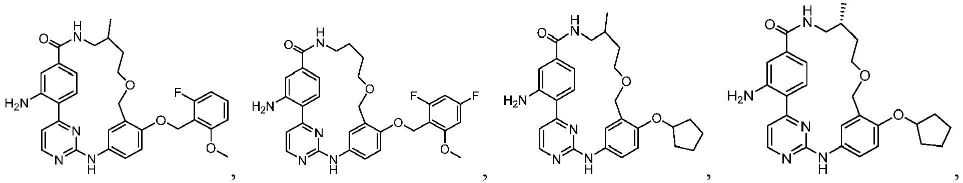

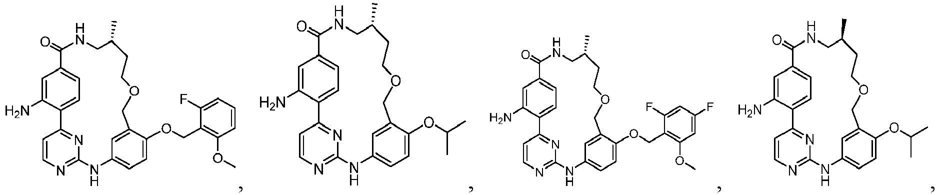

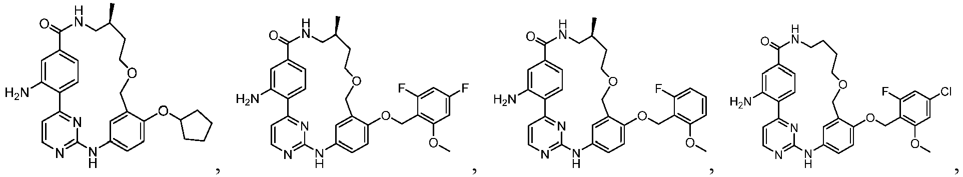

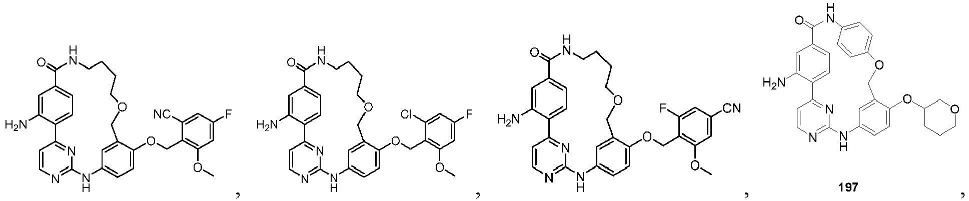

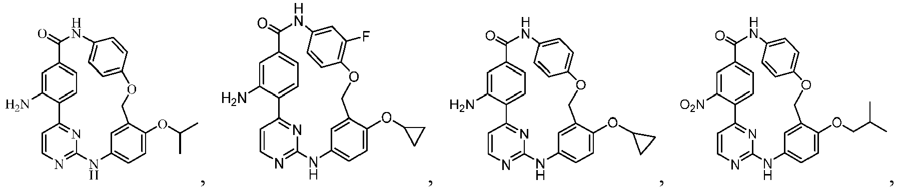

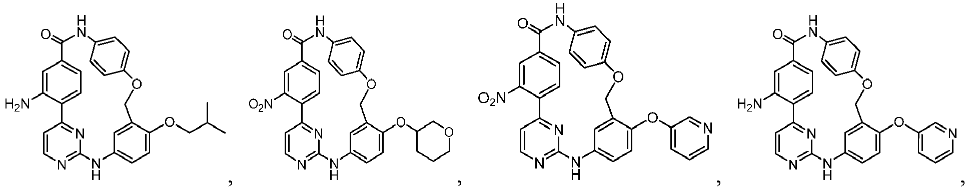

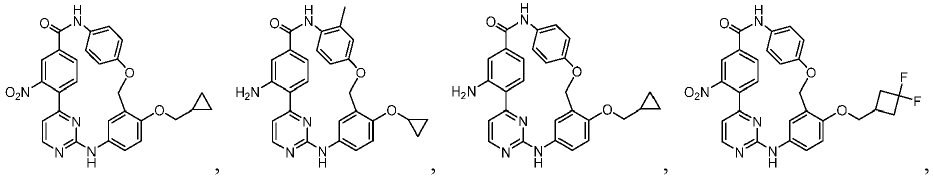

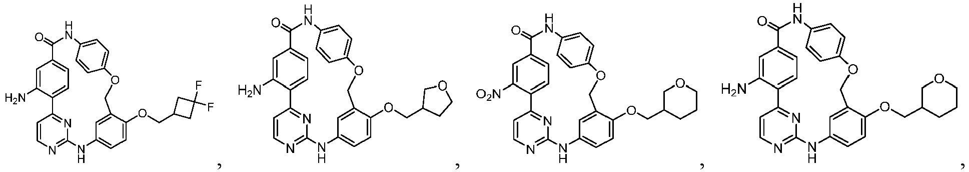

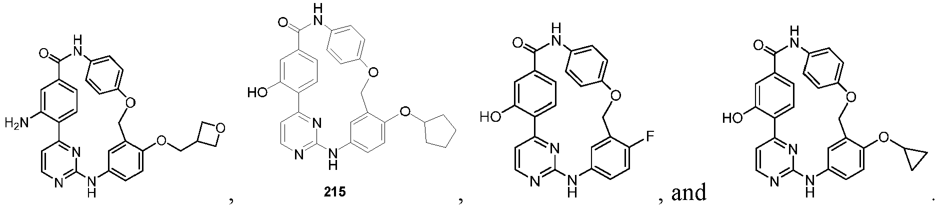

In another preferred embodiment, the compound or the stereoisomer or optical isomer, pharmaceutically acceptable salt, prodrug or solvate thereof is selected from the compound shown in the Examples.

In the second aspect of the invention, it provides a pharmaceutical composition comprising the compound or the stereoisomer or optical isomer, pharmaceutically acceptable salt, prodrug or solvate thereof of the first aspect, and a pharmaceutically acceptable carrier.

In another preferred embodiment, the pharmaceutical composition further comprises a drug selected from the group consisting of
PD-1 inhibitor (e. g., nivolumab, pimumab, pidilizumab, cemiplimab, JS-001, SHR-120, BGB-A317, IBI-308, GLS-010, GB-226, STW204, HX008, HLX10, BAT1306, AK105, LZM 009 or the biological analogue thereof, etc.), PD-L1 inhibitor (e. g, dulvalumab, atezumab, avelumab, CS1001, KN035, HLX20, SHR-1316, BGB-A333, JS003, CS1003, KL -A167, F 520, GR1405, MSB2311 or the biological analogue thereof, etc.), CD20 antibody (e. g, rituximab, obinutuzumab, ofatumumab, veltuzumab, tositumomab, 1311-tositumomab, ibritumomab tiuxetan, 90Y-ibritumomab tiuxetan, 90In-ibritumomab tiuxetan, ibritumomab tiuxetan, etc.), CD47 antibody (e. g, Hu5F9-G4, CC-90002, TTI-621, TTI-622, OSE-172, SRF-231, ALX-148, NI-1701, SHR-1603, IBI188, IMM01), ALK inhibitor (e. g, Ceritinib, Alectinib, Brigatinib, Lorlatinib, Ocatinib), PI3K inhibitors (e. g, Idelalisib, Duvelisib, Dactolisib, Taselisib, Bimiralisib, Omipalisib, Buparlisib, etc.), BTK inhibitor (e. g, ibrutinib, Tirabrutinib, Acalabrutinib, Zanubrutinib, Vecabrutinib, etc.), EGFR inhibitor (e. g, Afatinib, Gefitinib, Erlotinib , Lapatinib, Dacomitinib, Icotinib, Canertinib, Sapitinib, Naquotinib, Pyrotinib, Rociletinib, Osimertinib, etc.), VEGFR inhibitor (e. g, Sorafenib, Pazopanib, Regorafenib, Sitravatinib, Ningetinib, Cabozantinib, Sunitinib, Donafenib, etc.), HDAC inhibitor (e. g, Givinostat, Tucidinostat, Vorinostat, Fimepinostat, Droxinostat, Entinostat, Dacinostat, Quisinostat, Tacedinaline, etc.), CDK inhibitor (e. g, Palbociclib, Ribociclib, Abemaciclib, Milciclib, Trilaciclib, Lerociclib, etc.), MEK inhibitor (e. g, Simetinib (AZD6244), Trametinib (GSK1120212), PD0325901, U0126, Pimasertib (AS-703026), PD184352 (CI-1040), etc.), mTOR inhibitor (e. g, Vistusertib, etc.), SHP2 inhibitor (e. g, RMC-4630, JAB-3068, TNO155, etc.), and a combination thereof.

In another preferred embodiment, there is provided a method for preparing a pharmaceutical composition, comprising the step of mixing a pharmaceutically acceptable carrier with the compound or the stereoisomer or optical isomer, pharmaceutically acceptable salt, prodrug or solvate of the first aspect of the present invention, thereby forming the pharmaceutical composition.

In another preferred embodiment, the compound of the present invention can be prepared into powder, tablet, granule, capsule, solution, emulsion, suspension and the like.

In the third aspect of the present invention, it provides a use of the compound, or the stereoisomer or optical isomer, pharmaceutically acceptable salt, prodrug or solvate thereof of the first aspect or the pharmaceutical composition of the second aspect, for preparing a medicament or a pharmaceutical composition for treating or preventing a disease related to the activity or expression of JAK kinase.

In another preferred embodiment, the disease related to the activity or expression of JAK kinase is a disorder related to JAK3.

In another preferred embodiment, the disease related to the activity or expression of JAK3 is selected from the group consisting of organ transplant rejection, xenograft rejection, lupus erythematosus, multiple sclerosis, rheumatoid arthritis, psoriasis, cancer, asthma, atopic dermatitis, type I diabetes and diabetic complications, autoimmune thyroid disorders, ulcerative colitis, Crohn's disease, Alzheimer's disease, alopecia areata, mast cell-mediated allergic reactions, thromboembolic, allergic complications and vitiligo.

In another preferred embodiment, the cancer is selected from the group consisting of leukemia, lymphoma, and multiple myeloma.

In the fourth aspect of the present invention, it provides a preparation method for the compound or the stereoisomer or optical isomer, the pharmaceutically acceptable salt, the prodrug or the solvate thereof of the first aspect, comprising the following step:
in an inert solvent, compound A₈ undergoes ring-forming reaction in the presence of a catalyst to obtain the compound of formula I; 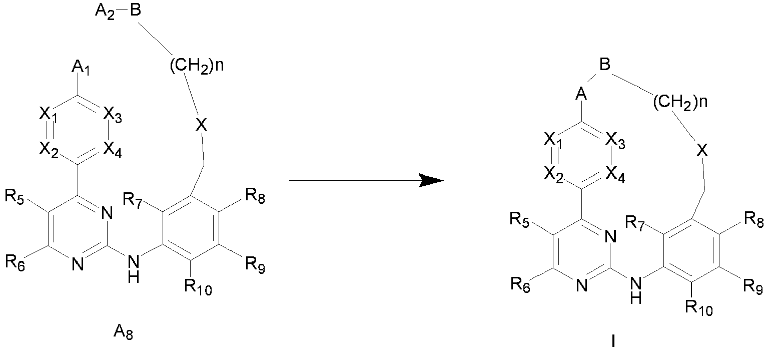 wherein,
A₁ is selected from the group consisting of carboxyl, sulfonic group, CO-O-R", and -CO-NH-R"; wherein, R" is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
A₂ is selected from the group consisting of amino and hydroxyl;
A, B, X₁, X₂, X₃, X₄, R₅, R₆, R₇, R₈, R₉, R₁₀, X and n are defined as above.

In an inert solvent, compound A₈ undergoes reaction in the presence of a catalyst or a condensing agent to obtain compound I.

In another preferred embodiment, the inert solvent is DCM.

In another preferred embodiment, the catalyst or condensing agent is selected from EDCI, DMAP, DCC, etc..

It should be understood that in the present invention, any of the technical features specifically described above and below (such as in the Example) can be combined with each other, thereby constituting new or preferred technical solutions. Limited by space, it will not be repeated herein.

### DETAILED DESCRIPTION OF THE INVENTION

Through extensive and in-depth research, the present inventors have accidentally discovered a new JAK inhibitor for the first time, which has a novel structure, and has good biological activity and extremely excellent selectivity. Specifically, the selectivity of the compounds of the present invention represented by the ratio of JAK3/JAK1 or the selectivity represented by the ratio of JAK3/JAK2 is increased by an average of about 12 times. Therefore, the side effects associated with JAK3 inhibition of the compounds of the present invention are extremely significantly reduced, and the safety will be significantly improved. On this basis, the present invention was completed.

In the present invention, unless otherwise specified, the terms used have the general meanings known to those skilled in the art.

When a substituent is described by a conventional chemical formula written from left to right, the substituent also includes a chemically equivalent substituent obtained by writing a structural formula from right to left. For example, -CH₂O- is equivalent to -OCH₂-.

As used herein, the term "about" means that the value can change by no more than 1% from the enumerated value when used in reference to a specific enumerated value. For example, as used herein, the expression "about 100" includes 99 and 101 and all values therebetween (e. g., 99.1, 99.2, 99.3, 99.4, etc.).

As used herein, the terms "contain" or "include(comprise)" may be open, semi-closed, and closed. In other words, the term also includes "consisting essentially of" or "consisting of".

As used herein, the term "alkyl" includes a linear or branched alkyl. For example, C₁-C₆ alkyl represents a linear or branched chain alkyl having 1 to 6 carbon atoms, such as methyl, ethyl, propyl, isopropyl, butyl, isobutyl, tert-butyl, and the like.

As used herein, the term "alkenyl" includes a linear or branched alkenyl. For example, C₂-C₆ alkenyl refers to a linear or branched chain alkenyl having 2-6 carbon atoms, such as vinyl, allyl, 1-propenyl, isopropenyl, 1-butenyl, 2-butenyl, or the like.

As used herein, the term "alkynyl" includes a straight or branched chain alkynyl. For example, C₂-C₆ alkynyl refers to a linear or branched chain alkynyl with 2-6 carbon atoms, such as acetenyl, propinyl, butynyl, or the like.

As used herein, the term "cycloalkyl" refers to a cyclic alkyl containing a specific number of C atoms, such as "C3-C10 cycloalkyl" refers to cycloalkyl having 3-10 (preferably 3, 4, 5, 6, 7 or 8) carbon atoms. It may be monocyclic, such as cyclopropyl, cyclobutyl, cyclopentyl, cyclohexyl, or the like. It can also be bicyclic, such as a bridged ring or a spiro ring. In the present invention, cycloalkyl is intended to include substituted cycloalkyl. The cycloalkyl can be fused to aryl, heteroaryl, or heterocyclyl ring, wherein the ring connected to the parent structure is cycloalkyl, such as 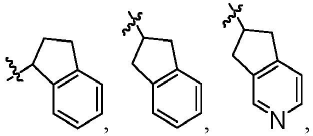 etc..

As used herein, the term "C1-C6 alkoxy" refers to a linear or branched chain alkoxy having 1-6 carbon atoms; which has the formula of C1-C6 alkyl-O- or C1-C5 alkyl-O-C1-C5 (e. g., -CH₂-O-CH₂CH₃, -CH₂-O-(CH₂)₂CH₃, -CH₂CH₂-O-CH₂CH₃), preferably C1-C6 alkyl-O-, such as methoxy, ethoxy, propoxy, isopropoxy, butoxy, isobutoxy, tert-butoxy, etc..

As used herein, "heterocyclyl" refers to a saturated or partially saturated cyclic group having a heteroatom selected from N, S, and O, and "3-10-membered heterocyclyl" refers to a saturated or partially saturated cyclic group having 3-10 atoms wherein 1-3 atoms are heteroatoms selected from N, S, or O. It can be monocyclic, and it can also be bicyclic, such as a bridged ring or a spiro ring. The 3-10 membered heterocyclyl is preferably 3-8 membered heterocyclyl, more preferably 3-6 membered, more preferably 6-8 membered heterocyclyl. Specific examples may be oxetanyl, azetidinyl, tetrahydro-2H-pyranyl, piperidinyl, piperazinyl, trahydrofuranyl, morpholinyl and pyrrolidinyl, etc. The heterocyclyl may be fused to heteroaryl, aryl or cycloalkyl ring, wherein the ring connected to the parent structure is heterocyclyl, such as 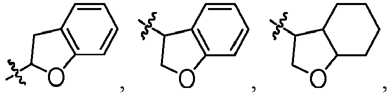 etc..

As used herein, "aryl" refers to an aromatic ring group having no heteroatoms on the ring, and "C6-C12 aryl" refers to an aromatic ring group having 6 to 12 carbon atoms without heteroatoms on the ring, the aryl may be fused to heteroaryl, heterocyclyl or cycloalkyl ring, wherein the ring connected to the parent structure is aryl ring. Such as phenyl (i. e., six-membered aryl), naphthyl, etc., wherein six-membered aryl is also intended to include six-membered aryl-fused 5-6-membered cycloalkyl (such as 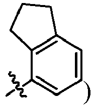 and six-membered aryl-fused 5-6-membered heterocyclyl (such as 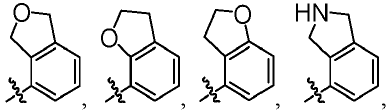 etc.). C6-C12 aryl is preferably C6-C10 aryl. Aryl can be optionally substituted or unsubstituted.

As used herein, "heteroaryl" refers to a cyclic aromatic group having 1-3 atoms selected from N, S, and O, and "5-12 membered heteroaryl" refers to a cyclic aromatic group having 5-12 atoms wherein 1-3 atoms are heteroatoms selected from N, S, and O. It can be monocyclic, and it can also be fused. Specific examples may be pyridyl, pyridazinyl, pyrimidinyl, pyrazinyl, triazinyl, pyrrolyl, pyrazolyl, imidazolyl, (1,2,3)-triazolyl, and (1,2,4)-triazolyl, tetrazolyl, furyl, thienyl, isoxazolyl, thiazolyl, oxazolyl, etc.. The heteroaryl ring may be fused to aryl, heterocyclyl or cycloalkyl, and the ring attached to the parent structure is heteroaryl. Heteroaryl can be optionally substituted or unsubstituted. When being substituted, the substituents are preferably one or more of the following groups independently selected from alkyl, deuterated alkyl, haloalkyl, alkoxy, haloalkoxy, alkenyl, alkynyl, alkylthio, alkylamino, halogen, amino, nitro, hydroxyl, sulfydryl, cyano, cycloalkyl, heterocyclyl, aryl, heteroaryl, cycloalkylthio, oxo, amido, sulfonamido, formyl, formamido, carboxyl and carboxylate, etc..

As used herein, "halogen" or "halogen atom" refers to F, Cl, Br, and I. More preferably, the halogen or halogen atom is selected from F, Cl and Br.

In the present invention, the term "amido" refers to a group with the structure of -CONRR', wherein R and R' can independently represent hydrogen, alkyl, cycloalkyl, aryl, heterocyclyl, as defined above. R and R' can be the same or different in dialkylamine fragments.

In the present invention, the term "sulfonamido" refers to a group with the structure of -SO₂NRR', wherein R and R' can independently represent hydrogen, alkyl, cycloalkyl, aryl, heterocyclyl, as defined above. R and R' can be the same or different in dialkylamine fragments.

In the present invention, the term "formyl" refers to a group comprising -CHO. In the present invention, the term "formamido" refers to a group containing 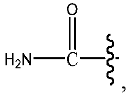 and the formamido is also intended to comprise substituted formamido having the formula of 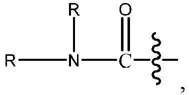 wherein each R independently represents hydrogen, alkyl, cycloalkyl, cycloalkenyl, aryl, heteroaryl, heterocyclyl, as defined above. Each R may be the same or different.

In the present invention, "amino" refers to NH₂.

In the present invention, "amine" refers to a group with the structure of -N-RR', R and R' each independently represent hydrogen, alkyl, cycloalkyl, aryl, heteroaryl, heterocyclyl, as defined above, R and R' may be the same or different, and not simultaneously hydrogen.

In the present invention, "sulfinyl" refers to a group with the structure of -S(O)-R, R independently represents hydrogen, alkyl, cycloalkyl, aryl, heteroaryl, heterocyclyl, as defined above.

In the present invention, "sulfonyl" refers to a group with the structure of -S(O)₂-R, R independently represents hydrogen, alkyl, cycloalkyl, aryl, heteroaryl, heterocyclyl, as defined above.

As used herein, an "ester group" refers to -C (O)-O-R or R-C (O)-O-, wherein R independently represents hydrogen, alkyl, cycloalkyl, aryl, heteroaryl, heterocyclyl with the definitions as described above.

In the present invention, the term "substituted" refers to one or more hydrogen atoms on a specific group being substituted by a specific substituent. The specific substituents are those described in the preceding paragraph or those present in each Example. Unless otherwise specified, a substituted group may have a substituent selected from a specific group at any substitutable position of the group, and the substituent may be the same or different in each position. Those skilled in the art should understand that the combinations of substituents contemplated by the present invention are those that are stable or chemically achievable.

Unless specifically indicated as "substituted or unsubstituted", the groups of the present invention may be substituted by substituents selected from the group consisting of deuterium, halogen, cyano, nitro, hydroxyl, amino, C1-C6 alkyl, C2-C6 alkenyl, C2-C6 alkynyl, C1-C6 alkoxy, 3-10-membered heterocyclyl, C3-C10 cycloalkyl, 5-12-membered heteroaryl, and C6-C12 aryl.

In the present invention, the term "more" independently refers to 2, 3, 4 or 5.

Unless otherwise specified, the structural formula described in the present invention is intended to include all isomeric forms (e. g., enantiomeric, diastereomeric, and geometric (or conformational) isomers): for example, R and S configurations of asymmetric centers, (Z) and (E) isomers of double bonds, etc. Thus, a single stereochemical isomer of the compound of the invention or a mixture of its enantiomers, diastereomers or geometric isomers (or conformational isomers) is within the scope of the invention.

As used herein, the term "tautomer" means that structural isomers with different energies can cross a low energy barrier and thus convert to each other. For example, proton tautomers (i.e. proton shift) include intertransformation through proton migration, such as 1H-indazole and 2H-indazole. Valence tautomers include interchange through some bonding electron recombination.

As used herein, the term "solvate" refers to a complex with specific proportion formed by the compound of the invention coordinates with a solvent molecule.

### Active ingredient

As used herein, "the compound of the present invention" refers to the compound represented by the formula I, and further comprises the stereoisomer or optical isomer, the pharmaceutically acceptable salt, the prodrug or the solvate of the compound of formula I. 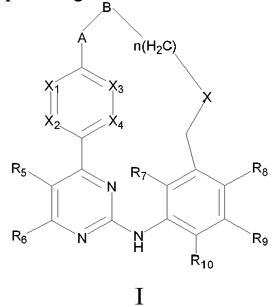 wherein,
X, n, A, B, X₁, X₂, X₃, X₄, R₅, R₆, R₇, R₈, R₉, and R₁₀ are defined as above.

Preferably, the compound of formula I or the stereoisomer or optical isomer, pharmaceutically acceptable salt, prodrug or solvate thereof, which has the structure shown in formula I' : 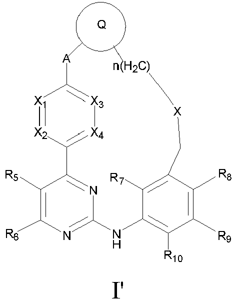 wherein,
Q, X, n, A, X₁, X₂, X₃, X₄, R₅, R₆, R₇, R₈, R₉, and R₁₀ are defined as above.

Preferably, in the above compounds, X₂ is C-R_{d}, and R_{d} is selected from the group consisting of D, amino, amine, C1-C6 alkyl, hydroxyl, and sulfydryl, the alkyl can be optionally substituted by one or more groups selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

When A is -C(= O)N-R_{b}, the carbonyl moiety is connected to 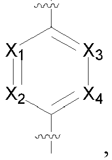 wherein R_{b} is defined as above.

Preferably, in the above compounds, X₁, X₂, X₃ and X₄ are each independently N or C-R_{d}; preferably, X₂ is CH, C-NH₂, C-N(R_{b})2, C-OH, C-SH; preferably, X₁ is CH, C-halogen; preferably, X₃ is CH; preferably, X₄ is CH.

Preferably, the compound shown in formula I or the stereoisomer or optical isomer, pharmaceutically acceptable salt, prodrug or solvate thereof, which has the structure shown in formula II: 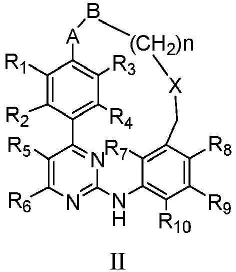 wherein,
A, B, R₁, R₂, R₃, R₄, R₅, R₆, R₇, R₈, R₉, R₁₀, and n are defined as above.

Preferably, the compound shown in the formula I or the stereoisomer or optical isomer, pharmaceutically acceptable salt, prodrug or solvate thereof has a structure shown in formula III: 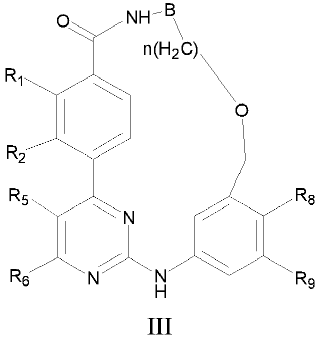 wherein,
B, m, n, R₁, R₂, R₅, R₆, R₈, and R₉ are defined as above.

Preferably, in the above compounds, B is selected from the substituted or unsubstituted group consisting of bond, 5-12-membered heteroarylene, and C6-C12 arylene; wherein, the "substituted" refers to being substituted by one or more groups selected from the group consisting of D, halogen, amino, amine, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl; n is 0 or 1.

Preferably, in the above compounds, A is selected from the group consisting of -C(= O)N-R_{b}, -C(= O)O-, -SO₂N(R_{b})- (preferably -C(=O)N-R_{b}), and R_{b} is defined as above.

Preferably, the compound or the stereoisomer or optical isomer, pharmaceutically acceptable salt, prodrug or solvate thereof has a structure shown in formula IV: 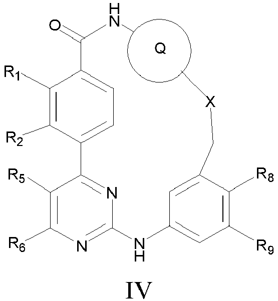 wherein,
Q, X, m, R₁, R₂, R₅, R₆, R₈, and R₉ are defined as above.

Preferably, the compound shown in the formula I or the stereoisomer or optical isomer, pharmaceutically acceptable salt, prodrug or solvate thereof has a structure shown in formula V 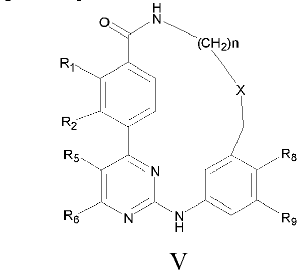 wherein, R₅, R₆, R₈, R₉, X, n, R₁, and R₂ are defined as above.

Preferably, in the above compounds, X is C(Rc)2, O or S, wherein Rc is defined as above.

Preferably, the compound of formula I or the stereoisomer or optical isomer, pharmaceutically acceptable salt, prodrug or solvate thereof has a structure shown in formula VI 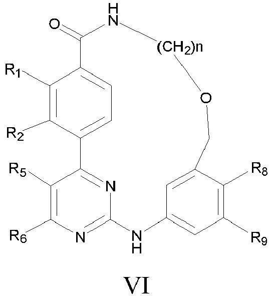 wherein, R₁, R₂, R₅, R₆, R₈, R₉ and n are defined as above.

Preferably, the compound of formula I or the stereoisomer or optical isomer, pharmaceutically acceptable salt, prodrug or solvate thereof has a structure shown in formula VII 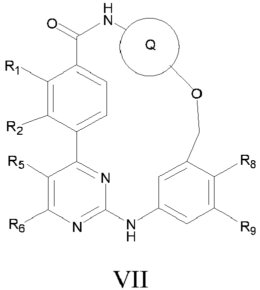 wherein, R₁, R₂, R₅, R₆, R₈, R₉ and Q are defined as above.

Preferably, the compound of formula I or the stereoisomer or optical isomer, pharmaceutically acceptable salt, prodrug or solvate thereof has a structure shown in formula VIII 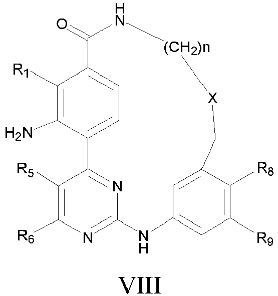 wherein, R₁, R₅, R₆, R₈, R₉, X and n are defined as above.

Preferably, in the above compounds, R₂ is selected from the group consisting of H, amino, amine, C1-C6 alkyl, hydroxyl, and sulfydryl;
preferably, in the above compounds, R₃ is H.

Preferably, in the above compounds, R₄ is H.

Preferably, in the above compounds, R₅ is H, halogen, cyano, C1-C6 alkyl, C2-C6 alkenyl, C2-C6 alkynyl, C1-C6 alkoxy, 3-10 membered heterocyclyl, and C3-C10 cycloalkyl, the alkyl, alkoxy, alkenyl, alkynyl, heterocyclyl and cycloalkyl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

Preferably, in the above compounds, R₆ is H.

Preferably, in the above compounds, R₇ is H.

Preferably, in the above compounds, R₈ is selected from the group consisting of H, halogen, amino, amine, C1-C6 alkyl, C2-C6 alkenyl, C2-C6 alkynyl, 3-10 membered heterocyclyl, C3-C10 cycloalkyl, 5-12 membered heteroaryl, C6-C12 aryl and -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl.

Preferably, in the above compounds, R₉ is selected from the group consisting of H, C1-C6 alkyl, C1-C6 alkoxy, 3-10-membered heterocyclyl, and C3-C10 cycloalkyl.

Preferably, in the above compounds, R₁₀ is H.

Preferably, in the above compounds, n is 1, 2, 3, 4 or 5.

The above-mentioned alkyl, alkoxy, alkenyl, alkynyl, heterocyclyl, cycloalkyl, heteroaryl, and aryl can be further optionally substituted by one or more Rₐ, wherein, each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

As used herein, "pharmaceutically acceptable salt" refers to a salt formed by a compound of the present invention and an acid or a base suitable for use as a medicine. Pharmaceutically acceptable salt comprises inorganic salt and organic salt. A preferred class of salt is a salt formed by a compound of the present invention with an acid. The acids suitable for salt formation include but are not limited to mineral acids such as hydrochloric acid, hydrobromic acid, hydrofluoric acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, phenylmethanesulfonic acid, and benzenesulfonic acid; and acidic amino acids such as aspartic acid and glutamic acid.

### Preparation method of compound

Methods for preparing compounds of formula I are described in the following schemes and examples. Raw materials and intermediates are purchased from commercial sources, prepared by known steps, or otherwise described. In some cases, the sequence of steps to perform the reaction scheme may be changed to facilitate the reaction or avoid unwanted side reaction products.

Generally, in the preparation process, each reaction is usually carried out in an inert solvent at room temperature to reflux temperature (e. g., 0°C to 150°C, preferably 10°C to 100°C). The reaction time is usually 0.1-60 hours, preferably 0.5-48 hours.

The preparation of the compound of the present invention includes the steps: in an inert solvent, compound A₈ is reacted in the presence of a catalyst to obtain compound I; 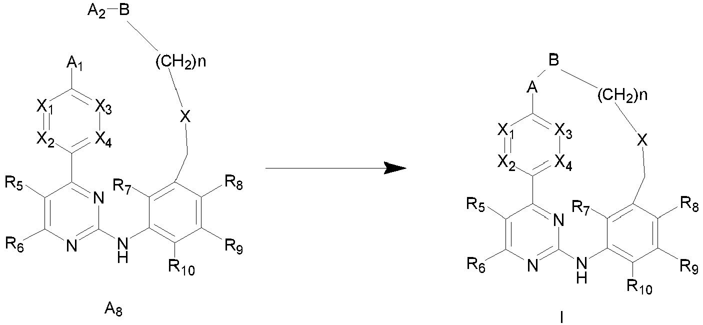 wherein,
A₁ is selected from the group consisting of carboxyl, sulfonic group, CO-O-R", and -CO-NH-R"; wherein, R" is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
A₂ is selected from the group consisting of amino and hydroxyl;
A, B, X₁, X₂, X₃, X₄, R₅, R₆, R₇, R₈, R₉, R₁₀, X and n are defined as above.

Preferably, the compound of formula A of the present invention can be obtained by the following steps: 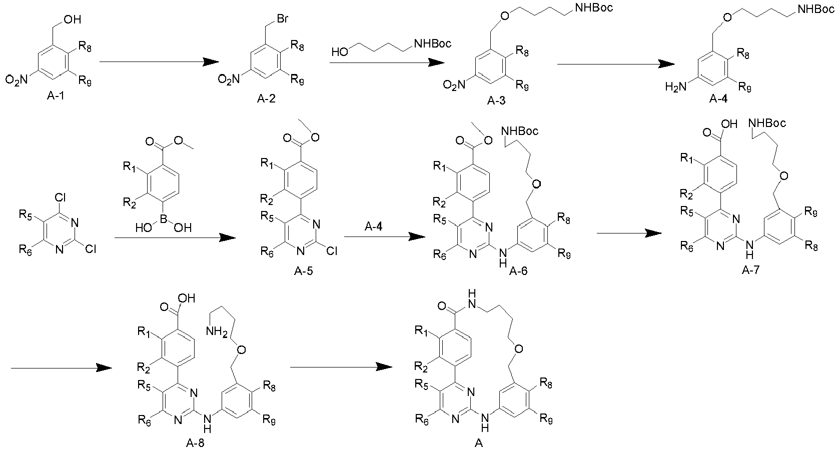 wherein, R₁, R₂, R₅, R₆, R₈, and R₉ are defined as above;
(i) in an inert solvent (such as DCM), compound A-1 reacts with a halogenating agent (such as carbon tetrabromide, triphenylphosphine) to obtain compound A-2;
(ii) in an inert solvent (such as DMF), in the presence of alkali (such as sodium hydride), compound A-2 reacts with 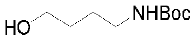 to obtain compound A-3;
(iii) in an inert solvent (such as ethanol), in the presence of a reducing agent (such as palladium/carbon), compound A-3 undergoes a reduction reaction to obtain compound A-4;
(iv) in an inert solvent such as DMF and/or 1, 4-dioxane and/or water), 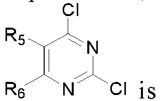 coupled with 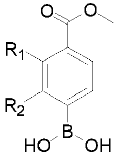 under a base (such as sodium carbonate, potassium carbonate, etc.) in the presence of a catalyst (such as bis-triphenylphosphorus palladium dichloride) to obtain compound A-5;
(v) in an inert solvent (such as 1, 4-dioxane), compound A- 5 is coupled with compound A-4 under a base (such as sodium carbonate, potassium carbonate, etc.) in the presence of a catalyst (such as tris (dibenzylidene acetone/2-dicyclohexylphosphino-2,4,6-triisopropylbiphenyl) to obtain compound A-6;
(vi) in an inert solvent (such as THF and water), the compound of formula A- 6 is hydrolyzed to obtain the compound of formula A- 7 under basic (lithium hydroxide monohydrate) condition;
(vii) in an inert solvent (such as EA), deprotection group (such as Boc) is deprotected from the compound of formula A-7 to obtain compound A-8 under acidic (such as hydrogen chloride) condition;
(viii) in an inert solvent (such as DCM), the compound A-8 undergos a condensation reaction in the presence of a condensing agent (such as O-(7-azabenzotriazol-1--yl)-N,N,N',N'-tetramethyluronium hexafluorophosphate/N,N-diisopropylethylamine) to obtain the compound of formula A.

In the above synthesis steps, the starting materials and reagents used can be commercially purchased or synthesized by methods reported in the literature.

### Pharmaceutical composition and method of administration

Since the compounds of the present invention have excellent JAK kinase inhibitory activity, the compound of the present invention or the stereoisomer or optical isomer, pharmaceutically acceptable salt, prodrug or solvate thereof, and the pharmaceutical composition containing the compound of the present invention as the main active ingredient can be used to prevent and/or treat (stabilize, mitigate or cure) JAK kinase-related diseases (e. g, skin diseases, rheumatoid arthritis, multiple sclerosis, type I diabetes, psoriatic arthritis, juvenile arthritis, Crohn's disease, myasthenia gravis, cancer (including prostate cancer, kidney cancer, liver cancer, breast cancer, lung cancer, thyroid cancer, Kaposi's sarcoma, Castleman's disease, pancreatic cancer, leukemia, lymphoma or multiple myeloma, etc.).

The pharmaceutical composition of the present invention comprises a safe and effective amount of the compound of the present invention and a pharmaceutically acceptable excipient or carrier, wherein "safe and effective amount" refers to the amount of compound is sufficient to significantly improve the condition, not to produce severe side effects. Typically, the pharmaceutical composition contains 1-2000 mg of the compound of the present invention/ dosage, and preferrably contains 10-200mg of the compound of the present invention / dosage. Preferably, "one dosage" is a capsule or a pill.

"Pharmaceutically acceptable carrier" refers to one or more compatible solid or liquid filler or gel substances, which are suitable for human use, and must be sufficiently pure and sufficiently low toxicity. "Compatible" herein refers to each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc..

There is no special limitation of administration mode for the compound or pharmaceutical compositions of the present invention, and the representative administration mode includes (but is not limited to) oral, parenteral (intravenous, intramuscular or subcutaneous).

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer,such as starch, lactose, sucrose, glucose, mannitol and silicic acid; (b) binders, such as hydroxymethyl cellulose, alginate, gelatin, polyvinylpyrrolidone, sucrose and arabic gum; (c) humectant, such as, glycerol; (d) disintegrating agent, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, for example, kaolin; and (i) lubricants such as talc, calcium stearate, magnesium stearate, solid polyethylene glycol, lauryl sodium sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, such as ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The compounds of the present invention can be administered alone or in combination with other pharmaceutically acceptable compounds (such as JAK inhibitors).

In combination administration, the pharmaceutical composition further comprises one or more (2, 3, 4, or more) other pharmaceutically acceptable compounds (e. g., JAK inhibitors). The one or more (2, 3, 4, or more) of the other pharmaceutically acceptable compounds (e. g., JAK inhibitors) may be used simultaneously, separately, or sequentially with the compounds of the present invention for the prevention and/or treatment of diseases related to the activity or expression of JAK kinase.

When the pharmaceutical composition is used, a safe and effective amount of the compound of the present invention is applied to a mammal (such as a human) in need of treatment, wherein the dose is considered as a pharmaceutically effective dose. For a person weighing 60kg, the daily dose is usually 1 to 2000mg, preferably 20 to 500mg. Of course, the particular dose should also depend on various factors, such as the route of administration, patient healthy status, which are well within the skills of an experienced physician..

### The main advantages of the present invention:

1. The compound of the invention has a novel structure and excellent JAK kinase inhibitory effect;
2. The compound of the present invention can be used as a JAK kinase inhibitor, especially as a highly selective inhibitor of JAK3.

The present invention will be further illustrated below with reference to the specific examples. It should be understood that these examples are only to illustrate the invention but not to limit the scope of the invention. The experimental methods without specific conditions in the following examples usually follow conventional conditions, or according to the conditions recommended by the manufacturer. Unless indicated otherwise, percentage and parts are calculated by weight.

Unless otherwise specified, the experimental materials and reagents used in the following examples can be obtained from commercial sources.

### General materials and testing methods:

Methods for the synthesis of the compounds of the present invention are shown in the following schemes, methods and examples. The starting materials are commercially available or can be prepared according to known methods in the art or described herein. The compounds of the present invention can be illustrated by the specific examples shown below. However, these specific examples should not be construed as being of the only kind of the present invention. These examples further detail the preparation of the compounds of the invention. Those skilled in the art will easily understand that known changes in conditions and processes can be used to prepare these compounds. Unless otherwise stated, all temperatures are in degrees Celsius.

Thin layer chromatography (PTLC) was prepared on a 20 × 20cm plate (500 micron of silica gel). Biotage rapid chromatography system was used for silica gel chromatography.

1H NMR was conducted by Bruker AscendTM400 spectrometer at 400MHz at 298°K, and the chemical shifts (PPM) of the residual protons in the deuterated reagent were given as reference;
CHCl3 δ = 7.26 ppm, CH3OH or CH3OD δ = 3.30 ppm, DMSO-*d₆* δ = 2.50 ppm

LCMS chromatography was conducted by Agilent Technology 1200 series or 6120 quadrupole spectrometer. For LC, mobile phase was acetonitrile (A) and water (B) and 0.01% formic acid, eluent gradients: 6.0 min 5-95% A, 5.0 min 60-95% A, 5.0 min 80-100% A and 10 min 85-100% A, and capillary column was SBC1850 mm × 4.6mm × 2.7 micron.

Mass spectrometry (MS) was determined by electrospray ion mass spectrometry (ESI).

HPLC mass spectrometry analysis conditions:
LC1:
   Column: SB-C18 50mm × 4.6mm × 2.7 µm
   Temperature: 50 °C
   Eluent: 5:95 to 95:5 volume/volume acetonitrile/water + 0.01% formic acid, 6 minutes.
   Flow rate: 1.5 mL/min, injection 5µL
   Detection: PDA, 200-600 nm
   MS: mass range 150-750 amu; Positive ion electrospray ionization
LC2:
   Column: SB-C18 50mm × 4.6mm × 2.7 µm
   Temperature: 50°C
   Eluent: 5:95 to 95:5 volume/volume acetonitrile/water + 0.05% TFA for more than 3.00 minutes.
   Flow rate: 1.5 mL/min, injection 5µL
   Detection: PDA, 200-600 nm
   MS: mass range 150-750 amu; Positive ion electrospray ionization
LC3:
   Column: SB-C18 50mm × 4.6mm × 2.7 µm
   Temperature: 50°C
   Eluent: 10:90 to 98:2 volume/volume acetonitrile/water + 0.05% TFA for more than 3.75 minutes.
   Flow rate: 1.0 mL/min, injection 10 µL
   Detection: PDA, 200-600 nm
   MS: mass range 150-750 amu; Positive ion electrospray ionization

### Abbreviations:

AcOH = acetic acid
Alk is alkyl
AR is aryl
Boc = tert-butoxycarbonyl
bs = broad peak
CH₂Cl₂= dichloromethane
d = doublet
dd = double doublet
DBU = 1,8-diazabicyclo[5.4.0]undec-7-ene
DCM = dichloromethane
DEAD = diethyl azodicarboxylate
DMF = N,N-dimethylformamide
DMSO = dimethyl sulfoxide
EA = ethyl acetate
ESI = electrospray ionization
Et = ethyl
EtOAc = ethyl acetate
EtOH = ethanol
h = hour
HOAc = acetic acid
LiOH = lithium hydroxide
m = multiple
Me = methyl
MeCN = acetonitrile
MeOH = methanol
MgSO₄= magnesium sulfate
min = minute
MS = mass spectrometry
NaCl = sodium chloride
NaOH = sodium hydroxide
Na₂SO₄= sodium sulfate
NMR = nuclear magnetic resonance spectrum
PE = petroleum ether
PG = protecting group
Ph = phenyl
rt = room temperature
s = singlet
t = triplet
TFA = trifluoroacetic acid
THF = tetrahydrofuran
TS = p-toluenesulfonyl (toluenesulfonyl)

Synthesis route of compound A1-A94:

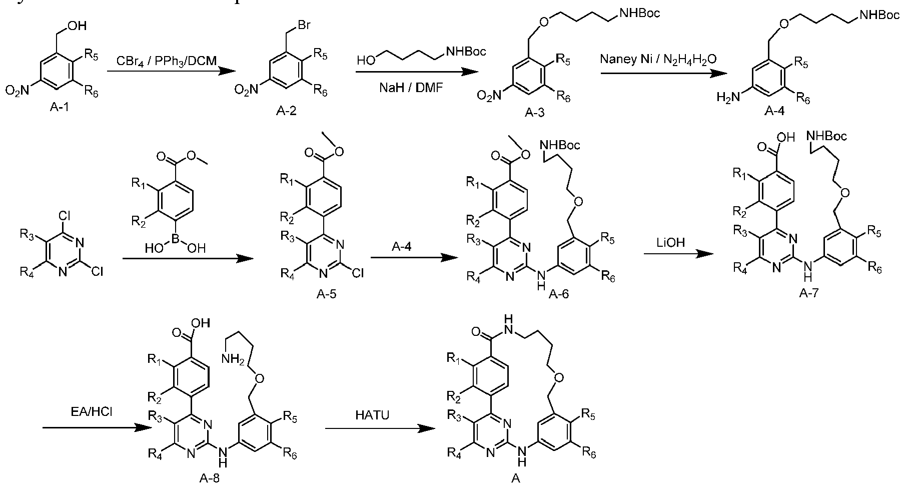

### Example1 Preparation of 4⁴-morpholin-6-oxa-3, 11-diaza-2 (4,2)-pyrimidina -1(1,4),4(1,3)-dibenzenacyclododecaphan-12-one(A1)

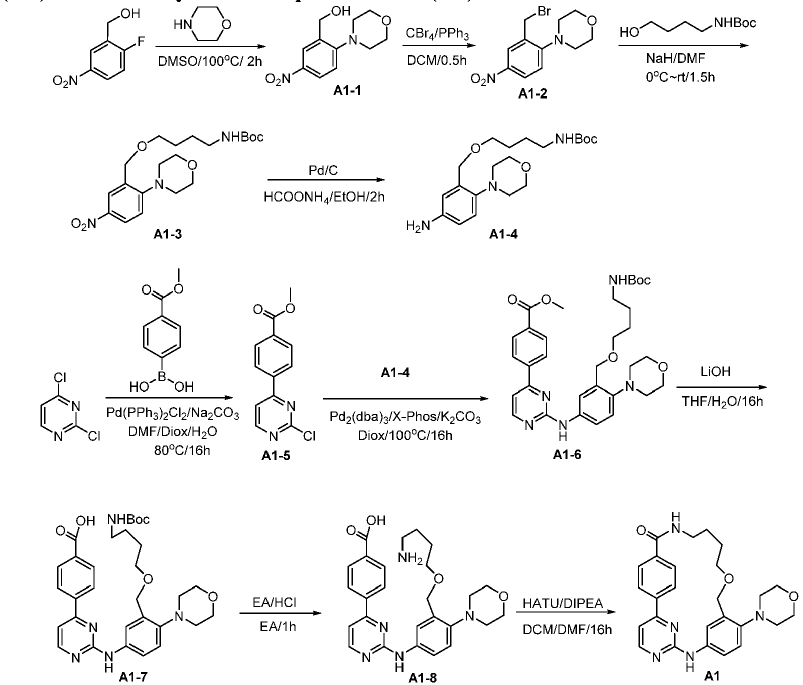

### Step 1:2-morpholin-5-nitrobenzyl alcohol(A1-1)

2-Fluoro-5-nitrobenzyl alcohol (10.0g, 58mmol) and equivalent morpholine were added to DMSO(100ml), heated to 100°C and stirred for 2 hours. After TLC confirmed that the reaction was completed, the reactin solution was poured into water, extracted with EA, washed with deionized water for three times, dried over anhydrous sodium sulfate. EA was concentrated, and the residue was purified by column chromatography to obtain 2-morpholin-5-nitrobenzyl alcohol (A1-1, 13.0g, yield 94%) as a yellow solid. MS (ESI) m/z: calcd 239.10 (M+H), found 239.23; ¹H NMR (400 MHz, DMSO): δ 8.34 (d, *J* = 2.7 Hz, 1H), 8.10 (dd, *J* = 8.9, 2.8 Hz, 1H), 7.17 (d, *J* = 8.9 Hz, 1H), 5.54 (t, *J* = 5.7 Hz, 1H), 4.55 (d, *J* = 5.7 Hz, 2H), 3.76 (t, *J* = 4.0 Hz, 4H), 3.01 (t, *J* = 4.0 Hz, 4H).

### Step 2: 4-(2-bromomethyl-4-nitrophenyl) morpholine(A1-2)

2-Morpholine -5-nitrobenzyl alcohol (**A1-1**, 10.00g, 42mmol) and triphenylphosphine (22.03g, 84mmol) were added to DCM(100ml), stirred for 10min, cooled to 0°C in an ice bath, and a solution of carbon tetrabromide (27.84g, 84mmol) in DCM (50ml) was added dropwise. After the addition, the reaction solution was stirred at room temperature for 1h. After TLC confirmed that the reaction was completed, the reaction solution was washed with deionized water, dried over anhydrous sodium sulfate. DCM was concentrated, and the residue was purified by column chromatography to obtain 4-(2-bromomethyl-4-nitrophenyl) morpholine (**A1-2,** 10.05g, yield 79%) as a yellow solid. MS (ESI) m/z: calcd 301.02 (M+H), found 301.1; ¹H NMR (400 MHz, CDCl₃): δ 8.38 (s, 1H), 8.17 (d, *J* = 8.8 Hz, 1H), 7.16 (d, *J* = 8.8 Hz, 1H), 4.65 (s, 2H), 3.92 (t, *J* = 4.8 Hz, 4H), 3.13 (t, *J* = 4.8 Hz, 4H).

### Step 3: tert-butyl 4-((2-morpholin-5-nitro) benzyloxy) butylamine-1-carboxylate (A1-3)

Under nitrogen protection, 4-(N-tert-butoxycarbonylamino)-1-butanol (189mg, 1.0mmol) was added to anhydrous DMF(4ml), cooled to 0°C in an ice bath, sodium hydride (53mg, 1.33mmol, 60%) was added, and continued to stir under an ice bath for 30min. Then a solution of 4-(2-bromomethyl-4-nitrophenyl) morpholine (**A1 -2, 200**mg, 0.67mmol) in DMF(4ml) was slowly added, the temperature was slowly rised to room temperature, continued stirring for 1h. After TLC confirmed that the reaction was completed, saturated ammonium chloride solution was added to quench under an ice bath, extracted with EA, washed with deionized water for three times, dried over anhydrous sodium sulfate. EA solution was concentrated, and the residue was purified by column chromatography to obtain **A1-3** (180mg, yield 66%). MS (ESI) m/z: calcd 410.23 (M+H), found 410.35; ¹H NMR (400 MHz, CDCl₃): δ 8.36 (d, *J* = 2.6 Hz, 1H), 8.16 (dd, *J* = 8.9, 2.7 Hz, 1H), 7.08 (d, *J* = 8.0 Hz, 1H), 4.55 (s, 2H), 3.90 (t, *J* = 4.0 Hz, 4H), 3.58 (t, *J* = 6.2 Hz, 2H), 3.22-3.12 (m, 2H), 3.08 (t, *J* = 4.0 Hz, 4H), 1.80 - 1.55 (m, 4H), 1.46 (s, 9H).

### Step 4:tert-butyl 4-((5-amino-2-morpholin)benzyloxy)butylamino-1-carboxylate(A1-4)

Tert-butyl 4-((2-morpholin-5-nitro) benzyloxy) butylamino-1-carboxylate (**A1-3**, 180mg, 0.44mmol) was added to absolute ethanol (7ml), and then ammonium formate (277mg, 4.4mmol) and palladium/carbon (47mg, 0.044mmol, 10% on carbon(wetted with ca. 55% water)) were added respectively, and the reaction solution was stirred at room temperature for 2h. After TLC and LCMS confirmed that the reaction was completed, the reaction solution was filtered by suction filtration, and the filtrate was concentrated. The residue was purified by column chromatography to obtain tert-butyl 4-((5-amino-2-morpholin) benzyloxy) butylamino-1-carboxylate (**A1-4**, 166mg, yield 99%) as a yellow oil. MS (ESI) m/z: Calcd 380.25 (M + H), found 380.17;

### Step 5: Methyl 4-(2-chloropyrimidin-4-)benzoate(A1-5)

Under nitrogen protection, 2,4-dichloropyrimidine (3.00g, 20.3mmol) and 4-methoxycarbonylphenylboronic acid (4.38g, 24.3mmol) were added to a mixed solvent of DMF, 1,4-dioxane, and water (24ml, 15ml, 6ml), and sodium carbonate (6.45g, 60.9mmol) was added, then bis(triphenylphosphine)palladium dichloride (1.42g, 2.03mmol) was added, and the reaction solution was heated to 80°C and stirred for 16h. After TLC confirmed that the reaction was completed, 1, 4-dioxane was concentrated, the solution was extracted with EA, and the organic phase was washed with deionized water for three times, dried over anhydrous sodium sulfate. EA was concentrated, and the residue was purified by column chromatography to obtain methyl 4-(2-chloropyrimidin-4-) benzoate (**A1-5**, 4.27g, yield 85%) as a white solid. MS (ESI) m/z: Calcd 249.04 (M + H), found 249.12; ¹H NMR (400 MHz, CDCl₃): δ 8.73 (d, J = 5.2 Hz, 1H), 8.24 - 8.16 (m, 4H), 7.73 (d, *J* = 5.2 Hz, 1H), 3.99 (s, 3H).

### Step 6: methyl 4-(2-((3-((4-(tert-butoxycarbonylamino) butoxy) methyl)-4-morpholinophenyl)amino)pyrimidin-4-) benzoate(A1-6)

Under nitrogen protection, methyl 4-(2-chloropyrimidin-4-) benzoate (**A1-5**, 119mg, 0.48mmol) and tert-butyl 4-((5-amino -2-morpholin) benzyloxy) butylamino-1-carboxylate (**A1-4**, 166mg, 0.44mmol) were added to 1,4-dioxane (5ml), then potassium carbonate (121mg, 0.88mmol), tris(dibenzylideneacetone)dipalladium (20mg, 0.022mmol) and 2-dicyclohexylphosphino-2,4,6-triisopropylbiphenyl (23mg, 0.048mmol) were added, heated to 100°C and stirred for 16h. After TLC confirmed that the reaction was completed, 1,4-dioxane was concentrated, deionized water and dichloromethane were added, and the liquid was separated. The organic phase was washed with deionized water for three times, dried over anhydrous sodium sulfate, dichloromethane was concentrated, and the residue was purified by column chromatography to obtain methyl 4-2-((3-((4-(tert-butoxycarbonylamino) butoxy)methyl)-4-morpholinophenyl) amino) pyrimidin-4-) benzoate (**A1-6**, 145mg, yield 56%) as a yellow solid. MS (ESI) m/z: calcd 592.72 (M+H), found 592.95; ¹H NMR (400 MHz, DMSO-d6): δ 9.68 (s, 1H), 8.59 (d, *J* = 5.1 Hz, 1H), 8.32 (d, *J* = 8.4 Hz, 2H), 8.12 (d, *J* = 8.1 Hz, 2H), 7.91 (s, 1H), 7.70 (d, *J* = 8.4 Hz, 1H), 7.46 (d, *J* = 5.2 Hz, 1H), 7.11 (d, *J* = 8.7 Hz, 1H), 6.77 (s, 1H), 4.53 (s, 2H), 3.91 (s, 3H), 3.74 (m, 4H), 3.50 (t, *J* = 4.0 Hz, 2H), 2.91 (d, *J* = 6.0 Hz, 2H), 2.84 (s, 4H), 1.49 (m, 4H), 1.35 (s, 9H).

### Step 7: 4-(2-((3-(4-(tert-butoxycarbonylamino) butoxy) methyl)-4-morpholinophenyl) amino) pyrimidin-4-) benzoic acid(A1-7)

Methyl 4-(2-((3-((4-((tert-butoxycarbonylamino) butoxy) methyl)-4-morpholinophenyl) amino) pyrimidin-4-) benzoate (**A1-6**, 145mg, 0.24mmol) was added to a mixed solution of THF and water (6ml/2ml), and then lithium hydroxide monohydrate (51mg, 1.23mmol) was added, and the reaction solution was stirred at room temperature for 16h. After TLC confirmed that the reaction was completed, 1M HCl was added to adjust the PH of the system to 7.0, the solvent was concentrated, and EA and water were added for liquid extraction. The solution was washed with deionized water, and dried over anhydrous sodium sulfate, and EA was concentrated to obtain 4-(2-((3-((4-((tert-butoxycarbonylamino) butoxy) methyl)-4-morpholinophenyl) amino) pyrimidin-4-) benzoic acid (**A1-7**, 124mg, yield 88%) as a yellow solid which was directly used in the next reaction.

### Step 8: 4-(2-((3-((4-aminobutoxy) methyl)-4-morpholinophenyl) amino) pyrimidin-4-) benzoic acid(A1-8)

4-(2-((3-((4-((tert-butoxycarbonylamino) butoxy) methyl)-4-morpholinophenyl) amino) pyrimidin-4-) benzoic acid (**A1-7**, 124mg, 0.21mmol) was added to EA(2ml), hydrogen chloride/EA solution (1mol/L, 1ml) was added dropwise, and stirred at room temperature for 1h. After TLC confirmed that the reaction was completed, the reaction solution was concentrated to obtain 4-(2-((3-((4-aminobutoxy) methyl)-4-morpholinophenyl) amino) pyrimidin-4-) benzoic acid (**A1-8**, 120mg, yield 100%) as a yellow solid. MS (ESI) m/z: calcd 478.25 (M + H), found 478.33.

### Step 9: 4⁴-morpholin-6-oxa-3,11-diaza-2(4,2)-pyrimidina-1(1,4),4(1,3)-dibenzenacyclododecaphan -12-one(A1)

O-(7-Azabenzotriazol-1-yl)-N,N,N',N'-tetramethyluronium hexafluorophosphate (143mg, 0.36mmol) and N,N-diisopropylethylamine (162mg, 1.2mmol) were added to DCM(10ml) and stirred at room temperature. A mixed solution of 4-(2-((3-((4-aminobutoxy) methyl)-4-morpholinophenyl) amino) pyrimidin-4-) benzoic acid (**A1-8**, 60mg, 0.11mmol) in DCM and DMF(3ml/7ml) was slowly added dropwise and kept for 1h, and the reaction solution was continued to be stirred at room temperature for 16h after addition. After LCMS confirmed that the reaction was completed, the reaction solution was concentrated, and the residue was purified by column chromatography to obtain 4⁴-morpholin-6-oxa-3, 11-diaza-2 (4,2)-pyrimidina -1(1,4),4(1,3)-dibenzenacyclododecaphan-12-one (**A1**, 15mg, yield 30%) as a yellow solid. MS (ESI) m/z: calcd 460.23 (M+H), found 460.52; ¹H NMR (400 MHz, DMSO-d6): δ 9.59 (s, 1H), 8.55 (d, *J* = 5.0 Hz, 1H), 8.41 (d, *J* = 2.0 Hz, 1H), 7.94 (d, *J* = 8.0 Hz, 3H), 7.52 (d, *J* = 7.9 Hz, 2H), 7.27 (d, *J* = 5.0 Hz, 1H), 7.04 (dd, J = 10.4, 5.3 Hz, 2H), 4.48 (s, 2H), 3.78 - 3.63 (m, 4H), 3.24 (t, *J* = 4.0 Hz, 2H), 2.83 - 2.75 (m, 4H), 2.69 (m, 2H), 1.40-1.34 (m, 2H), 1.20 - 1.10 (m, 2H).

### Example 2 Preparation of 1²-fluoro-4⁴-cyclopropyl-6-oxa-3,11-diaza-2 (4,2)-pyrimidina-1(1,4),4(1,3)-dibenzenacyclododecaphan-12-one(A2)

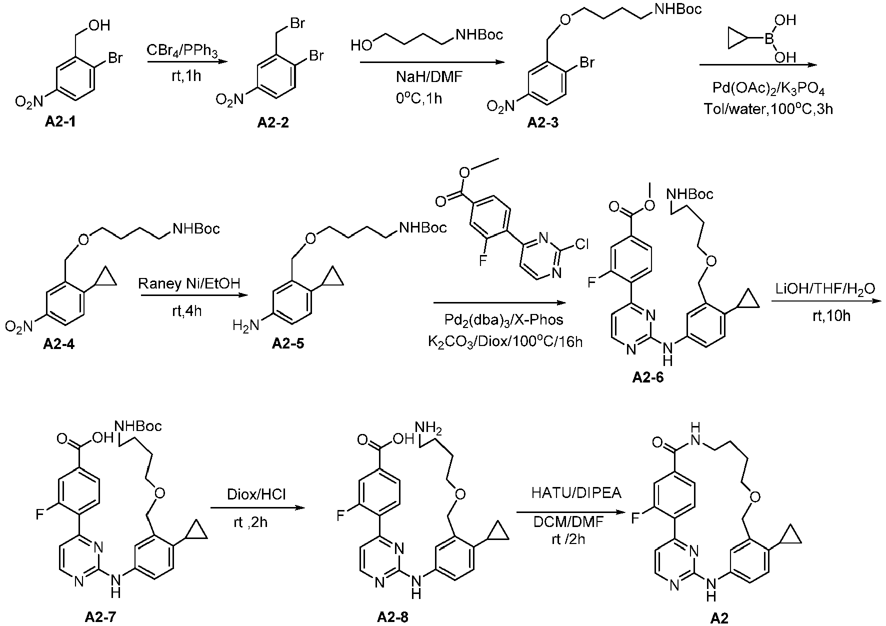

### Step 1: 1-bromo-2-bromomethyl-4-nitrobenzene(A2-2)

2-Bromo-5-nitrobenzyl alcohol(**A2-1**, 928 mg, 4.0mmol) and triphenylphosphine (1.98g, 6.0mmol) were added to DCM(40ml), stirred for 10min, cooled to 0°C under an ice bath, and a solution of carbon tetrabromide (1.57g,6.0mmol) in DCM (5ml) was added dropwise. After the addition, the reaction solution was stirred at room temperature for 2h. After TLC confirmed that the reaction was completed, DCM was concentrated, and the residue was purified by column chromatography to obtain compound **A2-2** (647 mg, yield 55%) as a yellow solid. ¹H NMR (400 MHz, CDCl₃): 8.36 (s, 1H), 8.05 (d, *J* = 8.8 Hz, 1H), 7.81 (d, *J* = 8.8 Hz, 1H), 4.66 (s, 2H).

### Step 2: tert-butyl 4-((2-bromo-5-nitro) benzyloxy) butylamino-1-carboxylate (A2-3)

Under nitrogen protection, 4-(N-tert-butoxycarbonylamino)-1-butanol (547mg, 2.9mmol) was added to anhydrous DMF(10 ml), cooled to 0°C under an ice bath, sodium hydride (132mg, 3.3mmol, 60%) was added, and continued to stir under ice bath for 30min. Then a solution of compound **A2 -2 (647** mg, 2.2mmol) in DMF(5ml) was slowly added, and continued to stir for 1h under an ice bath. After TLC confirmed that the reaction was completed, saturated ammonium chloride solution was added to quench under an ice bath, extracted with EA, washed with deionized water for three times, and dried over anhydrous sodium sulfate. EA solution was concentrated, and the residue was purified by column chromatography to obtain **A2-3** (440 mg, yield 50%). MS (ESI) m/z: calcd 347.08 (M + H-56), found 346.90.

### Step 3: tert-butyl 4-((2-cyclopropyl-5-nitro) benzyloxy) butylamino-1-carboxylate (A2-4)

Under nitrogen protection, compound **A2-3**(400 mg,1.0 mmol) and cyclopropylboric acid (129 mg, 1.5 mmol) were dissolved in toluene (16 mL) and water (2ml), then potassium phosphate (637 mg, 3.0 mmol), tricyclohexylphosphine tetrafluoroborate (147.3 mg, 0.40mmol) and palladium acetate (45 mg,0.20 mmol) were added, and the reaction solution was heated to 100°C and stirred for 3 hours. After TLC confirmed that the reaction was completed, toluene was concentrated, the solution was extracted with EA, and the organic phase was washed with deionized water for three times, and dried over anhydrous sodium sulfate. EA was concentrated, and the residue was purified by column chromatography to obtain compound **A2-4**(360 mg, yield 98%) as a light yellow oil. MS (ESI) m/z: calcd 365.20 (M + H), found 365.14.

### Step 4: 1²-fluoro-4⁴-cyclopropyl-6-oxa-3,11-diaza-2 (4,2)-pyrimidina-1(1,4),4(1,3)-dibenzenacyclododecaphan-12-one(A2)

Compound **A2**(30 mg) as a yellow solid was obtained from **A2-4** through a five-step reaction (similar to compound **A1**). MS (ESI) m/z: calcd 433.20 (M+H), found 433.30; ¹H NMR (400 MHz, DMSO-d6) δ 9.64 (s, 1H), 8.58 (d, *J* = 5.0 Hz, 1H), 8.22 (d, *J* = 2.3 Hz, 1H), 8.07-8.03 (m, 1H), 7.88-7.84 (m, 1H), 7.41-7.37 (m, 2H), 7.16 (dd, *J* = 5.0, 2.1 Hz, 1H), 6.97 (dd, *J* = 8.3, 2.3 Hz, 1H), 6.87 (d, *J* = 8.3 Hz, 1H), 4.55 (s, 2H), 3.23 (t, *J* = 6.4 Hz, 2H), 2.81-2.75 (m, 2H), 1.86 - 1.77 (m, 1H), 1.38-1.32 (m, 2H), 1.25-1.22 (m, 2H), 0.88 -0.77 (m, 2H), 0.57-0.53 (m, 2H).

Examples 3-94 were obtained by referring to the experimental steps of Examples 1 and 2 using different starting materials, as shown in Table 1 below.

**Table 1**

| Example | Structure | MS (calc) [M + H]⁺ MS (found) | Name |
|---|---|---|---|
| **3** | 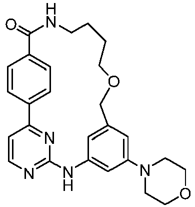 | 460.23/460.10 | 4⁵-morpholin-6-oxa-3,11-diaza-2(4, 2)-pyrimidina-1(1,4),4(1,3)-dibenze nacyclododecaphan-12-one |
| **4** | 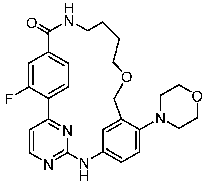 | 478.23/478.07 | 1²-fluoro-4⁴-morpholin-6-oxa-3,11-diaza-2(4,2)-pyrimidina-1(1,4),4(1, 3)-dibenzenacyclododecaphan-12-o ne |
| **5** | 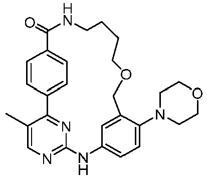 | 474.25/474.38 | 2⁵-methyl-4⁴-morpholin-6-oxa-3,11 -diaza-2(4,2)-pyrimidina-1(1,4),4(1, 3)-dibenzenacyclododecaphan-12-o ne |
| **6** | 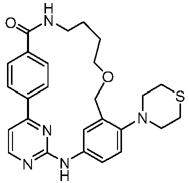 | 476.21/476.46 | 4⁴-thiomorpholin-6-oxa-3,11-diaza-2(4,2)-pyrimidina-1(1,4),4(1,3)-dib enzenacyclododecaphan-12-one |
| **7** | 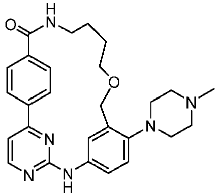 | 473.27/473.65 | 4⁴-(4-methylpiperazin-1-)-6-oxa-3,1 1-diaza-2(4,2)-pyrimidina-1(1,4),4( 1,3)-dibenzenacyclododecaphan-12-one |
| **8** | 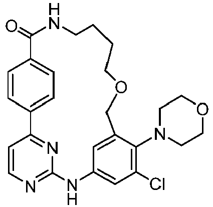 | 494.20/494.12 | 4⁵-chloro-4⁴-morpholin-6-oxa-3,11-diaza-2(4,2)-pyrimidina-1(1,4),4(1, 3)-dibenzenacyclododecaphan-12-o ne |
| **9** | 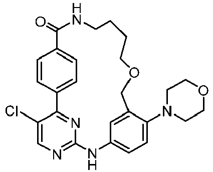 | 494.20/494.36 | 2⁵-chloro-4⁴-morpholin-6-oxa-3,11-diaza-2(4,2)-pyrimidina-1(1,4),4(1, 3)-dibenzenacyclododecaphan-12-o ne |
| **10** | 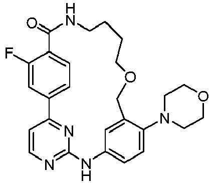 | 478.23/478.62 | 1³-fluoro-4⁴-morpholin-6-oxa-3,11-diaza-2(4,2)-pyrimidina-1(1,4),4(1, 3)-dibenzenacyclododecaphan-12-o ne |
| **11** | 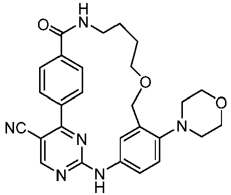 | 485.23/485.38 | 2⁵-cyano-4⁴-morpholin-6-oxa-3,11-diaza-2(4,2)-pyrimidina-1(1,4),4(1, 3)-dibenzenacyclododecaphan-12-o ne |
| **12** | 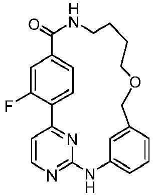 | 393.17/393.35 | 1²-fluoro-6-oxa-3,11-diaza-2(4,2)-p yrimidina-1(1,4),4(1,3)-dibenzenac yclododecaphan-12-one |
| **13** | 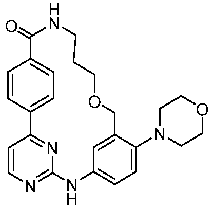 | 446.22/446.29 | 4⁴-morpholin-6-oxa-3,11-diaza-2(4, 2)-pyrimidina-1(1,4),4(1,3)-dibenze nacycloundecaphan-11-one |
| **14** | 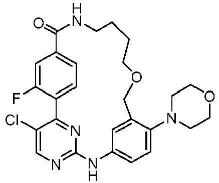 | 512.19/512.17 | 2⁵-chloro-1²-fluoro-4⁴-morpholin-6-oxa-3,11-diaza-2(4,2)-pyrimidina-1 (1,4),4(1,3)-dibenzenacyclododecap han-12-one |
| **15** | 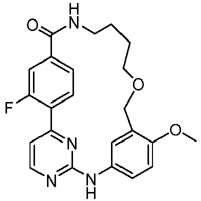 | 423.18/423.35 | 1²-fluoro-4⁴-methoxy-6-oxa-3,11-di aza-2(4,2)-pyrimidina-1(1,4),4(1,3)-dibenzenacyclododecaphan-12-one |
| **16** | 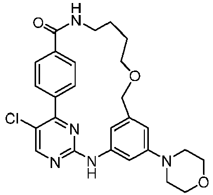 | 494.20/494.08 | 2⁵-chloro-4⁵-morpholin-6-oxa-3,11-diaza-2(4,2)-pyrimidina-1(1,4),4(1, 3)-dibenzenacyclododecaphan-12-o ne |
| **17** | 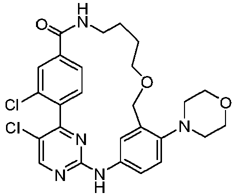 | 528.16/528.12 | 1²,2⁵-dichloro-4⁴-morpholin-6-oxa-3,11-diaza-2(4,2)-pyrimidina-1(1,4) ,4(1,3)-dibenzenacyclododecaphan-12-one |
| **18** | 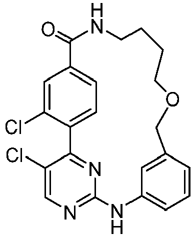 | 443.10/443.02 | 1²,2⁵-dichloro-6-oxa-3,11-diaza-2(4 ,2)-pyrimidina-1(1,4),4(1,3)-dibenz enacyclododecaphan-12-one |
| **19** | 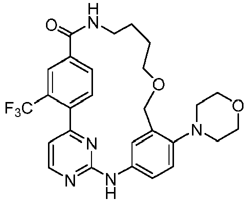 | 528.22/528.32 | 4⁴-morpholin-1²-trifluoromethyl-6-oxa-3,11-diaza-2(4,2)-pyrimidina-1 (1,4),4(1,3)-dibenzenacyclododecap han-12-one |
| **20** | 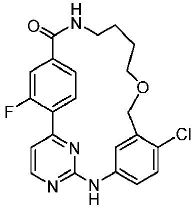 | 427.13/427.09 | 4⁴-chloro-1²-fluoro-6-oxa-3,11-diaz a-2(4,2)-pyrimidina-1(1,4),4(1,3)-di benzenacyclododecaphan-12-one |
| **21** | 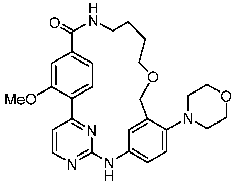 | 490.25/490.43 | 1²-methoxy-4⁴-morpholin-6-oxa-3,1 1-diaza-2(4,2)-pyrimidina-1(1,4),4( 1,3)-dibenzenacyclododecaphan-12-one |
| **22** | 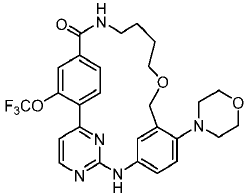 | 544.22/544.44 | 4⁴-morpholin-1²-trifluoromethoxy-6 -oxa-3,11-diaza-2(4,2)-pyrimidina-1(1,4),4(1,3)-dibenzenacyclododeca phan-12-one |
| **23** | 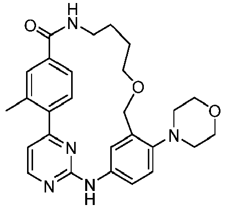 | 474.25/474.57 | 1²-methyl-4⁴-morpholin-6-oxa-3,11 -diaza-2(4,2)-pyrimidina-1(1,4),4(1, 3)-dibenzenacyclododecaphan-12-o ne |
| **24** | 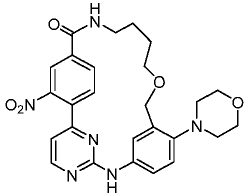 | 505.22/505.52 | 1²-nitro-4⁴-morpholin-6-oxa-3,11-di aza-2(4,2)-pyrimidina-1(1,4),4(1,3)-dibenzenacyclododecaphan-12-one |
| **25** | 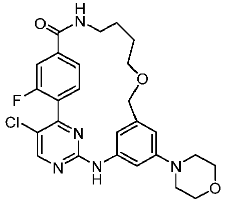 | 512.19/512.23 | 1²-fluoro-2⁵-chloro-4⁴-morpholin-6-oxa-3,11-diaza-2(4,2)-pyrimidina-1 (1,4),4(1,3)-dibenzenacyclododecap han-12-one |
| **26** | 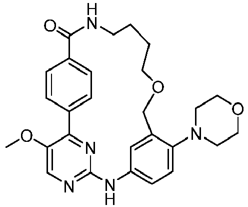 | 490.25/490.38 | 2⁵-methoxy-4⁴-morpholin-6-oxa-3,1 1-diaza-2(4,2)-pyrimidina-1(1,4),4( 1,3)-dibenzenacyclododecaphan-12-one |
| **27** | 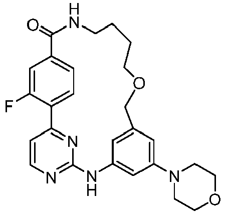 | 478.23/478.53 | 1²-fluoro-4⁵-morpholin-6-oxa-3,11-diaza-2(4,2)-pyrimidina-1(1,4),4(1, 3)-dibenzenacyclododecaphan-12-o ne |
| **28** | 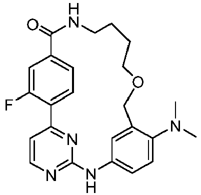 | 536.21/436.64 | 1²-fluoro-4⁴-dimethylamino-6-oxa-3,11-diaza-2(4,2)-pyrimidina-1(1,4) ,4(1,3)-dibenzenacyclododecaphan-12-one |
| **29** | 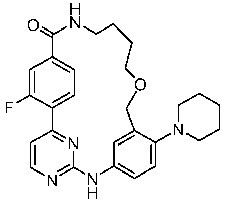 | 476.25/476.38 | 1²-fluoro-4⁴-(piperidin-1-)-6-oxa-3, 11-diaza-2(4,2)-pyrimidina-1(1,4),4 (1,3)-dibenzenacyclododecaphan-12 -one |
| **30** | 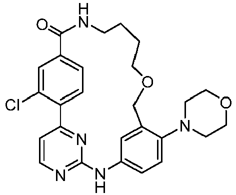 | 494.20/494.21 | 1²-chloro-4⁴-morpholin-6-oxa-3,11-diaza-2(4,2)-pyrimidina-1(1,4),4(1, 3)-dibenzenacyclododecaphan-12-o ne |
| **31** | 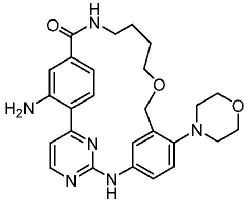 | 475.25/475.22 | 1²-amino-4⁴-morpholin-6-oxa-3,11-diaza-2(4,2)-pyrimidina-1(1,4),4(1, 3)-dibenzenacyclododecaphan-12-o ne |
| **32** | 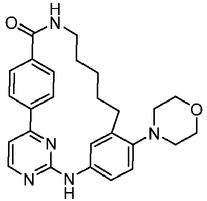 | 444.24/444.54 | 4⁴-morpholin-3,11-diaza-2(4,2)-pyri midina-1(1,4),4(1,3)-dibenzenacycl ododecaphan-11-one |
| **33** | 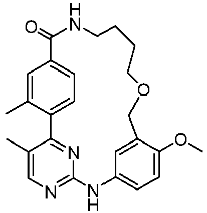 | 433.22/433.51 | 1²,2⁵-dimethyl-4⁴-methoxy-6-oxa-3, 11-diaza-2(4,2)-pyrimidina-1(1,4),4 (1,3)-dibenzenacyclododecaphan-12 -one |
| **34** | 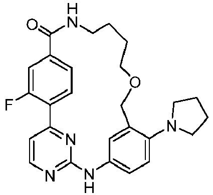 | 462.23/462.47 | 1²-fluoro-4⁴-(pyrrolidin-1-)-6-oxa-3 ,11-diaza-2(4,2)-pyrimidina-1(1,4), 4(1,3)-dibenzenacyclododecaphan-1 2-one |
| **35** | 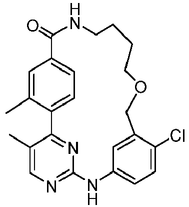 | 437.17/438.38 | 1²,2⁵-dimethyl-4⁴-chloro-6-oxa-3,11 -diaza-2(4,2)-pyrimidina-1(1,4),4(1, 3)-dibenzenacyclododecaphan-12-o ne |
| **36** | 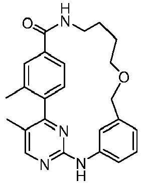 | 403.21/403.65 | 1²,2⁵-dimethyl-6-oxa-3,11-diaza-2( 4,2)-pyrimidina-1(1,4),4(1,3)-diben zenacyclododecaphan-12-one |
| **37** | 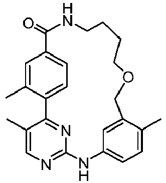 | 417.23/417.52 | 1²,2⁵,4⁴-trimethyl-6-oxa-3,11-diaza-2(4,2)-pyrimidina-1(1,4),4(1,3)-dib enzenacyclododecaphan-12-one |
| **38** | 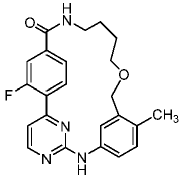 | 407.19/407.55 | 1²-fluoro-4⁴-methyl-6-oxa-3,11-diaz a-2(4,2)-pyrimidina-1(1,4),4(1,3)-di benzenacyclododecaphan-12-one |
| **39** | 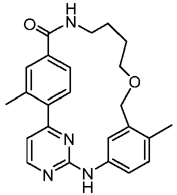 | 403.21/403.55 | 1²,4⁴-dimethyl-6-oxa-3,11-diaza-2( 4,2)-pyrimidina-1(1,4),4(1,3)-diben zenacyclododecaphan-12-one |
| **40** | 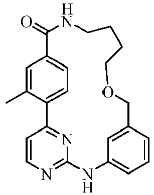 | 389.20/389.50 | 1²-methyl-6-oxa-3,11-diaza-2 (4,2)-pyrimidina-1(1,4),4(1,3)-dibe nzenacyclododecaphan-12-one |
| **41** | 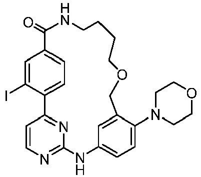 | 586.13/586.00 | 1²-iodine-4⁴-morpholin-6-oxa-3,11-diaza-2(4,2)-pyrimidina-1(1,4),4(1, 3)-dibenzenacyclododecaphan-12-o ne |
| **42** | 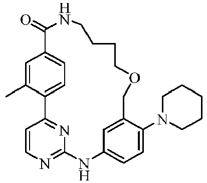 | 472.27/472.79 | 1²-methyl-4⁴-(piperidin-1-)-6-oxa-3, 11-diaza-2(4,2)-pyrimidina-1(1,4),4 (1,3)-dibenzenacyclododecaphan-12 -one |
| **43** | 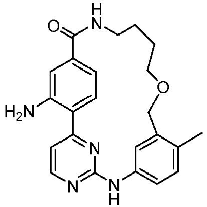 | 404.21/404.17 | 1²-amino-4⁴-methyl-6-oxa-3,11-dia za-2(4,2)-pyrimidina-1(1,4),4(1,3)-dibenzenacyclododecaphan-12-one |
| **44** | 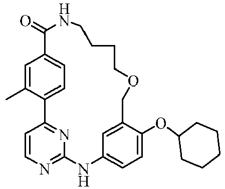 | 487.27/487.58 | 1²-methyl-4⁴-cyclohexyloxy-6-oxa-3,11-diaza-2(4,2)-pyrimidina-1(1,4) ,4(1,3)-dibenzenacyclododecaphan-12-one |
| **45** | 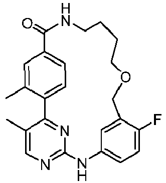 | 421.20/421.48 | 1²,2⁵-dimethyl-4⁴-fluoro-6-oxa-3,11 -diaza-2(4,2)-pyrimidina-1(1,4),4(1, 3)-dibenzenacyclododecaphan-12-o ne |
| **46** | 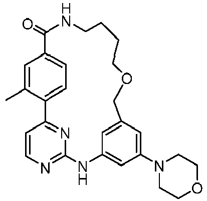 | 474.25/474.35 | 1²-methyl-4⁴-morpholin-6-oxa-3,11 -diaza-2(4,2)-pyrimidina-1(1,4),4(1, 3)-dibenzenacyclododecaphan-12-o ne |
| **47** | 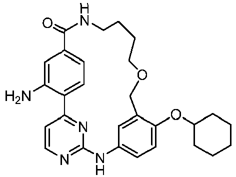 | 488.27/488.44 | 1²-amino-4⁴-cyclohexyloxy-6-oxa-3,11 -diaza-2(4,2)-pyrimidina-1(1,4),4(1, 3)-dibenzenacyclododecaphan-12-o ne |
| **48** | 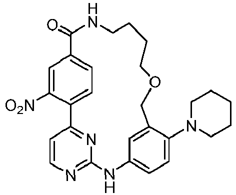 | 503.24/503.29 | 1²-nitro-4⁴-(piperidin-1-)-6-oxa-3,1 1-diaza-2(4,2)-pyrimidina-1(1,4),4( 1,3)-dibenzenacyclododecaphan-12-one |
| **49** | 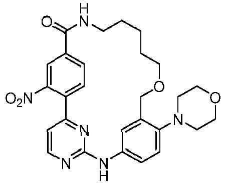 | 519.24/519.16 | 1²-nitro-4⁴-morpholin-6-oxa-3,12-di aza-2(4,2)-pyrimidina-1(1,4),4(1,3)-dibenzenacyclotridecaphan-13-one |
| **50** | 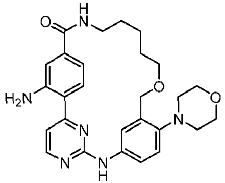 | 489.26/489.62 | 1²-amino-4⁴-morpholin-6-oxa-3,12-diaza-2(4,2)-pyrimidina-1(1,4),4(1, 3)-dibenzenacyclotridecaphan-13-o ne |
| **51** | 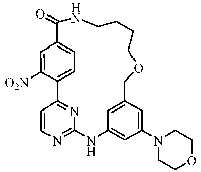 | 505.22/505.41 | 1²-nitro-4⁵-morpholin-6-oxa-3,11-di aza-2(4,2)-pyrimidina-1(1,4), 4(1,3)-dibenzenacyclododecaphan-12-one |
| **52** | 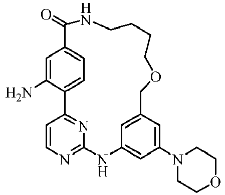 | 475.25/475.18 | 1²-amino-4⁵-morpholin-6-oxa-3,11-diaza-2(4,2)-pyrimidina-1(1,4),4(1, 3)-dibenzenacyclododecaphan-12-o ne |
| **53** | 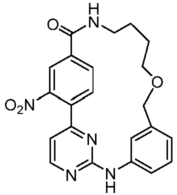 | 420.17/420.13 | 1²-nitro-6-oxa-3,11-diaza-2(4,2)-py rimidina-1(1,4),4(1,3)-dibenzenacyc lododecaphan-12-one |
| **54** | 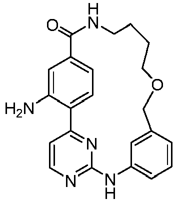 | 390.19/390.31 | 1²-amino-6-oxa-3,11-diaza-2(4,2)-p yrimidina-1(1,4),4(1,3)-dibenzenac yclododecaphan-12-one |
| **55** | 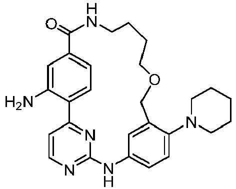 | 473.27/473.30 | 1²-amino-4⁴-(piperidin-1-)-6-oxa-3, 11-diaza-2(4,2)-pyrimidina-1(1,4),4 (1,3)-dibenzenacyclododecaphan-12 -one |
| **56** | 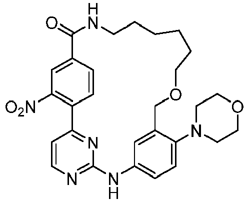 | 533.25/533.24 | 1²-nitro-4⁴-morpholin-6-oxa-3,13-di aza-2(4,2)-pyrimidina-1(1,4),4(1,3)-dibenzenacyclotetradecaphan-14-on e |
| **57** | 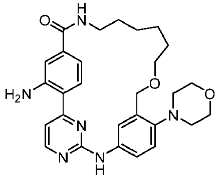 | 503.28/503.39 | 1²-amino-4⁴-morpholin-6-oxa-3,13-diaza-2(4,2)-pyrimidina-1(1,4),4(1, 3)-dibenzenacyclotetradecaphan-14 -one |
| **58** | 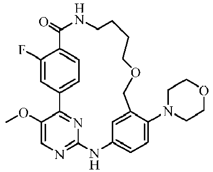 | 508.24/508.42 | 1³-fluoro-2⁵-methoxy-4⁴-morpholin -6-oxa-3,11-diaza-2(4,2)-pyrimidin a-1(1,4),4(1,3)-dibenzenacyclodode caphan-12-one |
| **59** | 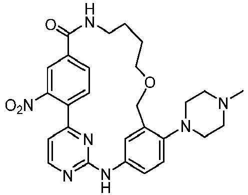 | 518.25/518.36 | 1²-nitro-4⁴-(4-methylpiperazin-1-)-6 -oxa-3,11-diaza-2(4,2)-pyrimidina-1(1,4),4(1,3)-dibenzenacyclododeca phan-12-one |
| **60** | 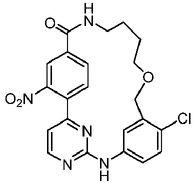 | 454.13/453.94 | 1²-nitro-4⁴-chloro-6-oxa-3,11-diaza-2(4,2)-pyrimidina-1(1,4),4(1,3)-dib enzenacyclododecaphan-12-one |
| **61** | 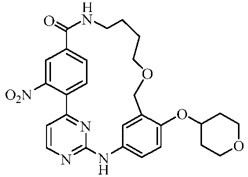 | 520.22/520.12 | 1²-nitro-4⁴-(2*H*-tetrahydropyran-4-o xo)-6-oxa-3,11-diaza-2(4,2)-pyrimi dina-1(1,4),4(1,3)-dibenzenacyclod odecaphan-12-one |
| **62** | 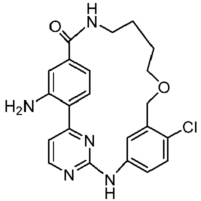 | 424.15/424.03 | 1²-amino-4⁴-chloro-6-oxa-3,11-diaz a-2(4,2)-pyrimidina-1(1,4),4(1,3)-di benzenacyclododecaphan-12-one |
| **63** | 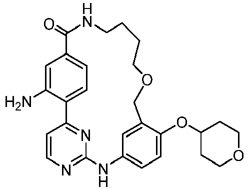 | 490.25/490.32 | 1²-amino-4⁴-(4-tetrahydropyranoxy) -6-oxa-3,11-diaza-2(4,2)-pyrimidin a-1(1,4),4(1,3)-dibenzenacyclodode caphan-12-one |
| **64** | 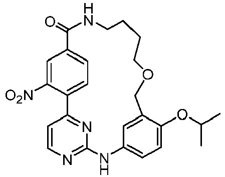 | 478.21/478.18 | 4⁴-isopropoxy-1²-nitro-6-oxa-3,11-diaza-2(4,2)-pyrimidina-1(1,4),4(1, 3)-dibenzenacyclododecaphan-12-o ne |
| **65** | 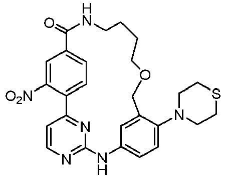 | 521.20/521.15 | 1²-nitro-4⁴-thiomorpholin-6-oxa-3, 11-diaza-2(4,2)-pyrimidina-1(1,4),4 (1,3)-dibenzenacyclododecaphan-12 -one |
| **66** | 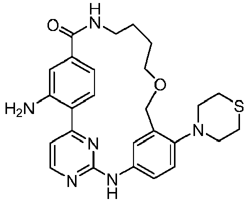 | 491.22/491.31 | 1²-amino-4⁴-thiomorpholin-6-oxa-3 ,11-diaza-2(4,2)-pyrimidina -1(1,4),4(1,3)-dibenzenacyclododec aphan-12-one |
| **67** | 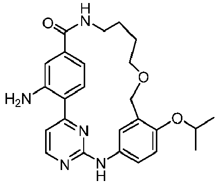 | 448.23/448.35 | 1²-amino-4⁴-isopropoxy-6-oxa-3,11 -diaza-2(4,2)-pyrimidina-1(1,4),4(1, 3)-dibenzenacyclododecaphan-12-o ne |
| **68** | 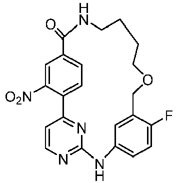 | 438.16/438.31 | 4⁴-fluoro-1²-nitro-6-oxa-3,11-diaza-2(4,2)-pyrimidina-1(1,4),4(1,3)-dib enzenacyclododecaphan-12-one |
| **69** | 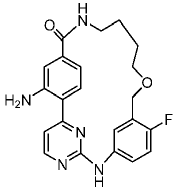 | 408.18/408.23 | 1²-amino-4⁴-fluoro-6-oxa-3,11-diaz a-2(4,2)-pyrimidina-1(1,4),4(1,3)-di benzenacyclododecaphan-12-one |
| **70** | 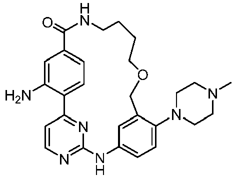 | 488.28/488.25 | 1²-amino-4⁴-(4-methylpiperazin-1-y 1)-6-oxa-3,11-diaza-2(4,2)-pyrimidi na-1(1,4),4(1,3)-dibenzenacyclodod ecaphan-12-one |
| **71** | 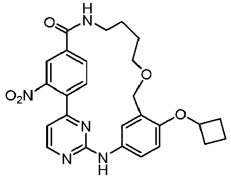 | 490.21/490.18 | 4⁴-cyclobutoxy-1²-nitro-6-oxa-3,11-diaza-2(4,2)-pyrimidina-1(1,4),4(1, 3)-dibenzenacyclododecaphan-12-o ne |
| **72** | 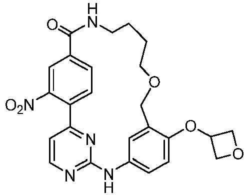 | 492.19/492.23 | 1²-nitro-4⁴-(oxetan-3-oxy)-6-oxa-3, 11-diaza-2(4,2)-pyrimidina-1(1,4),4 (1,3)-dibenzenacyclododecaphan-12 -one |
| **73** | 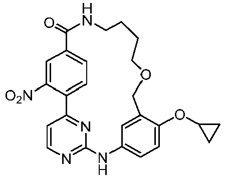 | 476.19/476.26 | 4⁴-cyclopropoxy-1²-nitro-6-oxa-3,1 1-diaza-2(4,2)-pyrimidina-1(1,4),4( 1,3)-dibenzenacyclododecaphan-12-one |
| **74** | 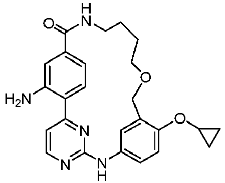 | 446.22/446.28 | 1²-amino-4⁴-cyclopropoxy-6-oxa-3, 11-diaza-2(4,2)-pyrimidina-1(1,4),4 (1,3)-dibenzenacyclododecaphan-12 -one |
| **75** | 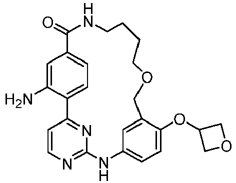 | 462.53/462.61 | 1²-amino-4⁴-(oxetan-3-oxy)-6-oxa-3,11-diaza-2(4,2)-pyrimidina-1(1,4) ,4(1,3)-dibenzenacyclododecaphan-12-one |
| **76** | 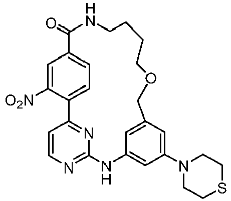 | 521.20/521.25 | 1²-nitro-4⁵-thiomorpholin-6-oxa-3, 11-diaza-2(4,2)-pyrimidina-1(1,4),4 (1,3)-dibenzenacyclododecaphan-12 -one |
| **77** | 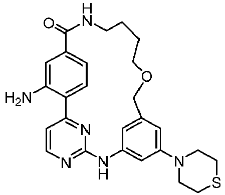 | 491.22/491.16 | 1²-amino-4⁵-thiomorpholin-6-oxa-3 ,11-diaza-2(4,2)-pyrimidina-1(1,4), 4(1,3)-dibenzenacyclododecaphan-1 2-one |
| **78** | 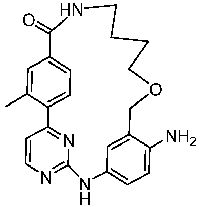 | 404.21/404.29 | 4⁴-amino-1²-methyl-6-oxa-3,11-dia za-2(4,2)-pyrimidina-1(1,4),4(1,3)-dibenzenacyclododecaphan-12-one |
| **79** | 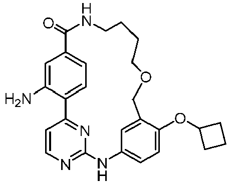 | 460.23/460.31 | 1²-amino-4⁴-cyclobutoxy-6-oxa-3,1 1-diaza-2(4,2)-pyrimidina-1(1,4),4( 1,3)-dibenzenacyclododecaphan-12-one |
| **80** | 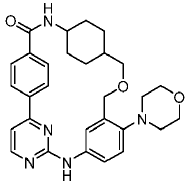 | 500.27/500.39 | 4⁴-morpholine-6-oxa-3,9-diaza-2(4, 2)-pyrimidina-1(1,4),4(1,3)-dibenze na-8 (1,4)-cyclododecaphan-10-one |
| **81** | 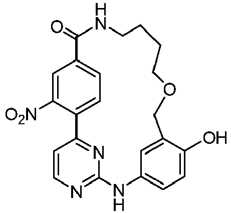 | 436.16/436.03 | 4⁴-hydroxy-1²-nitro-6-oxa-3,11-diaz a-2(4,2)-pyrimidina-1(1,4),4(1,3)-di benzenacyclododecaphan-12-one |
| **82** | 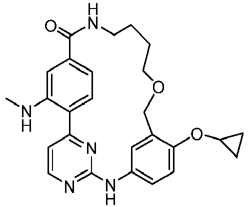 | 460.24/460.30 | 4⁴-cyclopropoxy-1²-methylamino-6 -oxa-3,11-diaza-2(4,2)-pyrimidina-1(1,4),4(1,3)-dibenzenacyclododeca phan-12-one |
| **83** | 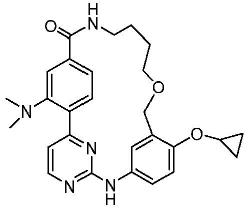 | 474.25/474.20 | 4⁴-cyclopropoxy-1²-dimethylamino-6-oxa-3,11-diaza-2(4,2)-pyrimidina -1(1,4),4(1,3)-dibenzenacyclododec aphan-12-one |
| **84** | 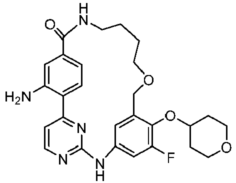 | 508.24/508.30 | 1²-amino-4⁵-fluoro-4⁴-((tetrahydro-2*H*-pyran-4-yl)oxy)-6-oxa-3,11-dia za-2(4,2)-pyrimidina-1(1,4),4(1,3)-dibenzenacyclododecaphan-12-one |
| **85** | 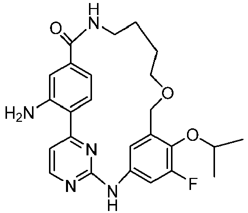 | 466.22/466.20 | 1²-amino-4⁵-fluoro-4⁴-isopropoxy-6 -oxa-3,11-diaza-2(4,2)-pyrimidina-1(1,4),4(1,3)-dibenzenacyclododeca phan-12-one |
| **86** | 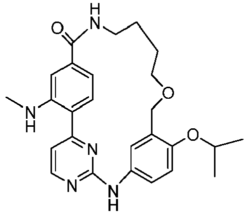 | 462.24/462.20 | 4⁴-isopropoxy-1²-methylamino-6-o xa-3,11-diaza-2(4,2)-pyrimidina-1( 1,4),4(1,3)-dibenzenacyclododecap han-12-one |
| **87** | 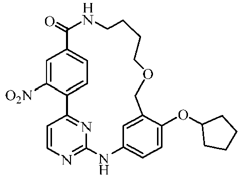 | 504.23/504.20 | 4⁴-cyclopentoxy-1²-nitro-6-oxa-3,1 1-diaza-2(4,2)-pyrimidina-1(1,4),4( 1,3)-dibenzenacyclododecaphan-12-one |
| **88** | 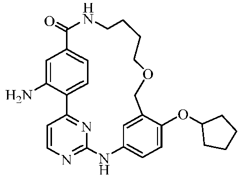 | 474.25/474.30 | 1²-amino-4⁴-cyclopentoxy-6-oxa-3, 11-diaza-2(4,2)-pyrimidina-1(1,4),4 (1,3)-dibenzenacyclododecaphan-12 -one |
| 89 | 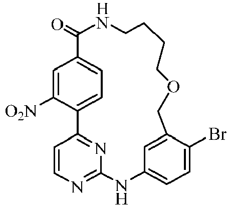 | 498.08,500.08/498. 80,500.80 | 4⁴-bromo-1²-nitro-6-oxa-3,11-diaza -2(4,2)-pyrimidina-1(1,4),4(1,3)-dib enzenacyclododecaphan-12-one |
| **90** | 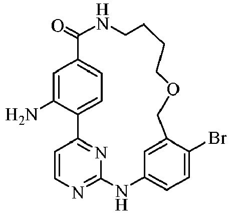 | 468.10,470.10/467. 70,469.40 | 1²-amino-4⁴-bromo-6-oxa-3,11-diaz a-2(4,2)-pyrimidina-1(1,4),4(1,3)-di benzenacyclododecaphan-12-one |
| **91** | 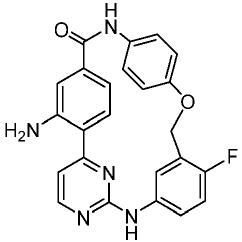 | 428.15/427.90 | 1²-amino-4⁴-fluoro-6-oxa-3,8-diaza-2(4,2)-pyrimidina-1,7(1,4),4(1,3)-tri benzenacyclononaphane-9-one |
| **92** | 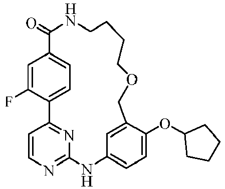 | 477.22/476.80 | 4⁴-cyclopentoxy-1²-fluoro-6-oxa-3, 11-diaza-2(4,2)-pyrimidina-1(1,4),4 (1,3)-dibenzenacyclododecaphan-12 -one |
| **93** | 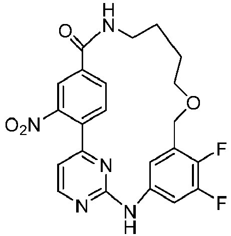 | 456.14/455.70 | 4⁴,4⁵-difluoro-1²-nitro-6-oxa-3,11-d iaza-2(4,2)-pyrimidina-1(1,4),4(1,3) -dibenzenacyclododecaphan-12-one |
| **94** | 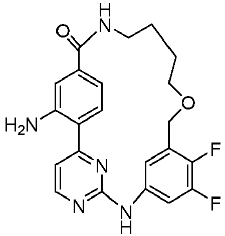 | 426.17/425.90 | 1²-amino-4⁴,4⁵-difluoro-6-oxa-3,11-diaza-2(4,2)-pyrimidina-1(1,4),4(1, 3)-dibenzenacyclododecaphan-12-o ne |

### Example 95 Preparation of 12-amino-44-cyclopropoxy-8-methyl-6-oxa-3,11-diaza-2 (4,2)-pyrimidina -1(1,4),4(1,3)-dibenzenacyclododecaphan-12-one(95)

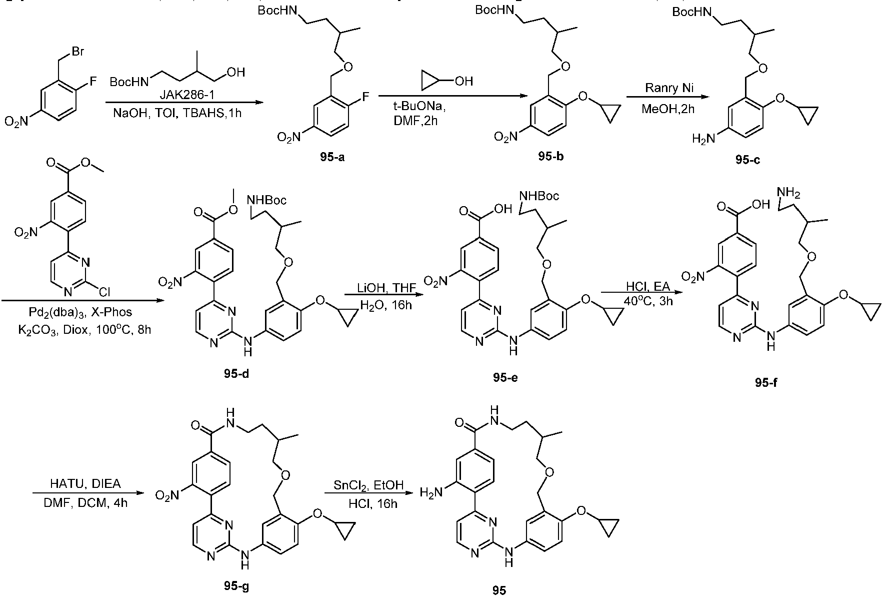

### Step 1:tert-butyl (4-((2-fluoro-5-nitrobenzyl) oxy)-3-methylbutyl) carbamate (95-a)

2-Fluoro-5-nitrobenzyl bromide (660mg,2.82mmol) was dissolved in Tol(15mL), 50% sodium hydroxide aqueous solution (15mL) was added to it, stirred for a while, then tert-butyl (4-hydroxy-3-methylbutyl) carbamate (800mg,3.95mmol) was added to the reaction solution, and reacted for 5-10min. TBAHS(130mg,0.394mmol) was weighed and added to the reaction solution, and reacted for 1h at room temperature. After TLC confirmed that the reaction was completed, EA and water were added for extraction, washed with deionized water, dried over anhydrous sodium sulfate. EA was concentrated, and the residue was purified by column chromatography to obtain tert-butyl (4-((2-fluoro-5-nitrobenzyl) oxy)-3-methylbutyl) carbamate (**95-a**, 440mg, yield 44%) as a yellow oil. ¹H NMR (400 MHz, CDCl₃): δ 8.39 (d, J = 3.0 Hz, 1H), 8.25 - 8.17 (m, 1H), 7.21 (t, J = 8.8 Hz, 1H), 4.62 (s, 2H), 3.46 - 3.40 (m, 2H), 3.27 - 3.12 (m, 2H), 1.92 - 1.85 (m, 1H), 1.68 - 1.61 (m, 2H), 1.46 (s, 9H), 1.02 (d, J = 6.7 Hz, 3H).

### Step 2: tert-butyl (4-((2-cyclopropoxy-5-nitrobenzyl) oxy)-3-methylbutyl) carbamate (95-b)

Tert-butyl (4-((2-fluoro-5-nitrobenzyl) oxy)-3-methylbutyl) carbamate (**95-a**, 440mg,1.24mmol), and cyclopropanol (432mg,7.44mmol) were dissolved in DMF(10mL), stirred for a while, sodium tert-butanol (594mg,6.18mmol) was slowly added under ice bath, and transferred to room temperature to react for 2 hours after addition. After TLC confirmed that the reaction was completed, EA and water were added for extraction, washed with deionized water, dried over anhydrous sodium sulfate. EA was concentrated to obtain tert-butyl (4-((2-cyclopropoxy-5-nitrobenzyl) oxy)-3-methylbutyl) carbamate (**95-b**, 400mg, yield 80%) as a yellow oil which was directly used in the next reaction.

### Step 3: tert-butyl (4-((5-amino-2-cyclopropyloxybenzyl) oxy)-3-methylbutyl) carbamate (95-c)

Tert-butyl (4-((2-cyclopropoxy-5-nitrobenzyl) oxy)-3-methylbutyl) carbamate (**95-b**, 400mg, 1.01mmol) was dissolved in methanol (20mL), and an appropriate amount of Raney Ni was added under the protection of H₂ and reacted at room temperature for 2h. After TLC confirmed that the reaction was completed, the reaction solution was filtered by suction filtration, the filtrate was concentrated, and the residue was purified by column chromatography to obtain tert-butyl (4-((5-amino-2-cyclopropyloxybenzyl) oxy)-3-methylbutyl) carbamate (**95-c**, 300mg, yield 81%) as a colorless oil. MS (ESI) m/z: calcd 365.24 (M+H), found 365.30.

### Step 4: methyl 4-(2-((3-((4-((tert-butoxycarbonyl) amino)-2-methylbutoxy) methyl)-4-cyclopropoxyphenyl) aminopyrimidin-4-yl)-3-nitrobenzoate (95-d)

Under nitrogen protection, methyl 4-(2-chloropyrimidin-4-yl)-3-nitrobenzoate (435mg,1.48mmol), and tert-butyl (4-((5-amino-2-cyclopropyloxybenzyl) oxy)-3-methylbutyl) carbamate (**95-c**, 300mg,0.822mmol) were added to 1,4-dioxane (20mL), tris(dibenzylideneacetone)dipalladium (150mg, 0.164mmol), 2-dicyclohexylphosphino-2,4,6-triisopropylbiphenyl (156mg, 0.328mmol), and potassium carbonate (227mg, 1.64mmol) were added and reacted at 100 °C for 8h. After TLC confirmed that the reaction was completed, 1,4-dioxane was concentrated, and deionized water and dichloromethane were added for liquid separation. The organic phase was washed with deionized water for three times, dried over anhydrous sodium sulfate, dichloromethane was concentrated, and the residue was purified by column chromatography to obtain methyl 4-(2-((3-((4-((tert-butoxycarbonyl) amino)-2-methylbutoxy) methyl)-4-cyclopropyoxyphenyl) aminopyrimidin-4-yl)-3-nitrobenzoate (**95-d**, 250mg, yield 49%) as a yellow solid. MS (ESI) m/z: calcd 622.28 (M+H), found 622.30;¹H NMR (400 MHz, CDCl₃) δ 8.61 - 8.54 (m, 1H), 8.54 - 8.44 (m, 1H), 8.33 (d, J = 7.8 Hz, 1H), 7.74 (d, J = 7.9 Hz, 1H), 7.56 (d, J = 8.3 Hz, 1H), 7.42 (s, 1H), 7.22 (d, J = 8.8 Hz, 1H), 7.15 (dd, J = 9.2, 5.3 Hz, 1H), 6.87 (d, J = 4.3 Hz, 1H), 4.49 (s, 2H), 4.03 (s, 3H), 3.58 - 3.48 (m, 1H), 3.39 - 3.31 (m, 2H), 3.20 (ddd, J = 8.3, 4.2, 2.3 Hz, 2H), 1.78 (s, 1H), 1.61 (dd, J = 5.5, 1.2 Hz, 2H), 1.45 (s, 9H), 0.96 (d, J = 6.8 Hz, 3H), 0.78 (dd, J = 8.5, 7.7 Hz, 4H).

### Step 5: 4-(2-((3-((4-((tert-butoxycarbonyl) amino)-2-methylbutoxy) methyl)-4-cyclopropyoxyphenyl) amino) pyrimidin-4-yl)-3-nitrobenzoic acid (95-e)

Methyl 4-(2-((3-((4-((tert-butoxycarbonyl) amino)-2-methylbutoxy) methyl)-4-cyclopropyoxyphenyl) aminopyrimidin-4-yl)-3-nitrobenzoate (**95-d**, 250mg, 0.403mmol) was added to a mixed solution of THF and water (10ml/3ml), then lithium hydroxide monohydrate (85mg, 2.01mmol) was added, stirred at room temperature for 16 hours. After TLC confirmed that the reaction was completed, 1M HCl was added to adjust the pH of the system to 5-6. The solvent was concentrated, EA and water were added for liquid extraction, washed with deionized water, and dried over anhydrous sodium sulfate. EA was concentrated to obtain 4-(2-((3-((4-((tert-butoxycarbonyl) amino)-2-methylbutoxy) methyl)-4-cyclopropyoxyphenyl) amino) pyrimidin-4-yl)-3-nitrobenzoic acid (**95-e,** 210mg, yield 86%) as a yellow solid which was directly used in the next reaction. MS (ESI) m/z: calcd 608.26 (M + H), found 608.30.

### Step 6: 4-(2-((3-((4-amino-2-methylbutoxy) methyl)-4-cycloproxyphenyl) amino) pyrimidin-4-yl)-3-nitrobenzoic acid (95-f)

4-(2-((3-((4-((Tert-butoxycarbonyl) amino)-2-methylbutoxy) methyl)-4-cyclopropyoxyphenyl) amino) pyrimidin-4-yl)-3-nitrobenzoic acid (**95-d**, 210mg, 0.345mmol) was added to EA(6ml), then hydrogen chloride/EA solution (3mol/L, 3ml) was added dropwise, stirred at 40 °C for 3 hours. After TLC confirmed that the reaction was completed, the solid was filtered out, and dried to obtain 4-(2-((3-((4-amino -2-methylbutoxy) methyl)-4-cyclopropoxyphenyl) amino) pyrimidin-4-yl)-3-nitrobenzoic acid (**95-f,** 100mg, yield 57%) as a yellow solid which was directly used in the next reaction. MS (ESI) m/z: calcd 508.21 (M + H), found 508.30.

### Step 7: 4⁴-cyclopropoxy-8-methyl-12-nitro-6-oxa-3, 11-diaza-2 (4,2)-pyrimidina-1(1,4),4(1,3)-dibenzenacyclododecaphan-12-one (95-g)

O-(7-Azabenzotriazol-1-yl)-N,N,N',N'-tetramethyluronium hexafluorophosphate (150mg, 0.394mmol) and N,N-diisopropylethylamine (102mg, 0.789mmol) were added to DCM(30ml) and stirred at room temperature. A mixed solution of 4-(2-((3-((4-amino -2-methylbutoxy) methyl)-4-cyclopropoxyphenyl) amino) pyrimidin-4-yl)-3-nitrobenzoic acid (**95-f**, 100mg, 0.197mmol) in DCM and DMF(10ml/5ml) was slowly added and kept for 0.5h, and the reaction solution was continued to be stirred at room temperature for 4h after addition. After LCMS confirmed that the reaction was completed, the reaction solution was concentrated, and the residue was purified by column chromatography to obtain 4⁴-cyclopropoxy-8-methyl-12-nitro-6-oxa-3, 11-diaza-2 (4,2)-pyrimidina-1(1,4),4(1,3)-dibenzenacyclododecaphan-12-one (**95-g,** 50mg, yield 52%) as a yellow solid. MS (ESI) m/z: calcd 490.20 (M+H), found 490.20;¹H NMR (400 MHz, DMSO-d6): δ 9.59 (s, 1H), 8.62 (d, J = 4.7 Hz, 1H), 8.17 (d, J = 15.7 Hz, 2H), 7.94 (s, 1H), 7.80 (d, J = 7.8 Hz, 2H), 7.19 (d, J = 4.6 Hz, 1H), 7.13 (d, J = 8.7 Hz, 1H), 7.05 (d, J = 6.4 Hz, 1H), 4.30 (q, J = 12.5 Hz, 2H), 3.79 (s, 1H), 3.13 (s, 1H), 2.93 (d, J = 7.4 Hz, 4H), 1.25 (s, 2H), 1.17 (s, 2H), 0.75 - 0.68 (m, 5H), 0.61 (s, 2H).

### Step 8: 1²-amino-4⁴-cyclopropoxy-8-methyl-6-oxa-3, 11-diaza-2 (4,2)-pyrimidina-1(1,4),4(1,3)-dibenzenacyclododecaphan-12-one (95)

Cyclopropoxy-8-methyl-12-nitro-6-oxa-3, 11-diaza-2 (4,2)-pyrimidina-1(1,4),4(1,3)-dibenzenacyclododecaphan-12-one (**95-g,** 50mg, 0.102mmol) was dissolved in ethanol (10mL), stannous chloride (185mg, 0.818mmol) and hydrogen chloride aqueous solution (0.5mL, 1mol/mL) were added, and reacted at room temperature for 16h. After TLC confirmed that the reaction was completed, ethanol was concentrated, saturated sodium bicarbonate aqueous solution was added to adjust the pH of the system to 8.0. EA and water were added for liquid extraction. The solution was washed with deionized water, dried over anhydrous sodium sulfate, EA was concentrated, purified by reverse phase chromatography, and freeze-dried to obtain 1²-amino-4⁴-cyclopropoxy-8-methyl-6-oxa-3, 11-diaza-2 (4,2)-pyrimidina-1(1,4),4(1,3)-dibenzenacyclododecaphan-12-one (**95**, 10mg, yield 22%) as a white solid. MS (ESI) m/z: calcd 460.23 (M+H), found 460.30; ¹H NMR (400 MHz, DMSO-d6): δ 9.45 (s, 1H), 8.51 (d, J = 4.7 Hz, 1H), 8.14 (s, 1H), 7.72 (s, 1H), 7.43 (d, J = 7.7 Hz, 1H), 7.16 (d, J = 8.6 Hz, 1H), 7.06 (s, 2H), 6.74 (s, 1H), 6.56 (d, J = 7.9 Hz, 1H), 5.93 (s, 2H), 4.38 (dd, J = 29.8, 14.2 Hz, 2H), 3.80 (s, 1H), 2.99 (d, J = 7.6 Hz, 1H), 2.74 (d, J = 50.1 Hz, 2H), 1.27 (d, J = 15.5 Hz, 4H), 0.78 (t, J = 27.1 Hz, 5H), 0.62 (s, 3H).

The following example compounds of **Table 2** were prepared according to the same method as the above example **95** by using the commercial compound or by referring to the preparation method of the intermediate compound shown.

**Table 2**

| Example | Structure | MS (calc) [M + H]⁺/ MS (found) | Name |
|---|---|---|---|
| **96** | 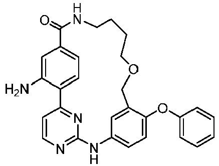 | 482.21/482.20 | 1²-amino-4⁴-phenoxy-6-oxa-3,11-diaza-2(4,2)-pyrimidina-1(1,4),4(1 ,3)-dibenzenacyclododecaphan-12 -one |
| 97 | 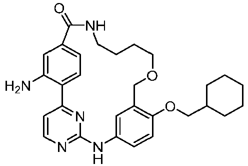 | 502.27/502.30 | 1²-amino-4⁴-(cyclohexylmethoxy) -6-oxa-3,11-diaza-2(4,2)-pyrimidi na-1(1,4),4(1,3)-dibenzenacyclodo decaphan-12-one |
| **98** | 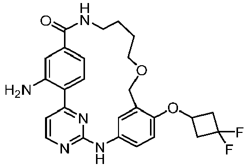 | 496.21/496.30 | 1²-amino-4⁴-(3,3-difluorocyclobut oxy)-6-oxa-3,11-diaza-2(4,2)-pyri midina-1(1,4),4(1,3)-dibenzenacyc lododecaphan-12-one |
| **99** | 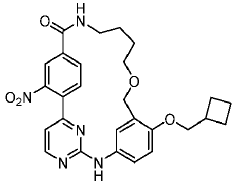 | 504.22/504.20 | 4⁴-(cyclobutylmethoxy)-1²-nitro-6 -oxa-3,11-diaza-2(4,2)-pyrimidina -1(1,4),4(1,3)-dibenzenacyclodode caphan-12-one |
| **100** | 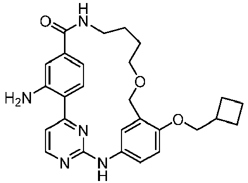 | 474.24/474.30 | 1²-amino-4⁴-(cyclobutylmethoxy)-6-oxa-3,11-diaza-2(4,2)-pyrimidin a-1(1,4),4(1,3)-dibenzenacyclodod ecaphan-12-one |
| **101** | 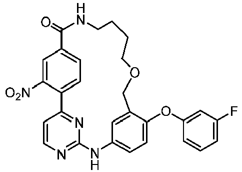 | 530.18/530.20 | 4⁴-(3-fluorophenoxy)-1²-nitro-6-o xa-3,11-diaza-2(4,2)-pyrimidina-1 (1,4),4(1,3)-dibenzenacyclododeca phan-12-one |
| **102** | 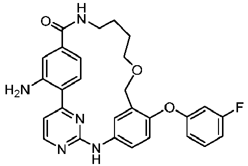 | 500.20/500.20 | 1²-amino-4⁴-(3-fluorophenoxy)-6-oxa-3,11-diaza-2(4,2)-pyrimidina-1(1,4),4(1,3)-dibenzenacyclodode caphan-12-one |
| **103** | 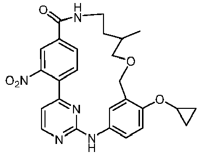 | 490.20/490.20 | 4⁴-cyclopropoxy-8-methyl-1²-nitr o-6-oxa-3,11-diaza-2(4,2)-pyrimid ina-1(1,4),4(1,3)-dibenzenacyclod odecaphan-12-one |
| **104** | 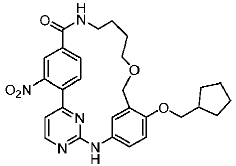 | 518.23/518.30 | 4⁴-(cyclopentylmethoxy)-1²-nitro-6-oxa-3,11-diaza-2(4,2)-pyrimidin a-1(1,4),4(1,3)-dibenzenacyclodod ecaphan-12-one |
| **105** | 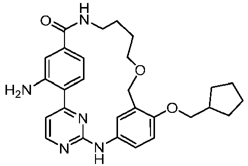 | 488.26/488.30 | 1²-amino-4⁴-(cyclopentylmethoxy )-6-oxa-3,11-diaza-2(4,2)-pyrimid ina-1(1,4),4(1,3)-dibenzenacyclod odecaphan-12-one |
| **106** | 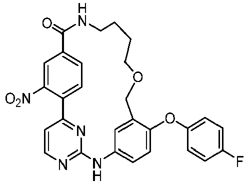 | 530.18/530.10 | 4⁴-(4-fluorophenoxy)-1²-nitro-6-o xa-3,11-diaza-2(4,2)-pyrimidina-1 (1,4),4(1,3)-dibenzenacyclododeca phan-12-one |
| **107** | 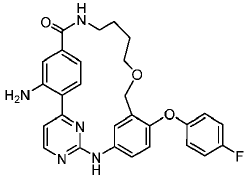 | 500.20/500.20 | 1²-amino-4⁴-(4-fluorophenoxy)-6-oxa-3,11-diaza-2(4,2)-pyrimidina-1(1,4),4(1,3)-dibenzenacyclodode caphan-12-one |
| **108** | 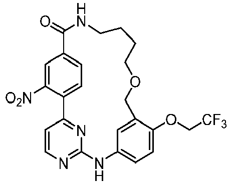 | 518.16/518.20 | 1²-nitro-4⁴-(2,2,2-trifluoroethoxy)-6-oxa-3,11-diaza-2(4,2)-pyrimidin a-1(1,4),4(1,3)-dibenzenacyclodod ecaphan-12-one |
| **109** | 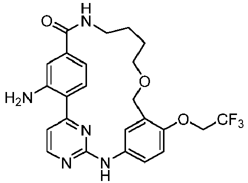 | 488.19/488.20 | 1²-amino-4⁴-(2,2,2-trifluoroethoxy )-6-oxa-3,11-diaza-2(4,2)-pyrimid ina-1(1,4),4(1,3)-dibenzenacyclod odecaphan-12-one |
| **110** | 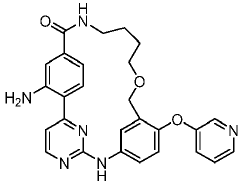 | 483.21/483.20 | 1²-amino-4⁴-(pyridin-3-oxy)-6-ox a-3,11-diaza-2(4,2)-pyrimidina -1(1,4),4(1,3)-dibenzenacyclodode caphan-12-one |
| **111** | 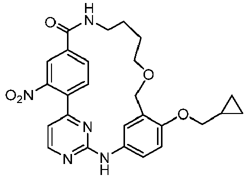 | 490.20/490.20 | 4⁴-(cyclopropylmethoxy)-1²-nitro-6-oxa-3,11-diaza-2(4,2)-pyrimidin a-1(1,4),4(1,3)-dibenzenacyclodod ecaphan-12-one |
| **112** | 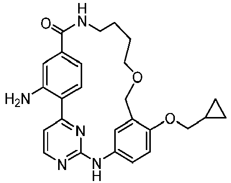 | 460.23/460.40 | 1²-amino-4⁴-(cyclopropylmethoxy )-6-oxa-3,11-diaza-2(4,2)-pyrimid ina-1(1,4),4(1,3)-dibenzenacyclod odecaphan-12-one |
| **113** | 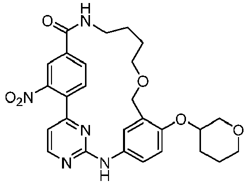 | 520.21/520.20 | 1²-nitro-4⁴-((tetrahydro-2H-pyran-3-yl)oxy)-6-oxa-3,11-diaza-2(4,2) -pyrimidina-1(1,4),4(1,3)-dibenze nacyclododecaphan-12-one |
| **114** | 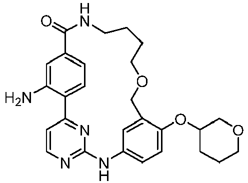 | 490.24/490.20 | 1²-amino-4⁴-((tetrahydro-2H-pyra n-3-yl)oxy)-6-oxa-3,11-diaza-2(4, 2)-pyrimidina-1(1,4),4(1,3)-diben zenacyclododecaphan-12-one |
| **115** | 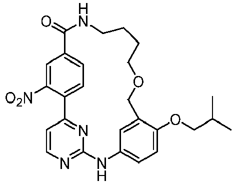 | 492.22/492.40 | 4⁴-isobutoxy-1²-nitro-6-oxa-3,11-diaza-2(4,2)-pyrimidina-1(1,4),4(1 ,3)-dibenzenacyclododecaphan-12 -one |
| **116** | 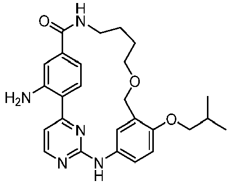 | 462.24/462.20 | 1²-amino-4⁴-isobutoxy-6-oxa-3,11 -diaza-2(4,2)-pyrimidina-1(1,4),4( 1,3)-dibenzenacyclododecaphan-1 2-one |
| **117** | 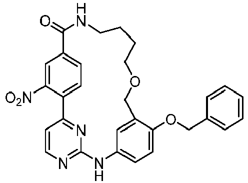 | 526.20/526.20 | 4⁴-(benzyloxy)-1²-nitro-6-oxa-3,1 1-diaza-2(4,2)-pyrimidina-1(1,4),4 (1,3)-dibenzenacyclododecaphan-12-one |
| **118** | 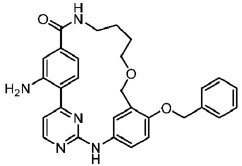 | 496.23/496.20 | 1²-amino-4⁴-(benzyloxy)-6-oxa-3, 11-diaza-2(4,2)-pyrimidina-1(1,4), 4(1,3)-dibenzenacyclododecaphan -12-one |
| **119** | 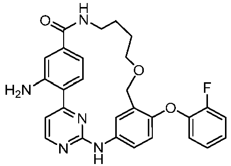 | 500.20/500.20 | 1²-amino-4⁴-(2-fluorophenoxy)-6-oxa-3,11-diaza-2(4,2)-pyrimidina-1(1,4),4(1,3)-dibenzenacyclodode caphan-12-one |
| **120** | 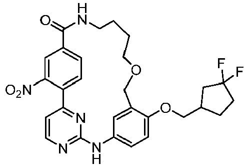 | 554.21/554.20 | 4⁴-((3,3-difluorocyclopentyl)meth oxy)-1²-nitro-6-oxa-3,11-diaza-2( 4,2)-pyrimidina-1(1,4),4(1,3)-dibe nzenacyclododecaphan-12-one |
| **121** | 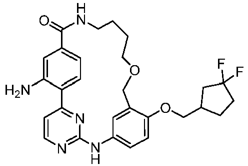 | 524.24/524.30 | 1²-amino-4⁴-((3,3-difluorocyclope ntyl)methoxy)-6-oxa-3,11-diaza-2 (4,2)-pyrimidina-1(1,4),4(1,3)-dib enzenacyclododecaphan-12-one |
| **122** | 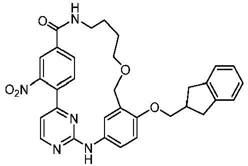 | 566.63/566.30 | 4⁴-((2,3-dihydro-1H-inden-2-yl) methoxy)-1²-nitro-6-oxa-3,11-diaz a-2(4,2)-pyrimidina-1(1,4),4(1,3)-dibenzenacyclododecaphan-12-on e |
| **123** | 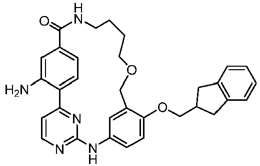 | 536.26/536.30 | 1²-amino-4⁴-((2,3-dihydro-1H-ind en-2-yl)methoxy)-6-oxa-3,11-diaz a-2(4,2)-pyrimidina-1(1,4),4(1,3)-dibenzenacyclododecaphan-12-on e |
| **124** | 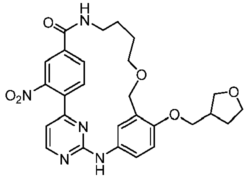 | 520.21/520.20 | 1²-nitro-4⁴-((tetrahydrofuran-3-yl) methoxy)-6-oxa-3,11-diaza-2(4,2) -pyrimidina-1(1,4),4(1,3)-dibenze nacyclododecaphan-12-one |
| **125** | 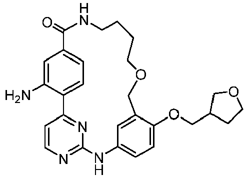 | 490.24/490.20 | 1²-amino-4⁴-((tetrahydrofuran-3-y l)methoxy)-6-oxa-3,11-diaza-2(4, 2)-pyrimidina-1(1,4),4(1,3)-dibenz enacyclododecaphan-12-one |
| **126** | 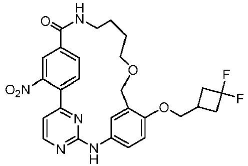 | 540.20/540.30 | 4⁴-((3,3-Difluorocyclobutyl)metho xy)-1²-nitro-6-oxa-3,11-diaza-2(4, 2)-pyrimidina-1(1,4),4(1,3)-dibenz enacyclododecaphan-12-one |
| **127** | 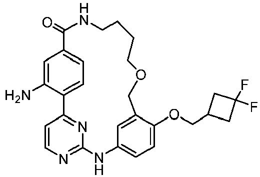 | 510.22/510.40 | 1²-Amino-4⁴-((3,3-difluorocyclob utyl) methoxy)-6-oxa-3,11-diaza-2 (4,2)-pyrimidina-1(1,4),4(1,3)-dib enzenacyclododecaphan-12-one |
| **128** | 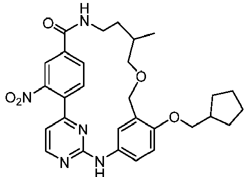 | 532.25/532.20 | 4⁴-(cyclopentylmethoxy)-8-methyl -1²-nitro-6-oxa-3,11-diaza-2(4,2)-pyrimidina-1(1,4),4(1,3)-dibenzen acyclododecaphan-12-one |
| **129** | 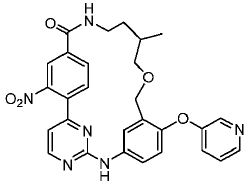 | 527.20/527.20 | 8-methyl-1²-nitro-4⁴-(pyridin-3-ox yl)-6-oxa-3,11-diaza-2(4,2)-pyrim idina-1(1,4),4(1,3)-dibenzenacyclo dodecaphan-12-one |
| **130** | 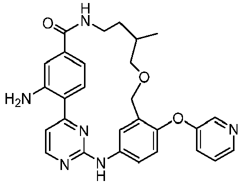 | 497.22/497.30 | 1²-amino-8-methyl-4⁴-(pyridin-3-oxy)-6-oxa-3,11-diaza-2(4,2)-pyri midina-1(1,4),4(1,3)-dibenzenacyc lododecaphan-12-one |
| **131** | 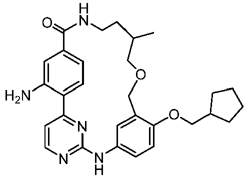 | 502.27/502.30 | 1²-amino-4⁴-(cyclopentylmethoxy )-8-methyl-6-oxa-3,11-diaza-2(4,2 )-pyrimidina-1(1,4),4(1,3)-dibenze nacyclododecaphan-12-one |
| **132** | 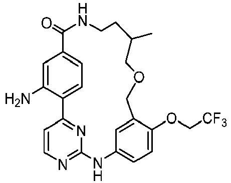 | 502.20/502.30 | 1²-amino-8-methyl-4⁴-(2,2,2-triflu oroethoxy)-6-oxa-3,11-diaza-2(4,2 )-pyrimidina-1(1,4),4(1,3)-dibenze nacyclododecaphan-12-one |
| **133** | 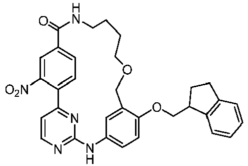 | 566.23/566.30 | 4⁴-((2,3-dihydro-1H-inden-1-yl) methoxy)-1²-nitro-6-oxa-3,11-diaz a-2(4,2)-pyrimidina-1(1,4),4(1,3)-dibenzenacyclododecaphan-12-on e |
| **134** | 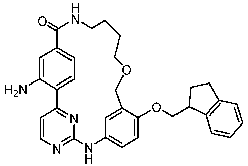 | 536.26/536.40 | 1²-amino-4⁴-((2,3-dihydro-1H-ind en-1 -yl)methoxy)-6-oxa-3,11-diaz a-2(4,2)-pyrimidina-1(1,4),4(1,3)-dibenzenacyclododecaphan-12-on e |
| **135** | 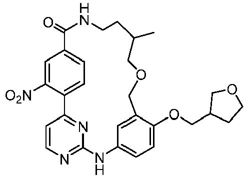 | 534.23/534.30 | 8-methyl-1²-nitro-4⁴-((tetrahydrof uran-3-yl)methoxy)-6-oxa-3,11-di aza-2(4,2)-pyrimidina-1(1,4),4(1,3 )-dibenzenacyclododecaphan-12-o ne |
| **136** | 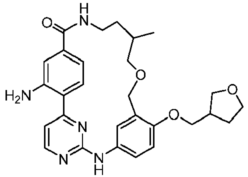 | 504.25/504.30 | 1²-amino-8-methyl-4⁴-((tetrahydro furan-3-yl)methoxy)-6-oxa-3,11-d iaza-2(4,2)-pyrimidina-1(1,4),4(1, 3)-dibenzenacyclododecaphan-12-one |
| **137** | 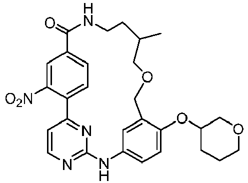 | 534.23/534.30 | 8-methyl-1²-nitro-4⁴-((tetrahydro-2H-pyran-3 -yl)oxy)-6-oxa-3,11-di aza-2(4,2)-pyrimidina-1(1,4),4(1, 3)-dibenzenacyclododecaphan-12-one |
| **138** | 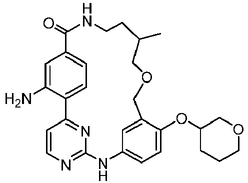 | 504.25/504.40 | 1²-amino-8-methyl-4⁴-((tetrahydro -2H-pyran-3-yl)oxy)-6-oxa-3,11-d iaza-2(4,2)-pyrimidina-1(1,4),4(1, 3)-dibenzenacyclododecaphan-12-one |
| **139** | 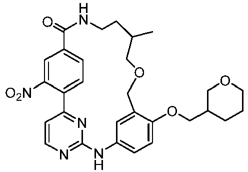 | 548.24/548.30 | 8-methyl-1²-nitro-4⁴-((tetrahydro-2H-pyran-3 -yl)methoxy)-6-oxa-3, 11-diaza-2(4,2)-pyrimidina-1(1,4) ,4(1,3)-dibenzenacyclododecapha n-12-one |
| **140** | 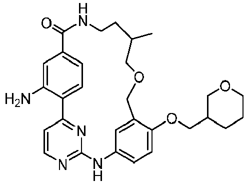 | 518.27/518.30 | 1²-amino-8-methyl-4⁴-((tetrahydro -2H-pyran-3 -yl)methoxy)-6-oxa-3, 11-diaza-2(4,2)-pyrimidina-1(1,4) ,4(1,3)-dibenzenacyclododecapha n-12-one |
| **141** | 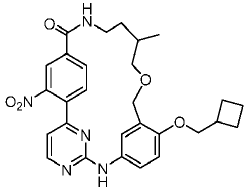 | 518.23/518.30 | 4⁴-(cyclobutylmethoxy)-8-methyl-1²-nitro-6-oxa-3,11-diaza-2(4,2)-p yrimidina-1(1,4),4(1,3)-dibenzena cyclododecaphan-12-one |
| **142** | 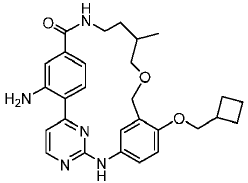 | 488.26/488.40 | 1²-amino-4⁴-(cyclobutylmethoxy)-8-methyl-6-oxa-3,11-diaza-2(4,2)-pyrimidina-1(1,4),4(1,3)-dibenzen acyclododecaphan-12-one |
| **143** | 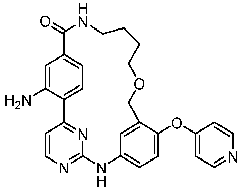 | 483.21/483.30 | 1²-amino-4⁴-(pyridin-4-oxyl)-6-ox a-3,11-diaza-2(4,2)-pyrimidina-1( 1,4),4(1,3)-dibenzenacyclododeca phan-12-one |
| **144** | 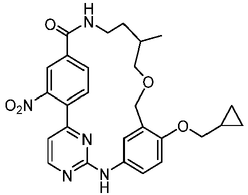 | 504.22/504.30 | 4⁴-(cyclopropylmethoxy)-8-methy 1-1²-nitro-6-oxa-3,11-diaza-2(4,2)-pyrimidina-1(1,4),4(1,3)-dibenzen acyclododecaphan-12-one |
| **145** | 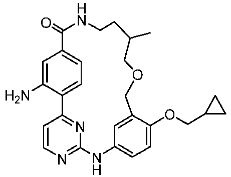 | 474.24/474.20 | 1²-amino-4⁴-(cyclopropylmethoxy )-8-methyl-6-oxa-3,11-diaza-2(4,2 )-pyrimidina-1(1,4),4(1,3)-dibenze nacyclododecaphan-12-one |
| **146** | 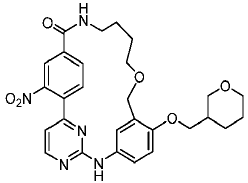 | 534.23/534.30 | 1²-nitro-4⁴-((tetrahydro-2H-pyran-3-yl)methoxy)-6-oxa-3,11-diaza-2 (4,2)-pyrimidina-1(1,4),4(1 ,3)-dib enzenacyclododecaphan-12-one |
| **147** | 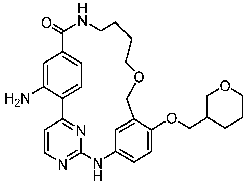 | 504.25/504.20 | 1²-amino-4⁴-(((tetrahydro-2H-pyra n-3-yl)methoxy)-6-oxa-3,11-diaza -2(4,2)-pyrimidina-1(1,4),4(1,3)-d ibenzenacyclododecaphan-12-one |
| **148** | 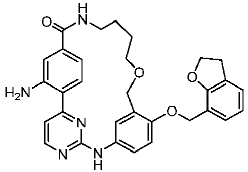 | 538.24/538.20 | 1²-amino-4⁴-((2,3-dihydrobenzofu ran-7-yl)methoxy)-6-oxa-3,11-dia za-2(4,2)-pyrimidina-1(1,4),4(1,3 )-dibenzenacyclododecaphan-12-o ne |
| **149** | 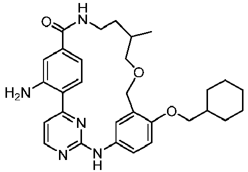 | 516.29/516.30 | 1²-amino-4⁴-(cyclohexylmethoxy) -8-methyl-6-oxa-3,11-diaza-2(4,2) -pyrimidina-1(1,4),4(1,3)-dibenze nacyclododecaphan-12-one |
| **150** | 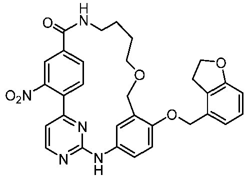 | 568.21/568.20 | 4⁴-((2,3-dihydrobenzofuran-4-yl) methoxy)-1²-nitro-6-oxa-3,11-diaz a-2(4,2)-pyrimidina-1(1,4),4(1,3)-dibenzenacyclododecaphan-12-on e |
| **151** | 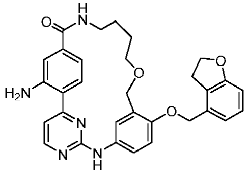 | 538.24/538.40 | 1²-amino-4⁴-((2,3-dihydrobenzofu ran-4-yl)methoxy)-6-oxa-3,11-dia za-2(4,2)-pyrimidina-1(1,4),4(1,3) -dibenzenacyclododecaphan-12-o ne |
| **152** | 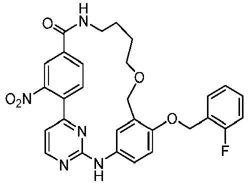 | 544.19/544.20 | 4⁴-((2-fluorobenzyl)oxy)-1²-nitro-6-oxa-3,11-diaza-2(4,2)-pyrimidin a-1(1,4),4(1,3)-dibenzenacyclodod ecaphan-12-one |
| **153** | 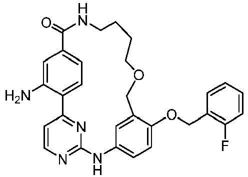 | 514.22/514.30 | 1²-amino-4⁴-((2-fluorobenzyl)oxy) -6-oxa-3,11-diaza-2(4,2)-pyrimidi na-1(1,4),4(1,3)-dibenzenacyclodo decaphan-12-one |
| **154** | 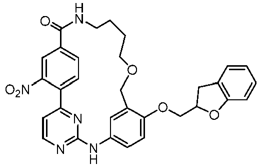 | 568.21/568.30 | 4⁴-((2,3-dihydrobenzofuran-2-yl) methoxy)-1²-nitro-6-oxa-3,11-diaz a-2(4,2)-pyrimidina-1(1,4),4(1,3)-dibenzenacyclododecaphan-12-on e |
| **155** | 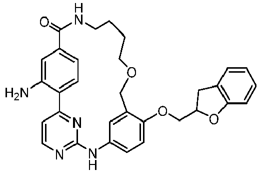 | 538.24/538.10 | 1²-amino-4⁴-((2,3-dihydrobenzofu ran-2-yl)methoxy)-6-oxa-3,11-dia za-2(4,2)-pyrimidina-1(1,4),4(1,3) -dibenzenacyclododecaphan-12-o ne |
| **156** | 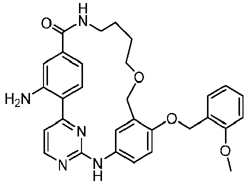 | 526.24/526.20 | 1²-amino-4⁴-((2-methoxybenzyl) oxyl)-6-oxa-3,11-diaza-2(4,2)-pyri midina-1(1,4),4(1,3)-dibenzenacyc lododecaphan-12-one |
| **157** | 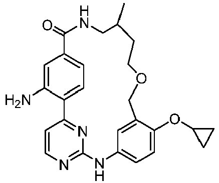 | 460.23/460.20 | 1²-amino-4⁴-cyclopropoxy-9-meth yl-6-oxa-3,11-diaza-2(4,2)-pyrimi dina-1(1,4),4(1,3)-dibenzenacyclo dodecaphan-12-one |
| **158** | 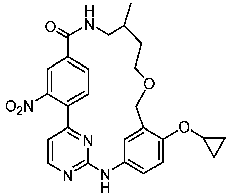 | 490.20/490.30 | 4⁴-cyclopropoxy-9-methyl-1²-nitr o-6-oxa-3,11-diaza-2(4,2)-pyrimid ina-1(1,4),4(1,3)-dibenzenacyclod odecaphan-12-one |
| **159** | 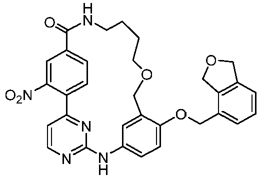 | 568.21/568.20 | 4⁴-((1,3-dihydroisobenzofuran-4-y l)methoxy)-1²-nitro-6-oxa-3,11-di aza-2(4,2)-pyrimidina-1(1,4),4(1,3 )-dibenzenacyclododecaphan-12-o ne |
| **160** | 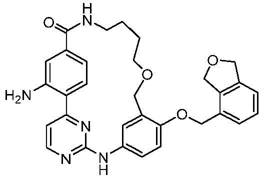 | 538.24/538.30 | 1²-amino-4⁴-((1,3-dihydroisobenz ofuran-4-yl)methoxy)-6-oxa-3,11-diaza-2(4,2)-pyrimidina-1(1,4),4(1 ,3)-dibenzenacyclododecaphan-12 -one |
| **161** | 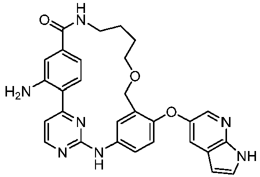 | 522.22/522.30 | 4⁴-((1H-pyrrolo[2,3-b]pyridin-5-yl )oxy)-1²-amino-6-oxa-3,11-diaza-2(4,2)-pyrimidina-1(1,4),4(1,3)-di benzenacyclododecaphan-12-one |
| **162** | 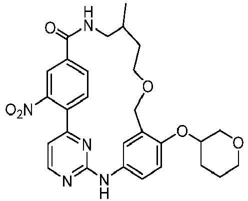 | 534.23/534.40 | 9-methyl-1²-nitro-4⁴-((tetrahydro-2H-pyran-3-yl)oxy)-6-oxa-3,11-di aza-2(4,2)-pyrimidina-1(1,4),4(1,3 )-dibenzenacyclododecaphan-12-o ne |
| **163** | 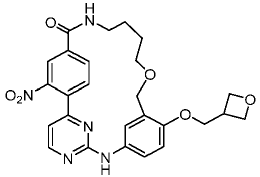 | 506.2/506.30 | 1²-nitro-4⁴-(oxetan-3-ylmethoxy)-6-oxa-3,11-diaza-2(4,2)-pyrimidin a-1(1,4),4(1,3)-dibenzenacyclodod ecaphan-12-one |
| **164** | 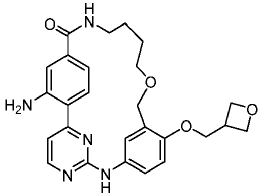 | 476.22/476.30 | 1²-amino-4⁴-(oxetan-3-ylmethoxy) -6-oxa-3-diaza-2 (4,2)-pyrimidina -1(1,4),4(1,3)-dibenzenacyclodode caphan-12-one |
| **165** | 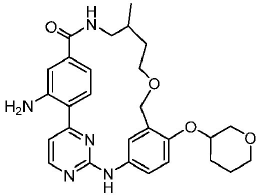 | 504.25/504.30 | 1²-amino-9-methyl-4⁴-((tetrahydro -2H-pyran-3-yl)oxy)-6-oxa-3,11-d iaza-2(4,2)-pyrimidina-1(1,4),4(1, 3)-dibenzenacyclododecaphan-12-one |
| **166** | 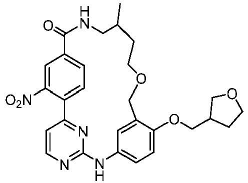 | 534.23/534.30 | 9-methyl-1²-nitro-4⁴-((tetrahydrof uran-3-yl)methoxy)-6-oxa-3,11-di aza-2(4,2)-pyrimidina-1(1,4),4(1,3 )-dibenzenacyclododecaphan-12-o ne |
| **167** | 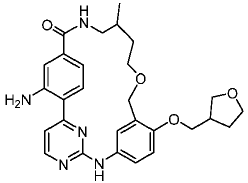 | 504.25/504.40 | 1²-amino-9-methyl-4⁴-((tetrahydro furan-3-yl)methoxy)-6-oxa-3,11-d iaza-2(4,2)-pyrimidina-1(1,4),4(1, 3)-dibenzenacyclododecaphan-12-one |
| **168** | 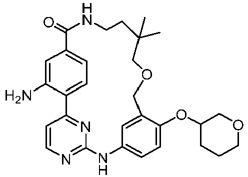 | 518.27/518.30 | 1²-amino-8,8-dimethyl-4⁴-((tetrah ydro-2H-pyran-3 -yl)oxy)-6-oxa-3, 11-diaza-2(4,2)-pyrimidina-1(1,4), 4(1,3)-dibenzenacyclododecaphan -12-one |
| **169** | 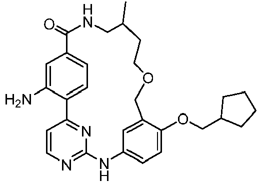 | 502.27/502.40 | 1²-amino-4⁴-(cyclopentylmethoxy )-9-methyl-6-oxa-3,11-diaza-2(4,2 )-pyrimidina-1(1,4),4(1,3)-dibenze nacyclododecaphan-12-one |
| **170** | 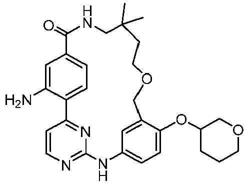 | 518.27/518.40 | 1²-amino-9, 9-dimethyl-4⁴-((tetrah ydro-2H-pyran-3 -yl)oxy)-6-oxa-3, 1 1-diaza-2(4,2)-pyrimidina-1(1,4), 4(1,3)-dibenzenacyclododecaphan -12-one |
| **171** | 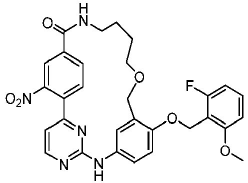 | 574.2/574.30 | 4⁴-((2-fluoro-6-methoxybenzyl) oxy)-1²-nitro-6-oxa-3,11-diaza-2 (4,2)-pyrimidina-1(1,4),4(1,3)-dib enzenacyclododecaphan-12-one |
| **172** | 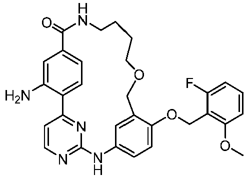 | 544.23/544.20 | 1²-amino-4⁴-((2-fluoro-6-methoxy benzyl) oxy)-6-oxa -3,11-diaza-2 (4,2)-pyrimidina-1(1,4),4(1,3)-dib enzenacyclododecaphan-12-one |
| **173** | 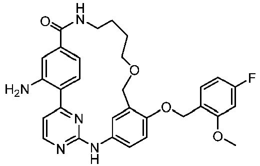 | 544.23/544.30 | 1²-amino-4⁴-((4-fluoro-2-methoxy benzyl) oxy)-6-oxa -3,11-diaza-2 (4,2)-pyrimidina-1(1,4),4(1,3)-dib enzenacyclododecaphan-12-one |
| **174** | 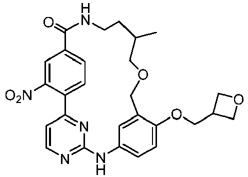 | 520.21/520.30 | 8-methyl-1²-nitro-4⁴-(oxetan-3-yl methoxy)-6-oxa-3-diaza-2(4,2)-py rimidina-1(1,4),4(1,3)-dibenzenac yclododecaphan-12-one |
| **175** | 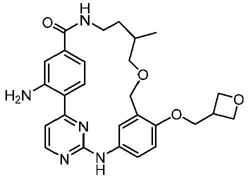 | 490.24/490.30 | 1²-amino-8-methyl-4⁴-(oxetin-3-yl methoxy)-6-oxa-3,11-diaza-2(4,2) -pyrimidina-1(1,4),4(1,3)-dibenze nacyclododecaphan-12-one |
| **176** | 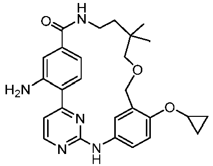 | 474.24/474.20 | 1²-amino-4⁴-cyclopropoxy-8,8-di methyl-6-oxa-3,11-diaza-2(4,2)-p yrimidina-1(1,4),4(1,3)-dibenzena cyclododecaphan-12-one |
| **177** | 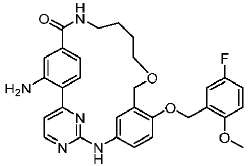 | 544.23/544.30 | 1²-amino-4⁴-((5-fluoro-2-methoxy benzyl)oxy)-6-oxa-3,11-diaza-2(4, 2)-pyrimidina-1(1,4),4(1,3)-dibenz enacyclododecaphan-12-one |
| **178** | 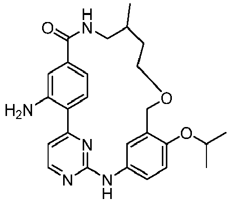 | 462.24/462.20 | 1²-amino-4⁴-isopropoxy-9-methyl-6-oxa-3,11-diaza-2(4,2)-pyrimidin a-1(1,4),4(1,3)-dibenzenacyclodod ecaphan-12-one |
| **179** | 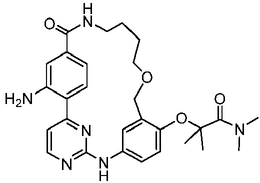 | 519.26/519.20 | 2-((1²-amino-12-oxo-6-oxa-3,11-d iaza-2(4,2)-pyrimidina-1(1,4),4(1, 3)-dibenzenacyclododecaphan-4⁴-yl)oxy)-N,N,2-trimethylpropiona mide |
| **180** | 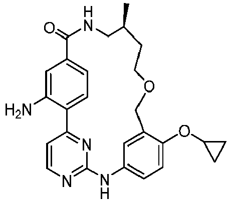 | 460.23/460.30 | (S)-1²-amino-4⁴-cyclopropoxy-9-methyl-6-oxa-3,1 1-diaza-2(4,2)-p yrimidina-1(1,4),4(1,3)-dibenzena cyclododecaphan-12-one |
| **181** | 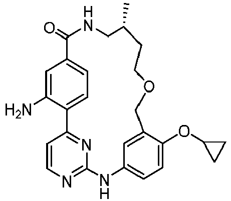 | 460.23/460.20 | (R)-1²-amino-4⁴-cyclopropoxy-9-methyl-6-oxa-3,11-diaza-2 (4,2)-pyrimidina-1(1,4),4(1,3)-dib enzenacyclododecaphan-12-one |
| **182** | 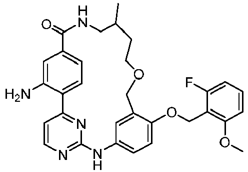 | 558.24/558.40 | 1²-amino-4⁴-((2-fluoro-6-methoxy benzyl)oxy)-9-methyl-6-oxa-3,11-diaza-2(4,2)-pyrimidina-1(1,4),4(1 ,3)-dibenzenacyclododecaphan-12 -one |
| **183** | 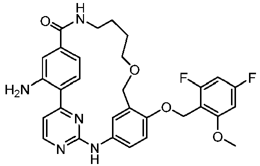 | 562.22/562.30 | 1²-amino-4⁴-((2,4-difluoro-6-meth oxybenzyl)oxy)-6-oxa-3,11-diaza-2(4,2)-pyrimidina-1(1,4),4(1,3)-di benzenacyclododecaphan-12-one |
| **184** | 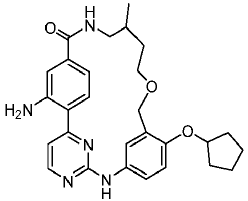 | 488.26/488.50 | 1²-amino-4⁴-(cyclopentoxy)-9-met hyl-6-oxa-3,11-diaza-2(4,2)-pyrim idina-1(1,4),4(1,3)-dibenzenacyclo dodecaphan-12-one |
| **185** | 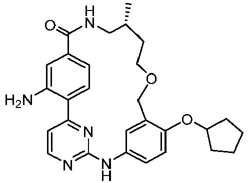 | 488.26/488.40 | (R)-1²-amino-4⁴-(cyclopentoxy)-9 -methyl-6-oxa-3,11-diaza-2(4,2)-p yrimidina-1(1,4),4(1,3)-dibenzena cyclododecaphan-12-one |
| **186** | 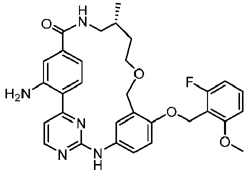 | 558.24/558.40 | (R)-1²-amino-4⁴-((2-fluoro-6-meth oxybenzyl)oxy)-9-methyl-6-oxa-3, 11-diaza-2(4,2)-pyrimidina-1(1,4), 4(1,3)-dibenzenacyclododecaphan -12-one |
| **187** | 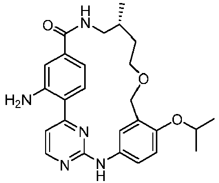 | 462.24/462.20 | (R)-1²-amino-4⁴-isopropoxy-9-me thyl-6-oxa-3,11-diaza-2(4,2)-pyri midina-1(1,4),4(1,3)-dibenzenacyc lododecaphan-12-one |
| **188** | 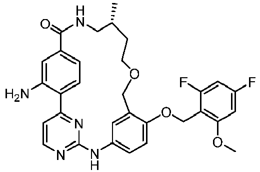 | 576.23/576.20 | (R)-1²-amino-4⁴-((2,4-difluoro-6-methoxybenzyl)oxy)-9-methyl-6-o xa-3,11-diaza-2(4,2)-pyrimidina-1 (1,4),4(1,3)-dibenzenacyclododeca phan-12-one |
| **189** | 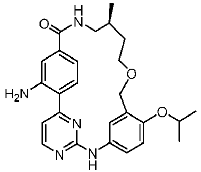 | 462.24/462.20 | (S)-1²-amino-4⁴-isopropoxy-9-met hyl-6-oxa-3,11-diaza-2(4,2)-pyrim idina-1(1,4),4(1,3)-dibenzenacyclo dodecaphan-12-one |
| **190** | 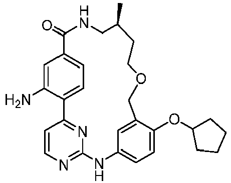 | 488.26/488.30 | (S)-1²-amino-4⁴-(cyclopentyloxy)-9-methyl-6-oxa-3,11-diaza-2(4,2)-pyrimidina-1(1,4),4(1,3)-dibenzen acyclododecaphan-12-one |
| **191** | 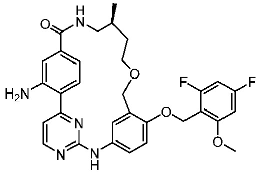 | 576.23/576.20 | (S)-1²-amino-4⁴-((2,4-difluoro-6-methoxybenzyl)oxy)-9-methyl-6-o xa-3,11-diaza-2(4,2)-pyrimidina-1 (1,4),4(1,3)-dibenzenacyclododeca phan-12-one |
| **192** | 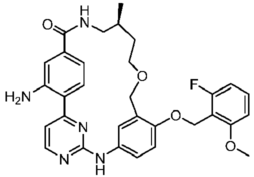 | 558.24/558.30 | (S)-1²-amino-4⁴-((2-fluoro-6-meth oxybenzyl)oxy)-9-methyl-6-oxa-3, 11-diaza-2(4,2)-pyrimidina-1(1,4), 4(1,3)-dibenzenacyclododecaphan -12-one |
| **193** | 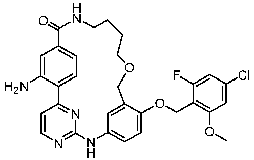 | 578.19/578.20 | 1²-amino-4⁴-((4-chloro-2-fluoro-6 -methoxybenzyl)oxy)-6-oxa-3,11-diaza-2(4,2)-pyrimidina-1(1,4),4(1 ,3)-dibenzenacyclododecaphan-12 -one |
| **194** | 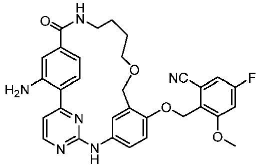 | 569.22/569.20 | 2-(((1²-amino-12-oxo-6-oxo-3,11-diaza-2(4,2)-pyrimidina-1(1,4),4(1 ,3)-dibenzenacyclododecaphan-4⁴-yl)oxy)methyl)-5-fluoro-3-methox ybenzonitrile |
| **195** | 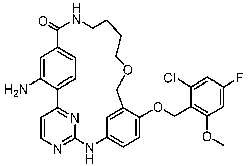 | 578.19/578.20 | 1²-amino-4⁴-((2-chloro-4-fluoro-6 -methoxybenzyl)oxy)-6-oxa-3,11-diaza-2(4,2)-pyrimidina-1(1,4),4(1 ,3)-dibenzenacyclododecaphan-12 -one |
| **196** | 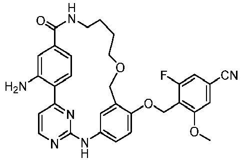 | 569.22/569.00 | 4-(((1²-amino-12-oxo-6-oxa-3,11-diaza-2(4,2)-pyrimidina-1(1,4),4(1 ,3)-dibenzenacyclododecaphan-4⁴-yl)oxy)methyl)-3-fluoro-5-methox ybenzonitrile |

### Example 197 Preparation of 1²-amino-4⁴-((tetrahydro-2H-pyran-3-yl) oxy)-6-oxa-3-8-diaza-2 (4,2)-pyrimidina-1,7(1,4), 4 (1,3)-tribenzenacyclononaphane-9-one (197)

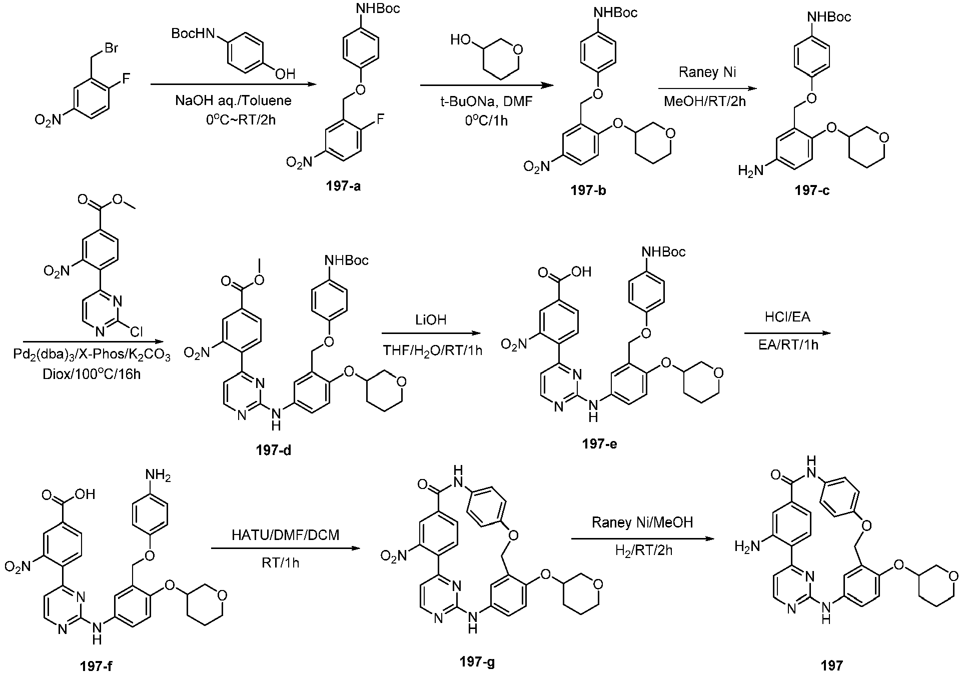

### Step 1: tert-butyl (4-((2-fluoro-5-nitrobenzyl) oxy) phenyl) carbamate (197-a)

2-Fluoro-5-nitrobenzyl bromide (5.0g, 21mmol) was dissolved in toluene (25ml), 50% NaOH solution (50ml) was slowly added under an ice bath, and then a solution of oxycarbonyl-4-hydroxyaniline (4.7g, 22mmol) in toluene (25ml) was added, and the solution was stirred at room temperature for 2 hours. After TLC confirmed that the reaction was completed, EA was extracted, washed with deionized water for three times, and dried over anhydrous sodium sulfate. EA was concentrated, and the residue was purified by column chromatography to obtain tert-butyl (4-(((2-fluoro-5-nitrobenzyl) oxy) phenyl) carbamate (**197-a**, 6.01g, yield 78%) a yellow solid. MS (ESI) m/z: Calcd 234.00 (M + H).

### Step 2: tert-butyl (4-((5-nitro-2-((tetrahydro-2H-pyran-3-yl) oxy) benzyl) oxy) phenyl) carbamate (197-b)

Tert-butyl (4-((2-fluoro-5-nitrobenzyl) oxy) phenyl) carbamate (**197-a**, 1g, 2.8mmol) and 3-hydroxytetrahydropyran (572mg,5.6mmol) were dissolved in DMF(10ml), sodium tert-butanol (333mg,3.36mmol) was slowly added under an ice bath, and stirred at 0°C for 1h. After TLC confirmed that the reaction was completed, water was added for dilution, EA was added for extraction, washed with deionized water for three times, and dried over anhydrous sodium sulfate. EA was concentrated to obtain tert-butyl (4-((5-nitro-2-((tetrahydro-2H-pyran-3-yl) oxy) benzyl) oxy) phenyl) carbamate (**197-b**, 1.20g, yield 97%) as a yellow oil. MS (ESI) m/z: Calcd 445.20 (M + H).

### Step 3: tert-butyl (4-((5-amino-2-((tetrahydro-2H-pyran-3-yl) oxy) benzyl) oxy) phenyl) carbamate (197-c)

Tert-butyl (4-((5-nitro-2-((tetrahydro-2H-pyran-3-yl) oxy) benzyl) oxy) phenyl) carbamate (**197**-b, 1.2g, 2.7mmol) was dissolved in methanol (20ml), and Raney Ni(120 mg, 10% wt) was added. Hydrogen bag was used to replace hydrogen for three times, and stirred at room temperature for 2h. After TLC confirmed that the reaction was completed, it was filtered with diatomite and the filtrate was spin-dried to obtain tert-butyl (4-((5-amino-2-((tetrahydro-2H-pyran-3-yl) oxy) benzyl) oxy) phenyl) carbamate (**197-c,** 1.10g, yield 98%) as a yellow oil. MS (ESI) m/z: Calcd 415.20 (M + H).

### Step 4: Methyl 4-(2-((3-((4-(tert-butoxycarbonyl) amino) phenoxy) methyl)-4-((tetrahydro-2H-pyran-3-yl) oxy) phenyl) amino) pyrimidin-4-yl)-3-nitrobenzoate (197-d)

Under nitrogen protection, tert-butyl (4-((5-amino-2-((tetrahydro-2H-pyran-3-yl) oxy) benzyl) oxy) phenyl) carbamate (**197-c**, 1.1g, 2.6mmol) and methyl 4-(2-chloropyrimidin-4-yl)-3-nitrobenzoate(914mg, 3.12mmol) were added to 1,4-dioxane (20ml), then potassium carbonate (718mg, 5.2mmol), tris(dibenzylideneacetone)dipalladium (238mg, 0.26mmol) and 2-dicyclohexylphosphino-2,4,6-triisopropylbiphenyl (242mg, 0.52mmol) were added. The reaction solution was heated to 100 °C and stirred for 16h. After TLC confirmed that the reaction was completed, 1, 4-dioxane was concentrated, deionized water and ethyl acetate were added for liquid separation. The organic phase was washed with deionized water for three times, dried over anhydrous sodium sulfate. Ethyl acetate was concentrated, and the residue was purified by column chromatography to obtain methyl 4-(2-((3-((4-((tert-butoxycarbonyl) amino) phenoxy) methyl)-4-((tetrahydro-2H-pyran-3-yl) oxy) phenyl) amino) pyrimidin -4-yl) 3-nitrobenzoate (**197-d**, 900mg, yield 50%) as a yellow solid. MS (ESI) m/z: Calcd 672.30 (M + H).

### Step 5: 4-(2-((3-((4-((tert-butoxycarbonyl) amino) phenoxy) methyl)-4-((tetrahydro-2H-pyran-3-yl) oxy) phenyl) amino) pyrimidin-4-yl) 3-nitrobenzoic acid (197-e)

Methyl 4-(2-((3-((4-((tert-butoxycarbonyl) amino) phenoxy) methyl)-4-((tetrahydro-2H-pyran-3-yl) oxy) phenyl) amino) pyrimidin-4-yl) 3-nitrobenzoate (**197-d**, 900mg, 1.3mmol) was added to a mixed solution of THF and water (16ml/4ml), then lithium hydroxide monohydrate (273mg, 6.5mmol) was added, and stirred at room temperature for 2 hours. After TLC confirmed that the reaction was completed, 1M HCl was added to adjust the pH of the system to 7.0. The solvent was concentrated, EA and water were added for liquid extraction, washed with deionized water, and dried over anhydrous sodium sulfate. EA was concentrated to obtain 4-(2-((3-((4-((tert-butoxycarbonyl) amino) phenoxy) methyl)-4-((tetrahydro-2H-pyran-3-yl) oxy) phenyl) amino) pyrimidin-4-yl) 3-nitrobenzoic acid (**197-e**, 769mg, yield 90%) as a yellow solid, MS (ESI) m/z: Calcd 658.20 (M + H) which was directly used in the next reaction.

### Step 6: 4-(2-((3-((4-aminophenoxy) methyl)-4-((tetrahydro-2H-pyran-3-yl) oxy) phenyl) amino) pyrimidin-4-yl)-3-nitrobenzoic acid (197-f)

4-(2-((3-((4-(Tert-butoxycarbonyl) amino) phenoxy) methyl)-4-((tetrahydro-2H-pyran-3-yl) oxy) phenyl) amino) pyrimidin-4-yl) 3-nitrobenzoic acid (**197-e**, 769mg, 1.17mmol) was added to EA(8ml), then hydrogen chloride/EA solution (3mol/L, 8ml) was added dropwise, stirred at room temperature for 1h. After TLC confirmed that the reaction was completed, the reaction solution was filtered to obtain 4-(2-((3-((4-aminophenoxy) methyl)-4-((tetrahydro-2H-pyran-3-yl) oxy) phenyl) amino) pyrimidin-4-yl) 3-nitrobenzoic acid (**197-f**, 750mg, yield 100%) as a yellow solid. MS (ESI) m/z: calcd 478.25 (M + H), found 558.20.

### Step 7: 1²-nitro-4⁴-((tetrahydro-2H-pyran-3-yl) oxy)-6-oxa -3,8-diaza-2 (4,2)-pyrimidina-1,7(1,4),4(1,3)-tribenzenacyclononaphane-9-one (197-g)

O-(7-Azabenzotriazol-1-yl)-N,N,N',N'-tetramethyluronium hexafluorophosphate (1.03g, 2.70mmol) and N,N-diisopropylethylamine (871mg, 6.75mmol) were added to DCM(10ml) and stirred at room temperature. A mixed solution of 4-(2-((3-((4-aminophenoxy) methyl)-4-((tetrahydro-2H-pyran-3-yl) oxy) phenyl) amino) pyrimidin-4-yl)-3-nitrobenzoic acid (**197-f**, 750mg, 1.35mmol) in DCM and DMF(7ml/7ml) was added slowly, and the reaction was continued to be stirred at room temperature for 1h after addition. After LCMS confirmed that the reaction was completed, the reaction solution was concentrated, then it was poured into ice water, the solid was precipitated, filtered and dried to obtain 1²-nitro-4⁴-((tetrahydro-2H-pyran-3-yl) oxy)-6-oxa-3,8-diaza -2(4,2)-pyrimidina-1,7(1,4),4(1,3)-tribenzenacyclononaphane-9-one (**197-g**, 530mg, yield 73%) as a yellow solid. MS (ESI) m/z: calcd 540.20 (M+H); ¹H NMR (400 MHz, DMSO-d6) δ 10.06 (s, 1H), 9.58 (s, 1H), 8.58 (d, J= 4.8 Hz, 1H), 7.76 (d, J=2.7 Hz, 1H), 7.72 (m, 1H), 7.62 (d, J= 7.8 Hz, 1H), 7.43 (d, J = 1.5 Hz, 1H), 7.13 (d, J = 4.8 Hz, 1H), 7.09 (d, J = 2.4 Hz, 1H), 7.03 (d, J = 9.0 Hz, 1H), 6.78 (s, 4H), 5.13 (s, 2H), 4.32 (m, 1H), 3.81 (m, 2H), 3.55 (m, 2H), 2.17-1.43 (m, 4H).

### Step 8: 1²-amino-4⁴-((tetrahydro-2H-pyran-3-yl) oxy) -6-oxa-3-8-diaza-2 (4,2)-pyrimidina-1,7(1,4),4 (1,3)-tribenzenacyclononaphane-9-one (197)

1²-Nitro-4⁴-((tetrahydro-2H-pyran-3-yl) oxy)-6-oxa-3,8-diaza-2 (4,2)-pyrimidina-1,7(1,4),4(1,3)-tribenzenacyclononaphane-9-one (**197-g,** 50mg, 0.09mmol) was dissolved in methanol (5ml), and then Raney Ni(120 mg, 10% wt) was added. Hydrogen bag was used to replace hydrogen for three times, and stirred at room temperature for 2h. After TLC confirmed that the reaction was completed, the reaction solution was filtered with diatomite and the filtrate was spin-dried to obtain 1²-amino-4⁴-((tetrahydro-2H-pyran-3-yl) oxy) -6-oxa-3,8-diaza-2 (4,2)-pyrimidina-1,7(1,4),4 (1,3)-tribenzenacyclononaphane-9-one (**197**, 26g, yield 57%) as a yellow solid. MS (ESI) m/z: calcd 510.20 (M+H); ¹H NMR (400 MHz, DMSO-d6) δ 9.61 (s, 1H), 9.43 (s, 1H), 8.46 (d, J = 5.1 Hz, 1H), 7.93 (d, J = 2.7 Hz, 1H), 7.08 (m, 1H), 7.05 - 7.01 (m, 2H), 6.97 (d, J = 5.1 Hz, 1H), 6.76 - 6.69 (m, 2H), 6.65 - 6.57 (m, 2H), 6.41 - 6.30 (m, 2H), 5.13 (d, J = 2.8 Hz, 2H), 4.34 (m, 1H), 3.82 (m, 2H), 3.60 - 3.54 (m, 2H), 2.08 - 1.49 (m, 4H).

The following example compounds of **Table 3** were prepared according to the same method as the above example **197** by using the commercial compound or by referring to the preparation method of the intermediate compound shown.

**Table 3**

| Example | Structure | MS (calc) [M + H]⁺/ MS (found) | Name |
|---|---|---|---|
| **198** | 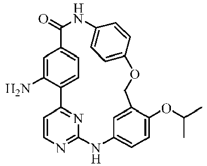 | 468.20/468.20 | 1²-amino-4⁴-isopropoxy-6-oxa-3,8-diaza-2(4,2)-pyrimidina-1,7(1,4),4( 1,3)-tribenzenacyclononaphane-9-o ne |
| **199** | 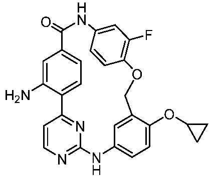 | 484.17/484.30 | 1²-amino-4⁴-cyclopropoxy-7²-fluor o-6-oxa-3,8-diaza-2(4,2)-pyrimidin a-1,7(1,4),4(1,3)-tribenzenacyclono naphane-9-one |
| **200** | 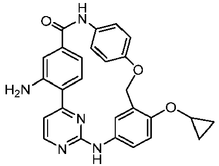 | 466.18/466.20 | 1²-amino-4⁴-cyclopropoxy-6-oxa-3, 8-diaza-2(4,2)-pyrimidina-1,7(1,4), 4(1,3)-tribenzenacyclononaphane-9-one |
| **201** | 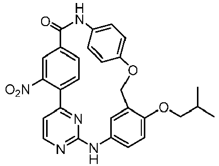 | 512.19/512.20 | 4⁴-isobutoxy-1²-nitro-6-oxa-3,8-dia za-2(4,2)-pyrimidina-1,7(1,4),4(1,3) -tribenzenacyclononaphane-9-one |
| **202** | 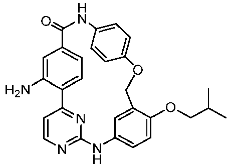 | 482.21/482.20 | 1²-amino-4⁴-isobutoxy-6-oxa-3,8-di aza-2(4,2)-pyrimidina-1,7(1,4),4(1, 3)-tribenzenacyclononaphane-9-one |
| **203** | 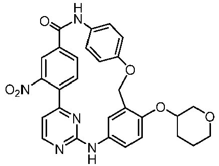 | 540.18/540.20 | 1²-nitro-4⁴-((tetrahydro-2H-pyran-3 -yl)oxy)-6-oxa-3,8-diaza-2(4,2)-pyr imidina-1,7(1,4),4(1,3)-tribenzenac yclononaphane-9-one |
| **204** | 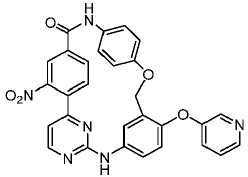 | 533.15/533.20 | 1²-nitro-4⁴-(pyridin-3-oxy)-6-oxa-3, 8-diaza-2(4,2)-pyrimidina-1,7(1,4), 4(1,3)-tribenzenacyclononaphane-9-one |
| **205** | 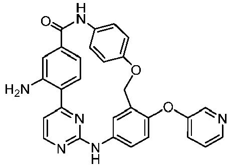 | 503.18/503.20 | 1²-amino-4⁴-(pyridin-3-oxy)-6-oxa-3,8-diaza-2(4,2)-pyrimidina-1,7(1,4 ),4(1,3)-tribenzenacyclononaphane-9-one |
| **206** | 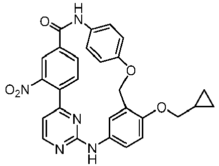 | 510.17/510.20 | 4⁴-(cyclopropylmethoxy)-1²-nitro-6 -oxa-3, 8-diaza-2(4,2)-pyrimidina-1, 7(1,4),4(1,3)-tribenzenacyclononap hane-9-one |
| **207** | 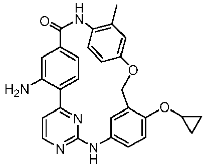 | 480.20/480.30 | 1²-amino-4⁴-cyclopropoxy-7³-meth yl-6-oxa-3,8-diaza-2(4,2)-pyrimidin a-1,7(1,4),4(1,3)-tribenzenacyclono naphane-9-one |
| **208** | 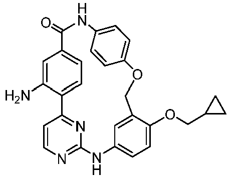 | 480.2/480.20 | 1²-amino-4⁴-(cyclopropylmethoxy)-6-oxa-3, 8-diaza-2(4,2)-pyrimidina-1,7(1,4),4(1,3)-tribenzenacyclonona phane-9-one |
| **209** | 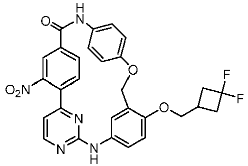 | 560.17/560.20 | 4⁴-((3,3-difluorocyclobutyl)methox y)-1²-nitro-6-oxa-3,8-diaza-2(4,2)-p yrimidina-1,7(1,4),4(1,3)-tribenzen acyclononaphane-9-one |
| **210** | 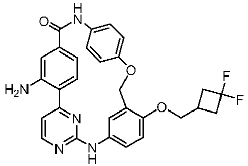 | 530.19/530.30 | 1²-amino-4⁴-((3,3-difluorocyclobut yl)methoxy)-6-oxa-3,8-diaza-2(4,2) -pyrimidina-1,7(1,4),4(1,3)-tribenz enacyclononaphane-9-one |
| **211** | 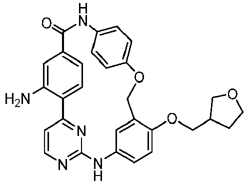 | 510.21/510.30 | 1²-amino-4⁴-((tetrahydrofuran-3-yl) methoxy)-6-oxa-3,8-diaza-2(4,2)-py rimidina-1,7(1,4),4(1,3)-tribenzena cyclononaphane-9-one |
| **212** | 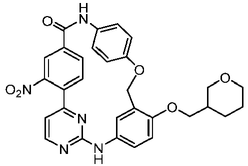 | 554.58/554.50 | 1²-nitro-4⁴-((tetrahydro-2H-pyran-3 -yl)methoxy)-6-oxa-3,8-diaza-2(4,2 )-pyrimidina-1,7(1,4),4(1,3)-tribenz enacyclononaphane-9-one |
| **213** | 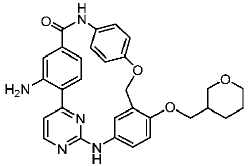 | 524.22/524.20 | 1²-amino-4⁴-((tetrahydro-2H-pyran-3-yl)methoxy)-6-oxa-3,8-diaza-2(4, 2)-pyrimidina-1,7(1,4),4(1,3)-triben zenacyclononaphane-9-one |
| **214** | 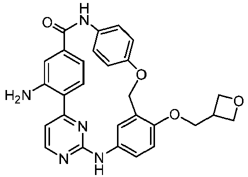 | 496.19/496.30 | 1²-amino-4⁴-(oxetan-3-ylmethoxy)-6-oxa-3, 8-diaza-2(4,2)-pyrimidina-1,7(1,4),4(1,3)-tribenzenacyclonona phane-9-one |

### Example 215 Preparation of 4⁴-(cyclopentoxy)-1²-hydroxy -6-oxa-3, 8-diaza-2 (4,2)-pyrimidina-1,7(1,4),4(1,3)-tribenzenacyclononaphane-9-one (215)

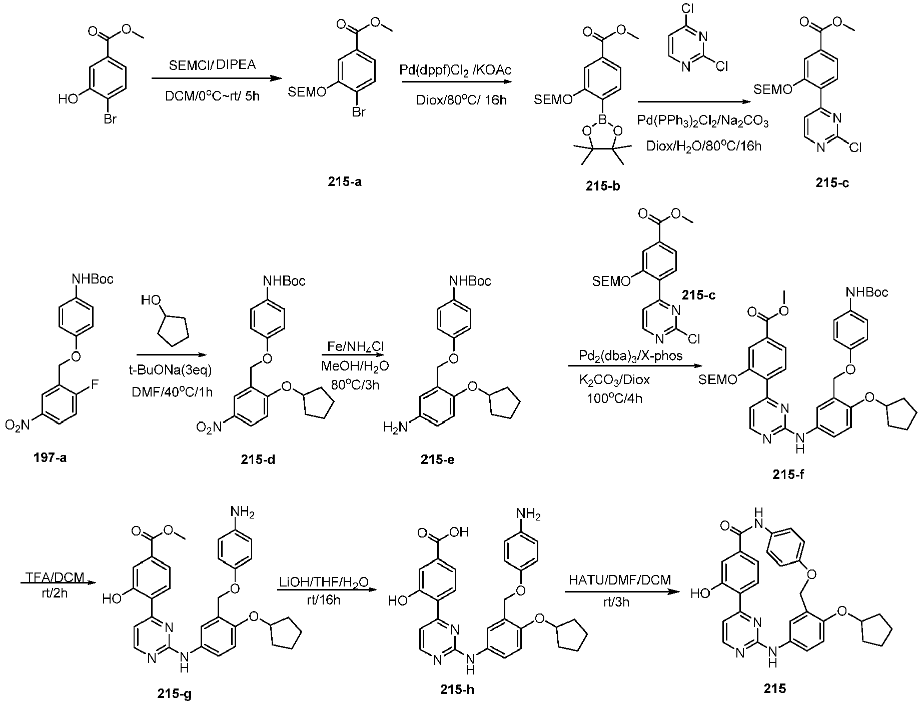

### Step 1: methyl 4-bromo-3-((2-(trimethylsilyl) ethoxy) methoxy) benzoate (215-a)

Under nitrogen protection, methyl 4-bromo-3-hydroxybenzoate (1.00g, 4.35mmol) and 2-(trimethylsilyl) ethoxymethyl chloride (1.45g, 8.70mmol) were added to anhydrous DCM(20ml), cooled to 0°C under ice bath, then stirred for 10min, and N,N-diisopropylethylamine (2.25g,17.39mmol) was slowly added dropwise. The reaction solution was slowly raised to room temperature and continued to be stirred for 5 hours after addition. After TLC confirmed that the reaction was completed, the reaction solution was filtered by suction filtration, the filtrate was concentrated, and the residue was purified by column chromatography to obtain methyl 4-bromo-3-((2-(trimethylsilyl) ethoxy) methoxy) benzoate (**215-a,** 1.52g, yield 97%) as a yellow oil.

### Step 2: methyl 4-(4,4,5, 5-tetramethyl-1, 3, 2-dioxaborolan-2-yl)-3-((2-(trimethylsilyl) ethoxy) methoxy) benzoate (215-b)

Under nitrogen protection, methyl 4-bromo-3-((2-(trimethylsilyl) ethoxy) methoxy) benzoate (**215-a**, 1.52g,4.2mmol), Bis(pinacolato)diboron (1.60g,6.3mmol), 1,1'-bis(diphenylphosphine) ferrocene] palladium dichloride (0.30g,0.42mmol) and potassium acetate (1.24g,12.6mmol) were added to 1, 4-dioxane (20ml), heated to 80°C and stirred for 16h. After TLC confirmed that the reaction was completed, the reaction solution was filtered by suction filtration, the filtrate was concentrated, and the residue was purified by column chromatography to obtain methyl 4-(4,4,5, 5-tetramethyl-1,3, 2-dioxaborolan-2-yl)-3-((2-(trimethylsilyl) ethoxy) methoxy) benzoate (**215-b**, 1.46g, yield 85%) as a colorless oil. ¹H NMR (400 MHz, DMSO-d6): δ 7.65 (d, J = 7.5 Hz, 1H), 7.60 - 7.56 (m, 2H), 5.29 (s, 2H), 3.86 (s, 3H), 3.74 (dd, J = 10.7, 5.6 Hz, 2H), 1.30 (s, 12H), 0.94 - 0.84 (m, 2H), -0.04 (s, 9H).

### Step 3: methyl 4-(2-chloropyrimidin-4-yl)-3-((2-(trimethylsilyl) ethoxy) methoxy) benzoate (215-c)

Under nitrogen protection, 2, 4-dichloropyrimidine (0.46g, 4.30mmol) and methyl 4-(4,4,5, 5-tetramethyl-1, 3, 2-dioxaboran-2-yl)-3-((2-(trimethylsilyl) ethoxy) methoxy) benzoate (**215-b**,1.46g, 3.58mmol) were added to a mixed solvent of 1,4-dioxane and water (20ml, 4ml), and sodium carbonate (0.76g, 7.16mmol) was added, then bis(triphenylphosphine)palladium dichloride (0.50g, 0.72mmol) was added, and the reaction soultion was heated to 80 °C and stirred for 16 hours. After TLC confirmed that the reaction was completed, 1, 4-dioxane was concentrated, the solution was extracted with EA, and the organic phase was washed with deionized water for three times, and dried over anhydrous sodium sulfate. EA was concentrated, and the residue was purified by column chromatography to obtain methyl 4-(2-chloropyrimidin-4-yl)-3-((2-(trimethylsilyl) ethoxy) methoxy) benzoate (**215-c**, 1.21g, yield 86%) as a colorless oil. ¹H NMR (400 MHz, CDCl₃): δ 8.66 (d, J = 5.2 Hz, 1H), 8.12 (d, J = 8.1 Hz, 1H), 7.97 (d, J = 5.2 Hz, 1H), 7.93 (s, 1H), 7.82 (d, J = 8.1 Hz, 1H), 5.39 (s, 2H), 3.96 (s, 3H), 3.76 (t, 2H), 1.01 - 0.92 (m, 2H), 0.01 (s, 9H).

### Step 4: tert-butyl (4-((2-(cyclopentoxy)-5-nitrobenzyl) oxy) phenyl) carbamate (215-d)

Tert-butyl (4-((2-fluoro-5-nitrobenzyl) oxy) phenyl) carbamate (**197-a**, 500mg, 1.38mmol) and cyclopentanol (238mg,2.76mmol) were added to DMF(10ml), stirred for 5 minutes under an ice bath, then sodium tert-butoxide (397mg,4.14mmol) was added. The temperature was slowly returned to room temperature and the reaction solution was stirred for 2 hours, and then heated at 40 °C for 1 hour. LCMS showed that the reaction was completed. Saturated ammonium chloride solution was added to quench the reaction, water and EA were added for extraction, and the organic phase was washed with deionized water for three times, and dried over anhydrous sodium sulfate. EA was concentrated to obtain tert-butyl (4-((2-(cyclopentoxy)-5-nitrobenzyl)oxy) phenyl) carbamate (**215-d**, 586mg, yield 99%) as a yellow oil. MS (ESI) m/z: calcd 429.19 (M+H), found 429.20;¹H NMR (400 MHz, CDCl₃) δ 8.40 (d,J = 2.8 Hz, 1H), 8.20 (dd,J = 8.8, 2.8 Hz, 1H), 7.31-7.26 (m, 2H), 6.97-6.94 (m, 3H), 6.49 (s, 1H), 5.05 (s, 2H), 4.97-4.93 (m, 1H), 2.03-1.88 (m, 4H), 1.86-1.80 (m, 2H), 1.74-1.66 (m, 2H), 1.54 (s, 9H).

### Step 5: tert-butyl (4-((5-amino-2-(cyclopentoxy) benzyl) oxy) phenyl) carbamate (215-e)

Tert-butyl (4-((2-(cyclopentoxy)-5-nitrobenzyl) oxy) phenyl) carbamate (**215-d**, 460mg, 1.074mmol) was added to a mixed solvent of methanol and water (20ml, 5ml), then Fe powder (300mg, 5.37mmol) and ammonium chloride (285mg, 5.37mmol) were added, and the reaction solution was heated and stirred at 80 °C for 3 hours. LCMS showed that the reaction was completed. After the solid was filtered out with diatomite, methanol was spin-dried, extracted with EA, and dried over anhydrous sodium sulfate. EA was concentrated, and the residue was purified by column chromatography to obtain tert-butyl (4-((5-amino-2-(cyclopentoxy) benzyl) oxy) phenyl) carbamate (**215-e**, 400mg, yield 93%) as a yellow oil. MS (ESI) m/z: calcd 399.22 (M + H), found 399.30.

### Step 6: Methyl 4-(2-((3-((4-((tert-butoxycarbonyl) amino) phenoxy) methyl)-4-(cyclopentoxy) phenyl) amino) pyrimidin-4-yl)-3-((2-(trimethylsilyl) ethoxy) methoxy) benzoate (215-1)

Under nitrogen protection, methyl 4-(2-chloropyrimidin-4-yl)-3-((2-(trimethylsilyl) ethoxy) methoxy) benzoate (**215-c**, 238mg, 0.602mmol), and tert-butyl (4-((5-amino-2-(cyclopentoxyl) benzyl) oxy) phenyl) carbamate (**215-e**, 200mg, 0.502mmol) were added to 1,4-dioxane (10mL), then tris(dibenzylideneacetone)dipalladium (46mg, 0.0502mmol), 2-dicyclohexylphosphino-2,4,6-triisopropylbiphenyl (48mg, 0.1004mmol) and potassium carbonate (139mg, 1.004mmol) were added, reacted at 100 °C for 4h. After TLC confirmed that the reaction was completed, 1,4-dioxane was concentrated, and deionized water and dichloromethane were added for liquid separation. The organic phase was washed with deionized water for three times, dried over anhydrous sodium sulfate, dichloromethane was concentrated, and the residue was purified by column chromatography to obtain methyl 4-(2-((3-((4-((tert-butoxycarbonyl) amino) phenoxy) methyl)-4-(cyclopentoxy) phenyl) amino) pyrimidin-4-yl)-3-((2-(trimethylsilyl) ethoxy) methoxy) benzoate (**215-f**, 141mg, yield 37%) as a yellow solid. MS (ESI) m/z: calcd 757.36 (M + H), found 757.30.

### Step 7: methyl 4-(2-((3-((4-aminophenoxy) methyl)-4-(cyclopentoxy) phenyl) amino) pyrimidin-4-yl)-3-hydroxybenzoate (215-g)

Under an ice bath, methyl 4-(2-((3-((4-(tert-butoxycarbonyl) amino) phenoxy) methyl)-4-(cyclopentoxy) phenyl) amino) pyrimidin-4-yl)-3-((2-(trimethylsilyl) ethoxy) methoxy) benzoate (**215-f**, 120mg,0. 158mmol) was added to dichloromethane (4mL), trifluoroacetic acid (1mL) was added, and the reaction was stirred at room temperature for 2 hours. LCMS showed that the reaction was completed. Dichloromethane and trifluoroacetic acid were concentrated to obtain methyl 4-(2-((3-((4-aminophenoxy) methyl)-4-(cyclopentoxy) phenyl) amino) pyrimidin-4-yl)-3-hydroxybenzoate(**215-g**, 82mg, yield 98%) which was directly used in the next reaction. MS (ESI) m/z: calcd 527.22 (M + H), found 527.3.

### Step 8: 4-(2-((3-((4-aminophenoxy) methyl)-4-(cyclopentoxy) phenyl) amino) pyrimidin-4-yl)-3-hydroxybenzoic acid (215-h)

Methyl 4-(2-((3-((4-aminophenoxy) methyl)-4-(cyclopentoxy) phenyl) amino) pyrimidin-4-yl)-3-hydroxybenzoate(**215-g**, 82mg, 0156mmol) was added to tetrahydrofuran and water (5mL, 1mL), and then lithium hydroxide monohydrate (33mg,0.779mmol) was added. The reaction was stirred overnight at room temperature, and LCMS showed that the reaction was completed. Tetrahydrofuran was concentrated, it was purified by medium pressure preparative liquid chromatography and lyophilized to obtain 4-(2-((3-((4-aminophenoxy) methyl)-4-(cyclopentoxy) phenyl) amino) pyrimidin-4-yl)-3-hydroxybenzoic acid (**215-h**, 20mg, yield 25%). MS (ESI) m/z: calcd 513.21 (M + H), found 513.20.

### Step 9: 4⁴-(cyclopentoxy)-1²-hydroxy -6-oxa-3,8-diaza-2 (4,2)-pyrimidina-1,7(1,4),4(1,3)-tribenzenacyclononaphane-9-one (215)

O-(7-Azabenzotriazol-1-yl)-N,N,N',N'-tetramethyluronium hexafluorophosphate (30mg, 0.078mmol) and N,N-diisopropylethylamine (50mg, 0.39mmol) were added to DMF(2ml) and stirred at room temperature. A solution of 4-(2-((3-((4-aminophenoxy) methyl)-4-(cyclopentoxy) phenyl) amino) pyrimidin-4-yl) 3-hydroxybenzoic acid (**215-h**, 20mg, 0.039mmol) in DMF(2ml) was added dropwise slowly and kept for 0.5h, and the reaction solution was continued to be stirred at room temperature for 3h after addition. After LCMS confirmed that the reaction was completed, it was purified by medium pressure preparative liquid chromatography and lyophilized to obtain 4⁴-(cyclopentoxy)-1²-hydroxy-6-oxa-3,8-diaza-2 (4,2)-pyrimidina-1,7(1,4),4(1,3)-tribenzenacyclononaphane-9-one (**215,** 0.75mg, yield 3.8%) as a yellow solid. MS (ESI) m/z: calcd 495.20 (M + H), found 495.20.

The following example compounds of **Table 4** were prepared according to the same method as the above example **215** by using the commercial compound or by referring to the preparation method of the intermediate compound shown.

**Table 4**

| Example | Structure | MS (calc) [M + H]⁺ MS (found) | Name |
|---|---|---|---|
| **216** | 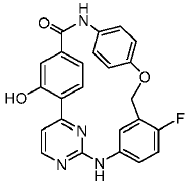 | 429.13/429.10 | 4⁴-fluoro-1²-hydroxy-6-oxa-3,8-dia za-2(4,2)-pyrimidina-1,7(1,4),4(1,3) -tribenzenacyclononaphane-9-one |
| **217** | 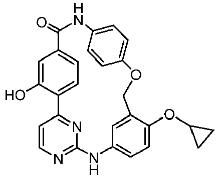 | 467.16/467.20 | 4⁴-cyclopropoxy-1²-hydroxy-6-oxa-3,8-diaza-2(4,2)-pyrimidina-1,7(1,4 ),4(1,3)-tribenzenacyclononaphane-9-one |

### Example 218: Biological Test

### Biological test method

The activity measurement method of JAK kinase was to use homogeneous time-resolved fluorescence technique. The reaction was performed in 384 shallow well plates with a total reaction volume of 10 µL. The mixture of kinase protein, compound, ATP and substrate was carried out in a reaction buffer of 50 mM Hepes (pH7.0),NaN3 0.02%,BSA 0.01%,0. 1mM Orthocanadate, 5 mM MgCl₂, and 1 mM DTT. After reacting for 1 hour, an antibody capable of recognizing substrate phosphorylation, dye XL-615 and detection buffer (Cisbio) containing EDTA were added to the system. The reaction signal of kinase was detected by PE company's porous plate detector. The parameter settings were excitation light at 320 nm, emission light at 615 nm and 665 nm. The activity of JAK is indirectly reflected by the signal ratio of 665 nm and 615 nm. In the reaction, the background well without enzyme and the total enzyme activity well without compound were set.

The IC50 value of the compound inhibiting protein was obtained by the formula: Y = 100/(1+10^((LogIC50-X)^{∗}HillSlope)). In JAK1 reaction system, the concentration of ATP was 2 µM, and the concentration of JAK1 protein was 0.2 ng/µL.

In JAK2 reaction system, the concentration of ATP was 2 µM, and the concentration of JAK2 protein was 0.01 ng/µL.

In JAK3 reaction system, the concentration of ATP was 2 µM, and the concentration of JAK3 protein was 0.04 ng/µL.

In TYK2 reaction system, the concentration of ATP was 2 µM, and the concentration of TYK2 protein was 0.2 ng/µL.

The test data are divided into the following types: A: IC₅₀ <10 nM; B: IC₅₀ 11-100 nM; C: IC₅₀ 101-1000 nM; D: IC₅₀ 1001-10000 nM; E: IC₅₀ > 10000 nM.

The results are shown in Table 4.

**Table 4**

| Table of JAK inhibitory activity | | | | | | | | | |
|---|---|---|---|---|---|---|---|---|---|
| Compound number | JAK 1 activity | JAK 2 activity | JAK 3 activity | TYK 2 activity | Compound number | JAK 1 activity | JAK 2 activity | JAK 3 activity | TYK 2 activity |
| **1** | B | B | A | C | **2** | C | C | C | C |
| **3** | C | C | C | E | **4** | D | C | B | C |
| **5** | B | B | B | B | **6** | C | B | B | C |
| **7** | C | B | B | C | **8** | C | B | B | C |
| **9** | B | A | A | B | **10** | B | A | A | B |
| **11** | D | C | C | D | **12** | C | C | B | D |
| **13** | E | E | E | E | **14** | C | B | B | C |
| **15** | C | C | B | D | **16** | B | B | B | D |
| **17** | D | C | C | D | **18** | D | C | C | E |
| **19** | D | D | D | E | **20** | C | C | B | D |
| **21** | D | C | C | D | **22** | D | C | C | D |
| **23** | D | C | B | D | **24** | D | D | C | D |
| **25** | D | D | C | D | **26** | C | B | B | C |
| **27** | C | B | B | C | **28** | C | B | B | C |
| **29** | C | B | B | D | **30** | C | B | B | C |
| **31** | C | B | A | C | **32** | C | A | B | C |
| **33** | D | D | D | E | **34** | C | C | B | D |
| **35** | E | E | D | E | **36** | E | E | D | E |
| **37** | E | E | D | E | **38** | C | C | C | D |
| **39** | D | D | C | E | **40** | D | D | C | E |
| **41** | D | C | C | D | **42** | D | C | B | C |
| **43** | D | D | C | E | **44** | D | C | B | D |
| **45** | D | D | D | E | **46** | D | C | B | D |
| **47** | D | D | B | D | **48** | D | D | C | D |
| **49** | D | C | D | D | **50** | C | B | A | B |
| **51** | D | D | C | E | **52** | C | B | A | C |
| **53** | D | E | D | E | **54** | C | C | B | D |
| **55** | C | B | A | C | **56** | E | D | D | E |
| **57** | D | C | B | C | **58** | B | B | B | C |
| **59** | D | D | C | E | **60** | C | C | C | D |
| **61** | D | D | C | E | **62** | C | D | C | D |
| **63** | C | B | A | D | **64** | D | C | C | E |
| **65** | D | D | D | D | **66** | C | B | B | C |
| **67** | C | C | A | D | **68** | D | D | C | E |
| **69** | D | C | B | D | **70** | D | C | B | D |
| **71** | E | E | D | E | **72** | D | D | C | E |
| **73** | D | D | C | E | **74** | D | C | A | D |
| **75** | D | C | B | D | **76** | D | C | C | E |
| **77** | C | B | B | C | **78** | D | C | B | E |
| **79** | C | C | B | D | **80** | D | D | D | E |
| **81** | D | D | C | E | **82** | D | D | D | E |
| **83** | D | D | E | E | **84** | C | C | B | D |
| **85** | C | C | B | E | **86** | D | D | C | E |
| **87** | E | E | C | E | **88** | C | B | A | D |
| **89** | D | D | D | E | **90** | C | C | B | E |
| **91** | E | E | C | E | **92** | D | C | B | D |
| **93** | D | D | C | E | **94** | D | D | B | E |
| **95** | D | B | A | D | **96** | D | D | B | E |
| **97** | D | D | B | D | **98** | C | C | B | D |
| **99** | E | D | C | E | **100** | C | C | A | D |
| **101** | E | D | D | E | **102** | D | D | B | E |
| **103** | E | D | C | E | **104** | E | D | C | E |
| **105** | D | C | A | E | **106** | E | D | C | E |
| **107** | D | C | B | E | **108** | D | D | C | E |
| **109** | D | C | B | D | **110** | D | C | A | D |
| **111** | D | D | C | E | **112** | D | C | A | E |
| **113** | D | D | C | E | **114** | C | B | A | D |
| **115** | D | D | C | E | **116** | C | C | A | D |
| **117** | D | E | C | E | **118** | D | D | B | E |
| **119** | D | D | B | E | **120** | D | D | C | E |
| **121** | D | C | A | D | **122** | E | E | C | E |
| **123** | D | D | B | D | **124** | D | D | C | E |
| **125** | C | C | A | D | **126** | E | D | C | E |
| **127** | D | C | A | D | **128** | E | E | C | E |
| **129** | D | D | C | E | **130** | D | C | B | E |
| **131** | D | C | B | E | **132** | D | C | A | E |
| **133** | E | E | D | E | **134** | D | D | B | D |
| **135** | D | D | C | E | **136** | D | C | A | D |
| **137** | D | D | C | E | **138** | D | C | A | D |
| **139** | D | D | C | E | **140** | D | C | A | D |
| **141** | E | E | D | E | **142** | D | D | B | E |
| **143** | E | E | C | E | **144** | E | E | D | E |
| **145** | C | C | A | D | **146** | E | D | C | E |
| **147** | C | C | A | D | **148** | D | C | A | E |
| **149** | D | D | B | E | **150** | E | E | D | E |
| **151** | D | C | B | D | **152** | D | D | C | E |
| **153** | D | C | A | E | **154** | D | D | C | E |
| **155** | D | C | B | D | **156** | D | C | B | E |
| **157** | C | B | A | C | **158** | D | E | C | E |
| **159** | E | E | D | E | **160** | D | C | B | D |
| **161** | D | D | D | E | **162** | D | C | C | D |
| **163** | D | D | D | E | **164** | D | C | B | D |
| **165** | C | B | A | C | **166** | D | D | C | E |
| **167** | C | C | A | C | **168** | E | D | D | E |
| **169** | D | C | B | D | **170** | D | D | C | D |
| **171** | D | D | D | E | **172** | D | C | B | D |
| **173** | D | C | B | D | **174** | D | D | D | E |
| **175** | D | C | B | D | **176** | E | E | D | E |
| **177** | D | D | C | E | **178** | C | B | A | C |
| **179** | D | D | C | E | **180** | D | C | B | D |
| **181** | C | B | A | C | **182** | D | C | A | D |
| **183** | D | C | A | D | **184** | D | C | A | D |
| **185** | D | C | B | D | **186** | D | C | A | D |
| **187** | C | B | B | C | **188** | D | C | B | D |
| **189** | C | C | B | E | **190** | D | C | B | D |
| **191** | D | D | C | D | **192** | D | D | C | E |
| **193** | D | D | B | D | **194** | D | C | B | E |
| **195** | D | D | B | D | **196** | D | C | A | D |
| **197** | D | C | B | D | **198** | D | C | B | D |
| **199** | C | D | B | D | **200** | D | C | B | E |
| **201** | E | E | D | E | **202** | D | D | B | E |
| **203** | D | D | C | E | **204** | D | E | D | E |
| **205** | D | D | B | E | **206** | D | C | A | E |
| **207** | C | C | B | D | **208** | E | D | B | E |
| **209** | E | D | D | E | **210** | E | E | B | E |
| **211** | C | C | B | D | **212** | D | D | D | E |
| **213** | D | D | B | E | **214** | D | C | B | D |
| **215** | D | C | B | D | **216** | C | C | B | D |
| **217** | C | C | B | D | | | | | |

### Discussion:

The above experimental results suggest that
(1) The compound of formula I of the present invention exhibits JAK3 inhibitory activity, and has good selectivity to JAK1, JAK2 and TYK2. The IC50 value of the compound of the present invention can be as low as 10nM or less, so that for subjects weighting about 70kg (such as patients, especially patients with rheumatoid arthritis or psoriasis), daily doses of 10mg to 30mg can be extremely effective in inhibiting JAK, especially JAK3.
(2) The compound of formula I of the present invention exhibits very excellent JAK selectivity, i .e. the IC50 ratio of JAK1/JAK3 and/or the IC50 ratio of JAK2/JAK3 are superior to the currently marketed drugs.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula I or a stereoisomer or an optical isomer, a pharmaceutically acceptable salt, a prodrug or a solvate thereof, 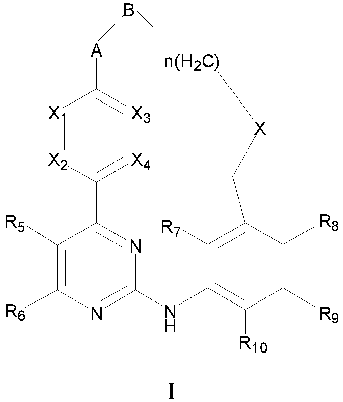 wherein,
X₁, X₂, X₃ and X₄ are each independently selected from the group consisting of N, C and C-R_{d}; and 0, 1, 2 and 3 of X₁, X₂, X₃ and X₄ are N;
or, when X₄ is C-R_{d}, R_{d} is fused with X₂ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or, when X₂ is C-R_{d}, R_{d} is fused with X₁ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or, when X₃ is C-R_{d}, R_{d} is fused with X₄ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or, when X₄ is C-R_{d}, R_{d} is fused with X₃ to form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
A is selected from the group consisting of -C(= O)N-R_{b}, -C(= O)O-, -SO₂N(R_{b})-, -C(= O)N-R_{b}-SO₂-, O and S; wherein R_{b} is selected from the group consisting of H, and substituted or unsubstituted C1-C6 alkyl;
B is selected from the substituted or unsubstituted group consisting of bond, C(R_{c})₂, C3-C10 cycloalkylene, 3-10-membered heterocyclylene, 5-12-membered heteroarylene, C6-C12 arylene, and (CH₂)m-R'-; R_{c} is each independently selected from the group consisting of H, halogen, amino, nitro, hydroxyl, cyano, carboxyl, sulfonyl, sulfinyl, amido, sulfonamido, ester group, C1-C6 alkyl, C1-C6 alkoxy, 3-10 membered heterocycloalkyl, C3-C10 cycloalkyl, 5-12 membered heteroaryl, and C6-C12 aryl; R' is independently selected from the substituted or unsubstituted group consisting of 5-12 membered heteroarylene, and C6-C12 arylene;
X is selected from the substituted or unsubstituted group consisting of bond, C(R_{c})₂, (CH₂)m-O-, C(= O)O-, O, N-R_{b}, S, SO, SO₂, C3-C10 cycloalkylene, 3-10-membered heterocyclylene, 5-12-membered heteroarylene, and C6-C12 arylene;
R₅, R₆, R₇, R₈, R₉, R₁₀ and R_{d} are each independently selected from the group consisting of H, D, halogen, amino, amine, nitro, hydroxyl, sulfydryl, cyano, carboxyl, sulfonyl, sulfinyl, amido, sulfonamido, ester group, formyl, formamido, C1-C6 alkyl, C2-C6 alkenyl, C2-C6 alkynyl, 3-10-membered heterocyclyl, C3-C10 cycloalkyl, 5-12-membered heteroaryl, C6-C12 aryl and -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
or R₅ and R₆ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₈ and R₉ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₉ and R₁₀ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
the H atom in (CH₂)n and (CH₂)m can be optionally substituted by one or more Rₐ;
m is 1, 2, 3, 4 or 5;
n is 0, 1, 2, 3, 4, 5, 6, 7 or 8;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

2. The compound of formula I or the stereoisomer or optical isomer, pharmaceutically acceptable salt, prodrug or solvate thereof of claim 1, wherein the compound has a structure shown in formula I': 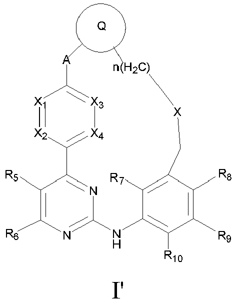 wherein,
Q is selected from the substituted or unsubstituted group consisting of (CH₂)m-R'-, C3-C10 cycloalkylene, 3-10-membered heterocyclylene, 5-12-membered heteroarylene, and C6-C12 arylene; wherein R' is independently selected from the substituted or unsubstituted group consisting of 5-12 membered heteroarylene, and C6-C12 arylene;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
X, m, n, A, X₁, X₂, X₃, X₄, R₅, R₆, R₇, R₈, R₉, and R₁₀ are as defined in claim 1.

3. The compound of formula I or the stereoisomer or optical isomer, pharmaceutically acceptable salt, prodrug or solvate thereof of claim 1, wherein the compound has a structure shown in formula II: 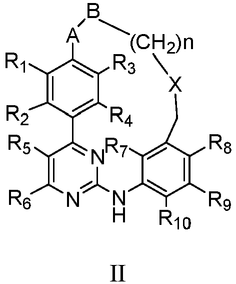 wherein,
R₁, R₂, R₃, R₄, R₅, R₆, R₇, R₈, R₉ and R₁₀ are each independently selected from the group consisting of H, D, halogen, amino, amine, nitro, hydroxyl, sulfydryl, cyano, carboxyl, sulfonyl, sulfinyl, amido, sulfonamido, ester group, formyl, formamido, C1-C6 alkyl, C2-C6 alkenyl, C2-C6 alkynyl, 3-10-membered heterocyclyl, C3-C10 cycloalkyl, 5-12-membered heteroaryl, C6-C12 aryl and -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
or R₁ and R₂ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₃ and R₄ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₅ and R₆ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₈ and R₉ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
or R₉ and R₁₀ together with the C atoms to which they are attached form substituted or unsubstituted 5-6-membered cycloalkyl, substituted or unsubstituted 5-6-membered heterocyclyl, substituted or unsubstituted 5-6-membered aryl or substituted or unsubstituted 5-6-membered heteroaryl;
the H atom in (CH₂)m and (CH₂)n can be optionally substituted by one or more Rₐ;
A, B, m, n and X are defined as in claim 1;
the "substituted" refers to being substituted by one or more groups selected from the group consisting of D, halogen, amino, amine, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

4. The compound of formula I or the stereoisomer or optical isomer, pharmaceutically acceptable salt, prodrug or solvate thereof of claim 1, wherein the compound has a structure shown in formula III: 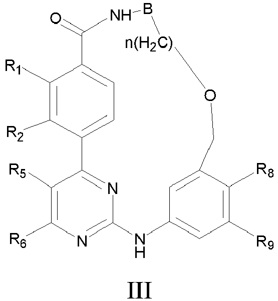 wherein,
B is selected from the substituted or unsubstituted group consisting of bond, (CH₂)m-R'-, C3-C10 cycloalkylene, 3-10-membered heterocyclylene, 5-12-membered heteroarylene, and C6-C12 arylene; wherein R' is independently selected from the substituted or unsubstituted group consisting of 5-12 membered heteroarylene, and C6-C12 arylene;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
m, n, R₅, R₆, R₈, and R₉ are defined as in claim 1, and R₁ and R₂ are as defined in claim 3.

5. The compound of formula I or the stereoisomer or optical isomer, pharmaceutically acceptable salt, prodrug or solvate thereof of claim 1, wherein the compound has a structure shown in formula IV 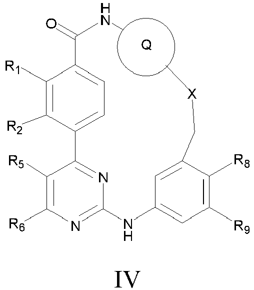 wherein,
Q is selected from the substituted or unsubstituted group consisting of (CH₂)m-R'-, 5-12 membered heteroarylene and C6-C12 arylene; wherein R' is independently selected from the substituted or unsubstituted group consisting of 5-12 membered heteroarylene, and C6-C12 arylene; the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
X, m, R₁, R₂, R₅, R₆, R₈, and R₉ are as defined in claim 1.

6. The compound of formula I or the stereoisomer or optical isomer, pharmaceutically acceptable salt, prodrug or solvate thereof of claim 1, wherein the compound has a structure shown in formula V 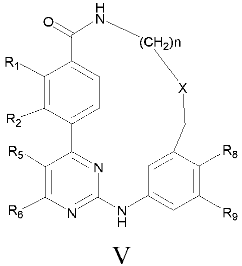 wherein, R₅, R₆, R₈, R₉, X and n are as defined in claim 1; R₁ and R₂ are as defined in claim 3.

7. The compound of formula I or the stereoisomer or optical isomer, pharmaceutically acceptable salt, prodrug or solvate thereof of claim 1, wherein the compound has a structure shown in formula VI 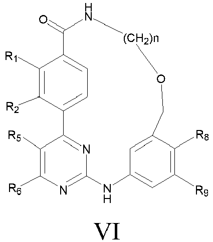
wherein, R₁ and R₂ are each independently selected from H, D, halogen, amino, amine, nitro, hydroxyl, sulfydryl, cyano, carboxyl, sulfonyl, sulfinyl, amido, sulfonamido, ester group, formyl, formamido, C1-C6 alkyl, C2-C6 alkenyl, C2-C6 alkynyl, 3-10-membered heterocyclyl, C3-C10 cycloalkyl, 5-12-membered heteroaryl, C6-C12 aryl or -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl; the alkyl, alkoxy, alkenyl, alkynyl, heterocyclyl, cycloalkyl, heteroaryl and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.
R₅, R₆, R₈, R₉ and n are as defined in claim 1.

8. The compound of formula I or the stereoisomer or optical isomer, pharmaceutically acceptable salt, prodrug or solvate thereof of claim 1, wherein the compound has a structure shown in formula VII 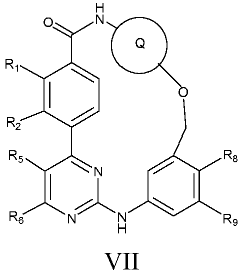 wherein,
Q is selected from the substituted or unsubstituted group consisting of (CH₂)m-R'-, C3-C10 cycloalkylene, 3-10-membered heterocyclylene, 5-12-membered heteroarylene, and C6-C12 arylene; wherein R' is independently selected from the substituted or unsubstituted group consisting of 5-12 membered heteroarylene, and C6-C12 arylene;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl; the alkyl, alkoxy, alkenyl, alkynyl, heterocyclyl, cycloalkyl, heteroaryl and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
m, R₅, R₆, R₈, and R₉ are as defined in claim 1.

9. The compound of formula I or the stereoisomer or optical isomer, pharmaceutically acceptable salt, prodrug or solvate thereof of claim 1, wherein the compound has a structure shown in formula VIII 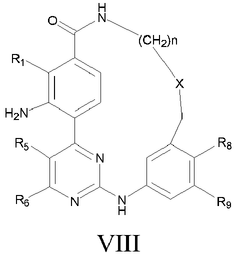
wherein, R₁ is selected from H, D, halogen, amino, amine, nitro, hydroxyl, sulfydryl, cyano, carboxyl, sulfonyl, sulfinyl, amido, sulfonamido, ester group, formyl, formamido, C1-C6 alkyl, C2-C6 alkenyl, C2-C6 alkynyl, 3-10-membered heterocyclyl, C3-C10 cycloalkyl, 5-12-membered heteroaryl, C6-C12 aryl or -OR₁₁; wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl; the alkyl, alkoxy, alkenyl, alkynyl, heterocyclyl, cycloalkyl, heteroaryl and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.
R₅, R₆, R₈, R₉, X and n are as defined in claim 1.

10. The compound or the stereoisomer or optical isomer, pharmaceutically acceptable salt, prodrug or solvate thereof of any one of claims 1-9, wherein, R₈ is selected from the group consisting of H, halogen, amino, amine, C1-C6 alkyl, C2-C6 alkenyl, C2-C6 alkynyl, 3-10 membered heterocyclyl, C3-C10 cycloalkyl, 5-12 membered heteroaryl, C6-C12 aryl and -OR₁₁;
wherein R₁₁ is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, hydroxyl, cyano, amido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
the alkyl, alkenyl, alkynyl, heterocyclyl, cycloalkyl, heteroaryl and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

11. The compound or the stereoisomer or optical isomer, pharmaceutically acceptable salt, prodrug or solvate thereof of any one of claims 1-10, wherein, R₈ is selected from the group consisting of C1-C6 alkyl, C3-C10 cycloalkyl, 3-10 membered heterocyclyl, and -OR₁₁;
R₁₁ is selected from the substituted or unsubstituted group consisting of C1-C6 alkyl, 3-10 membered heterocyclyl, and C3-C10 cycloalkyl;
the "substituted" refers to being substituted by one or more groups selected from the group consisting of D, halogen, amino, amine, hydroxyl, cyano, amido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
the alkyl, alkenyl, alkynyl, heterocyclyl, cycloalkyl, heteroaryl and aryl may be further optionally substituted by one or more Rₐ, wherein each Rₐ is independently selected from the group consisting of halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl.

12. The compound or the stereoisomer or optical isomer, pharmaceutically acceptable salt, prodrug or solvate thereof of claim 1, wherein the compound is selected from the group consisting of

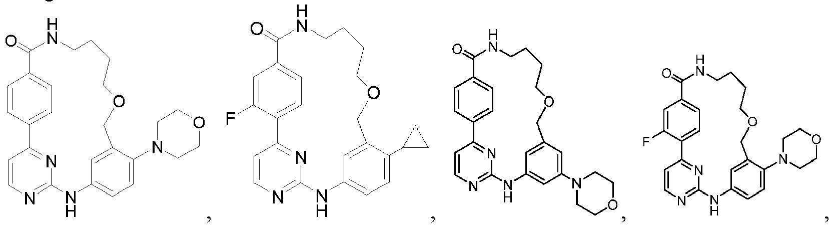

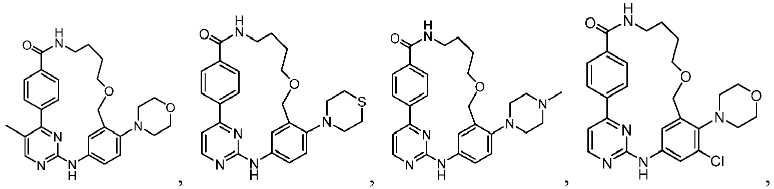

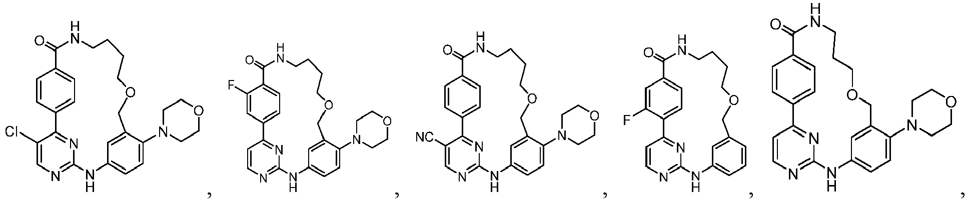

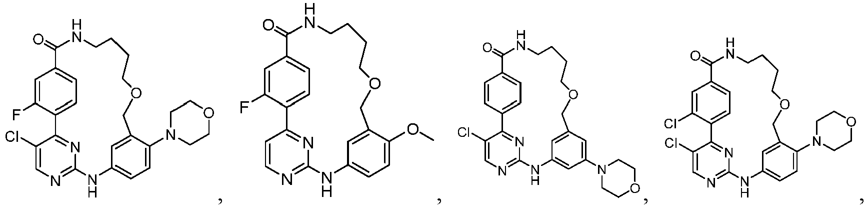

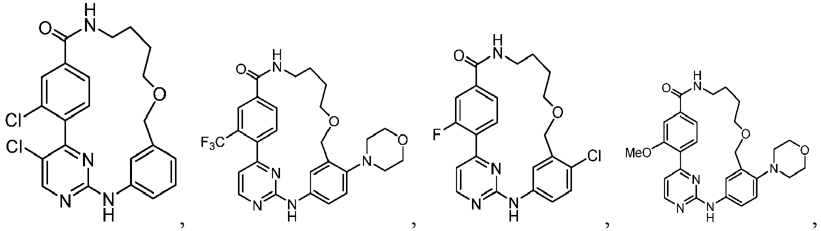

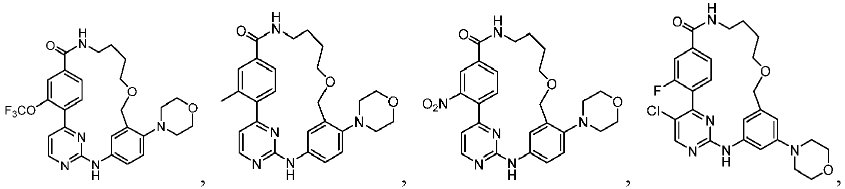

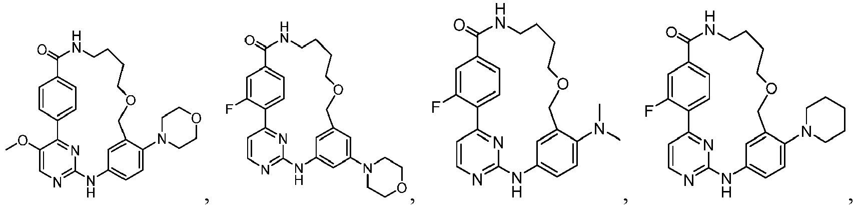

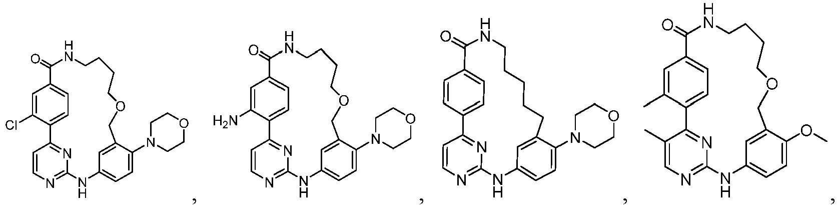

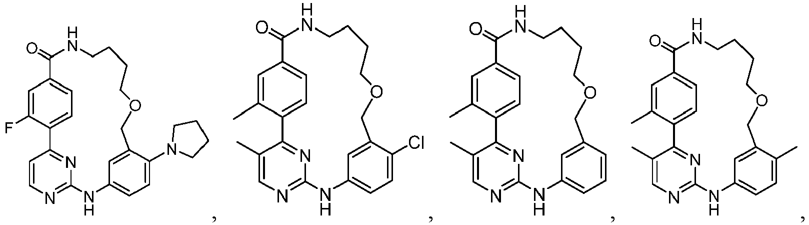

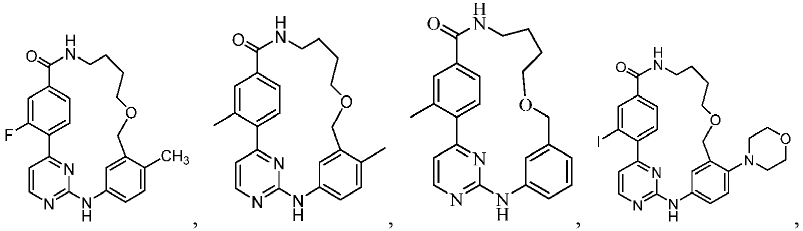

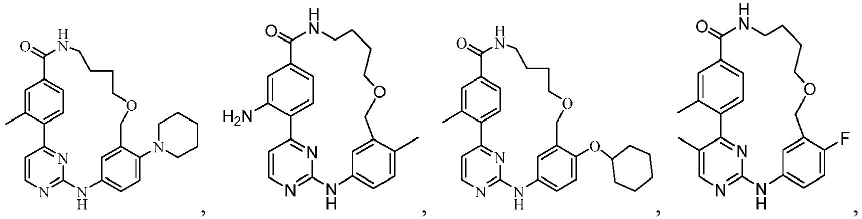

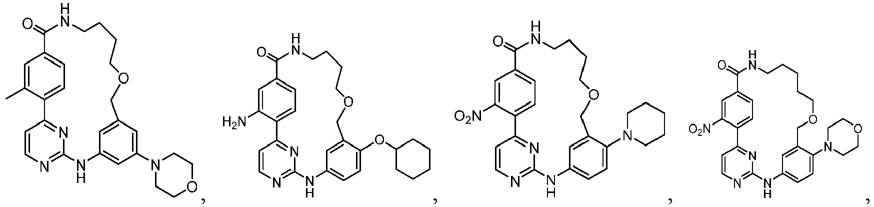

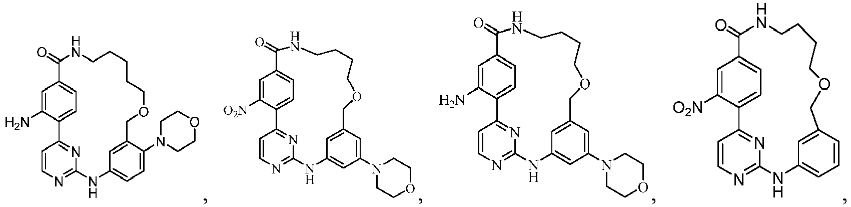

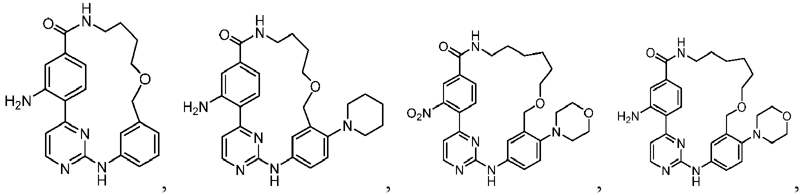

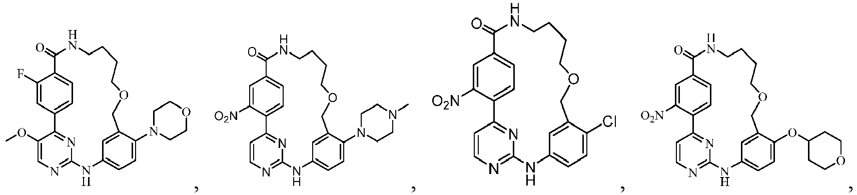

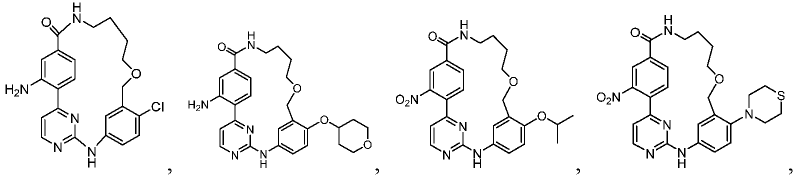

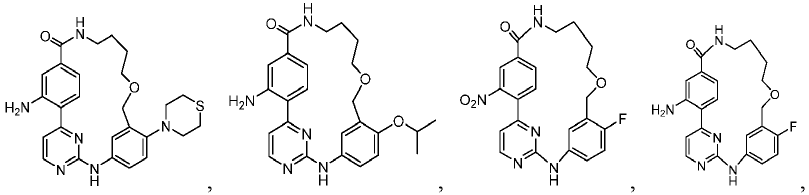

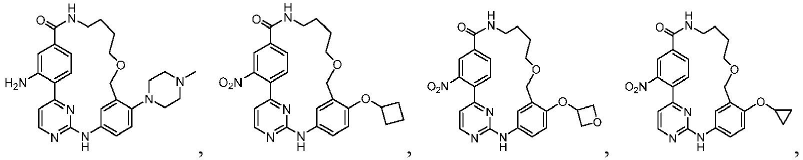

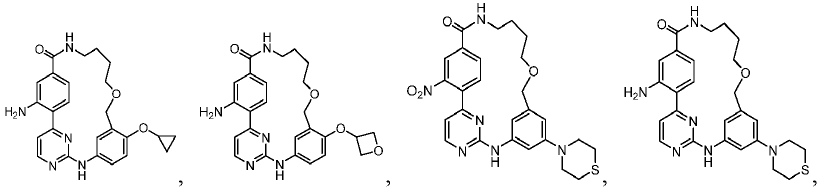

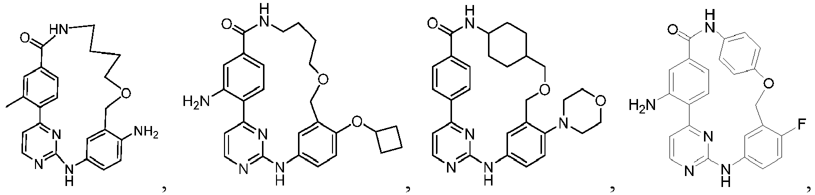

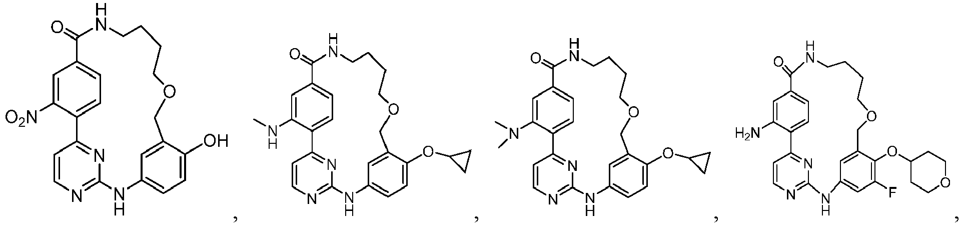

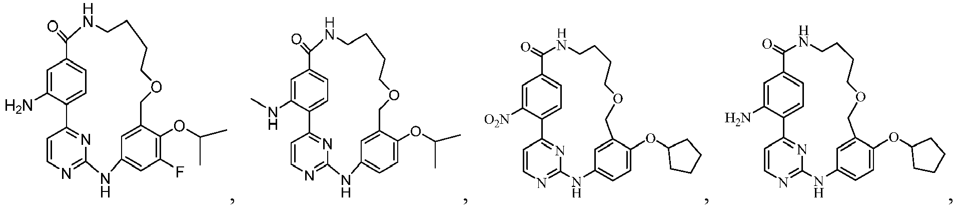

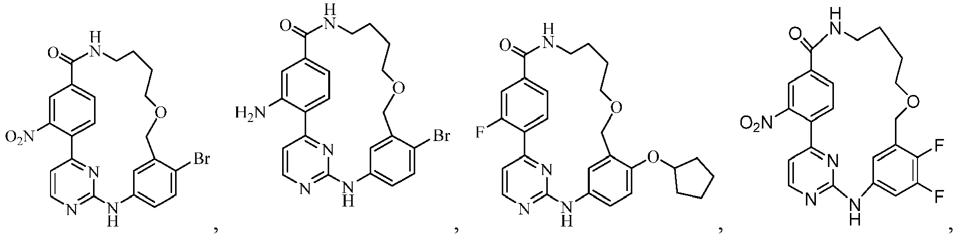

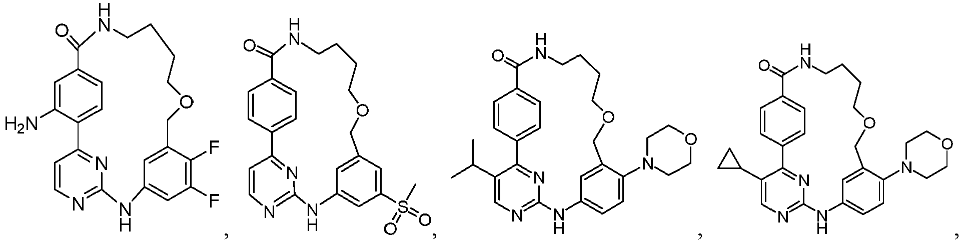

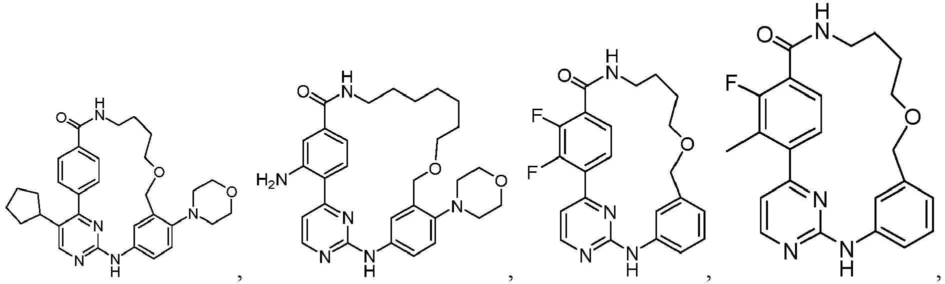

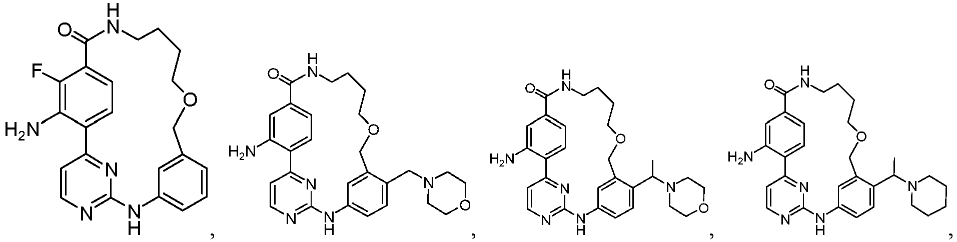

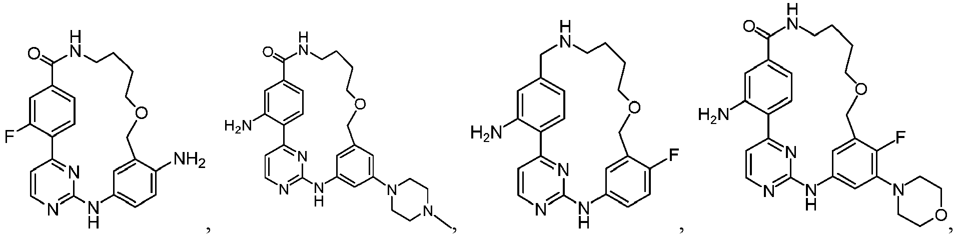

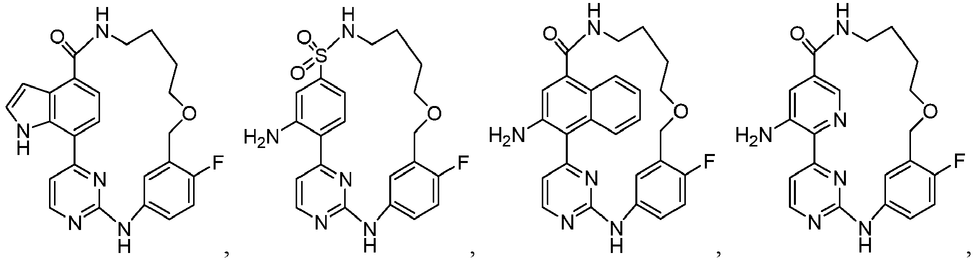

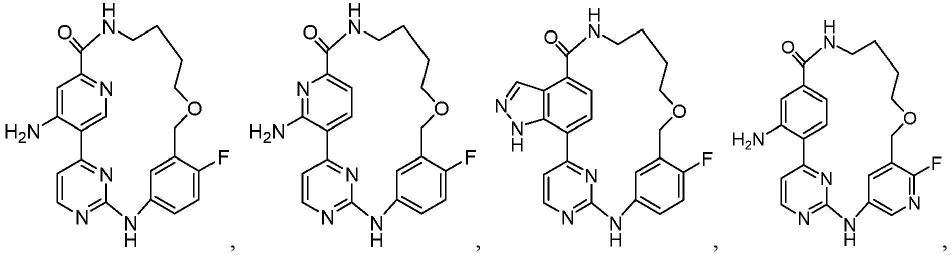

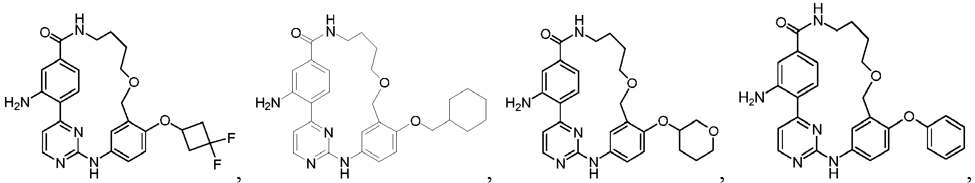

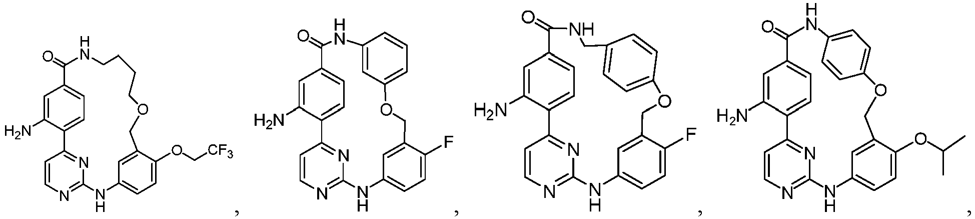

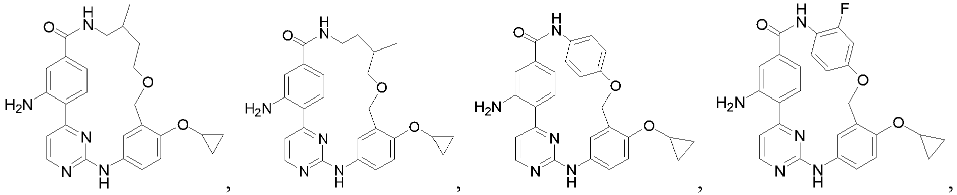

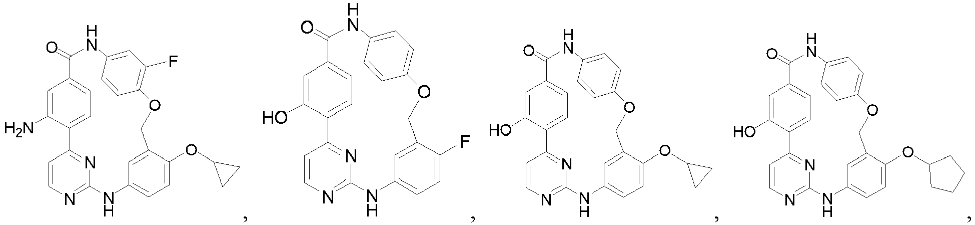

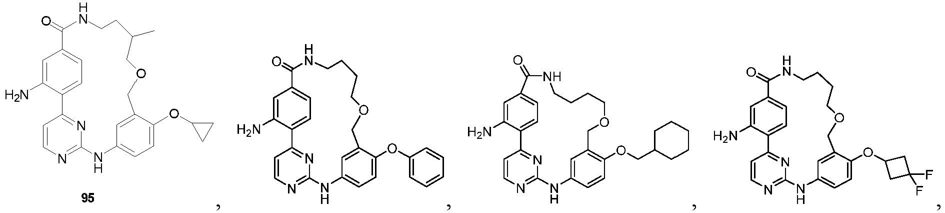

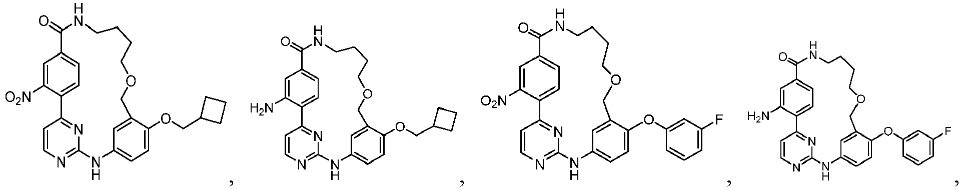

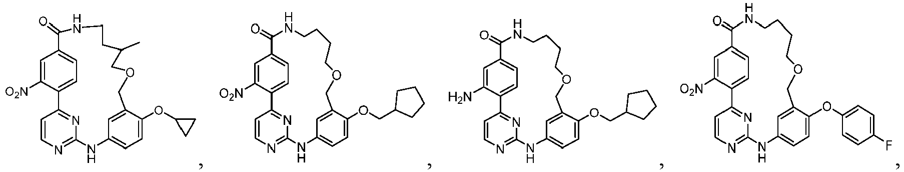

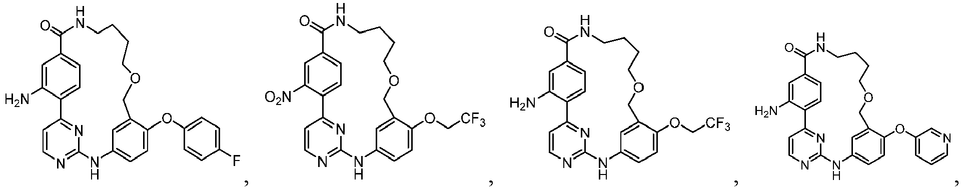

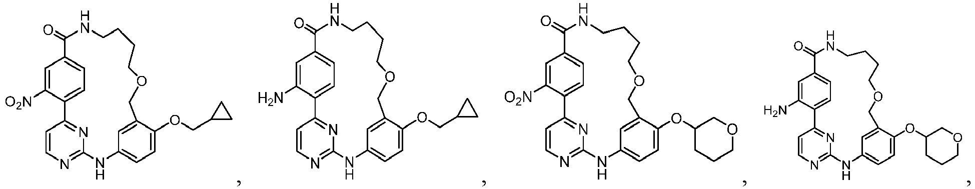

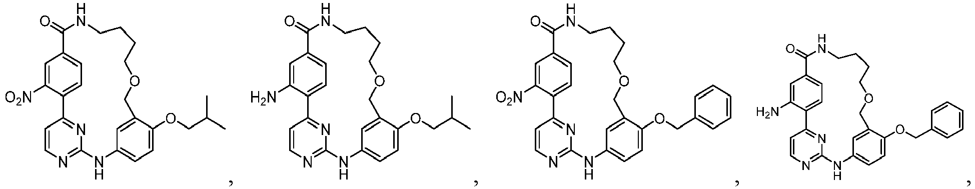

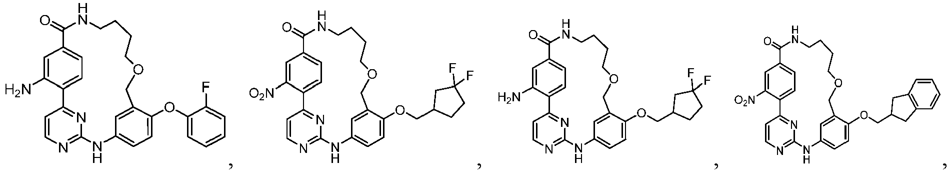

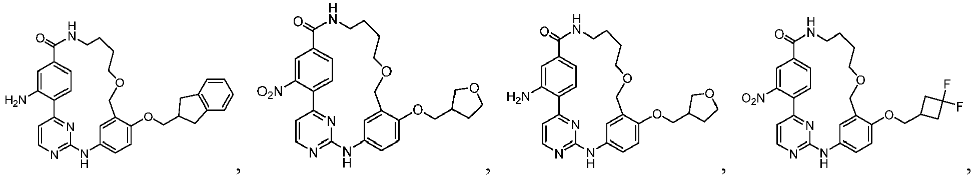

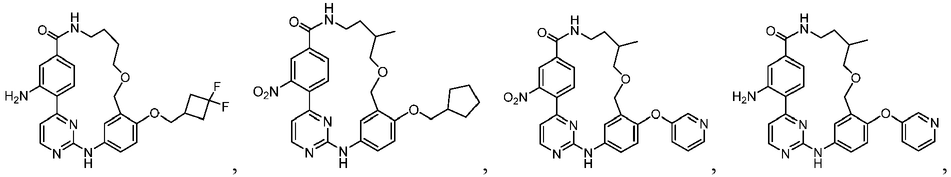

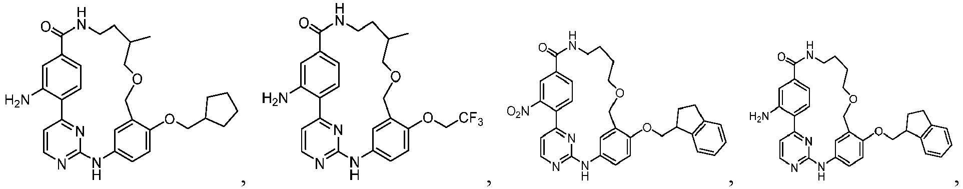

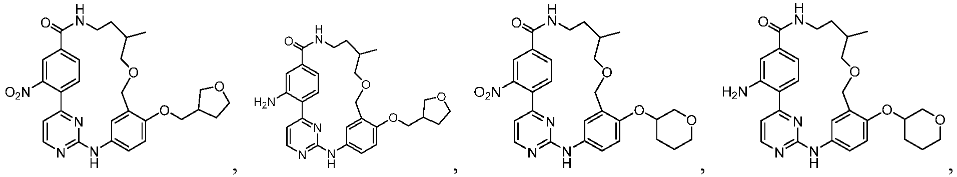

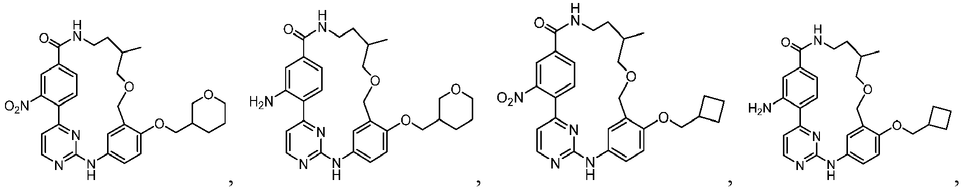

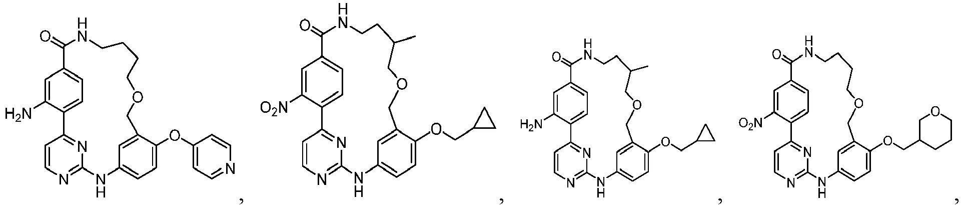

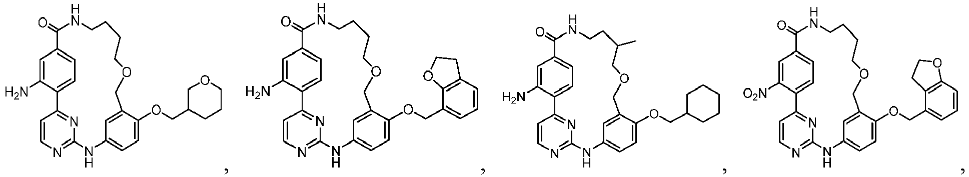

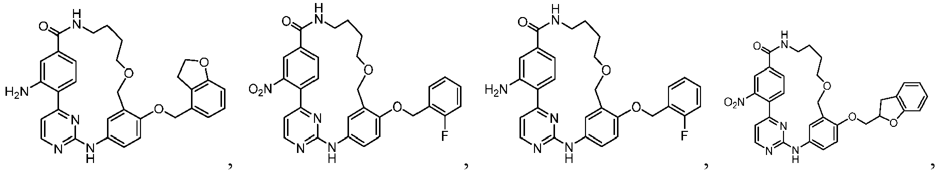

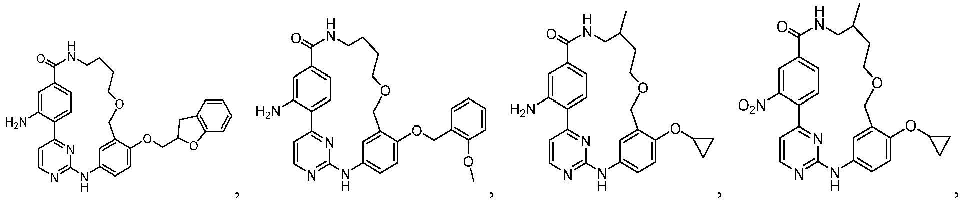

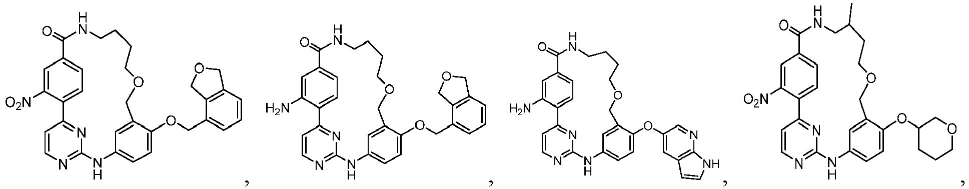

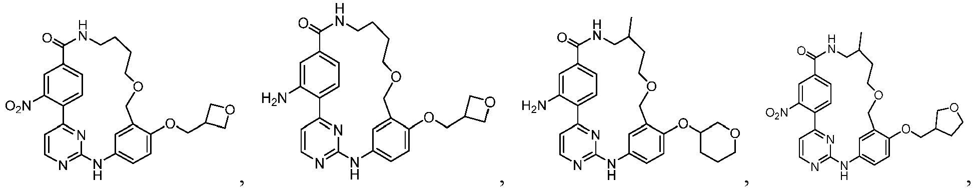

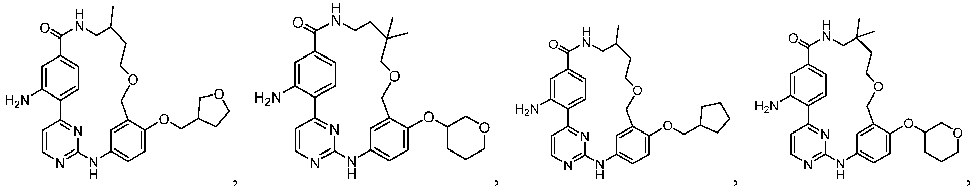

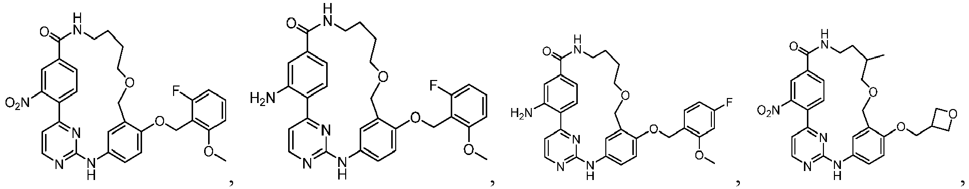

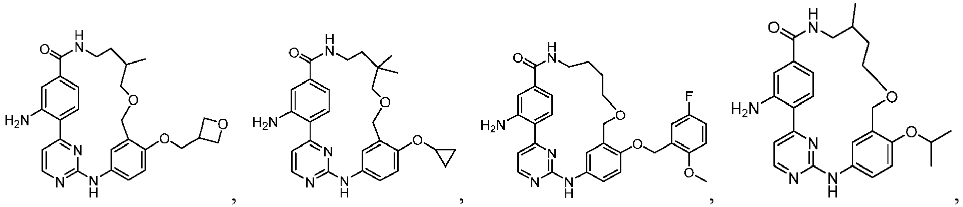

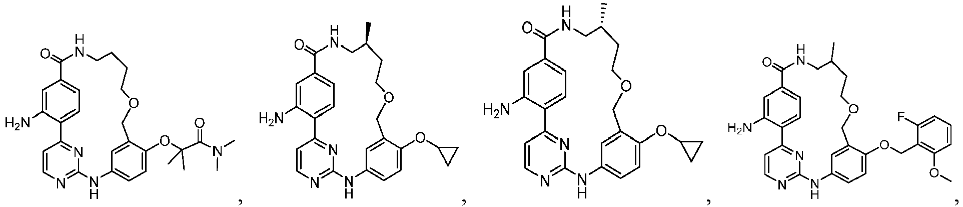

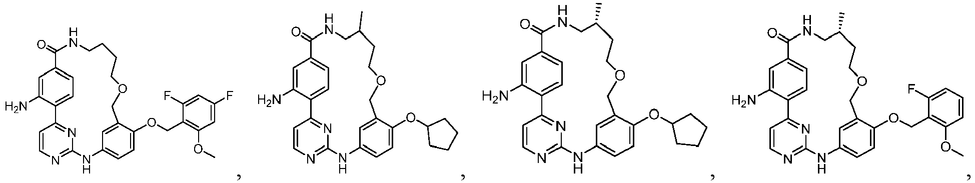

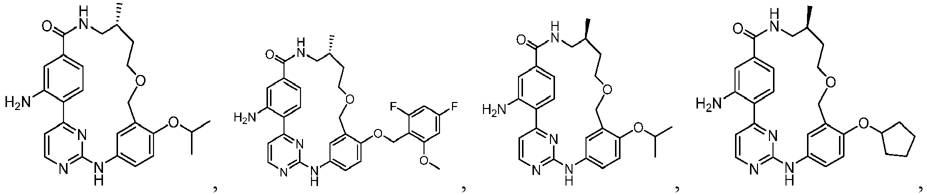

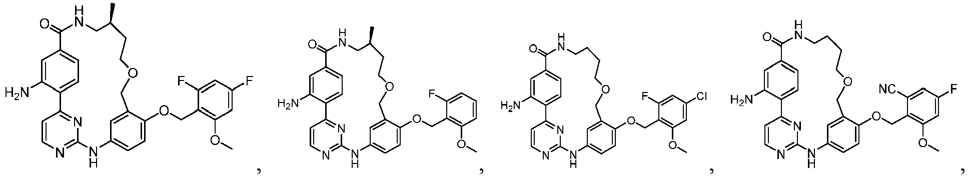

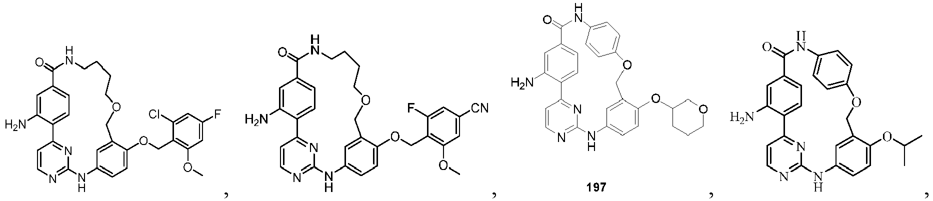

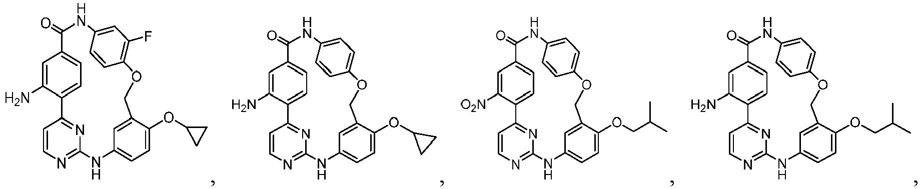

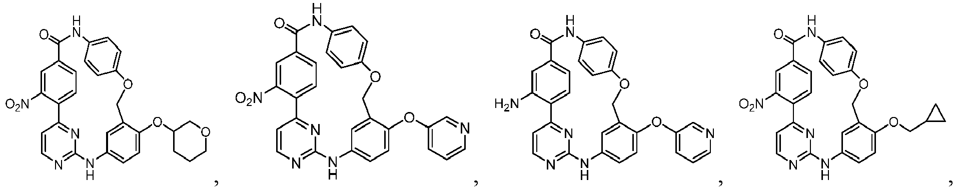

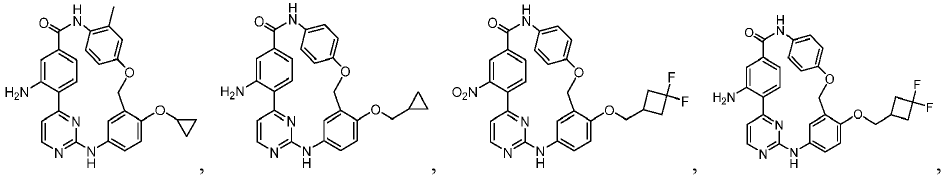

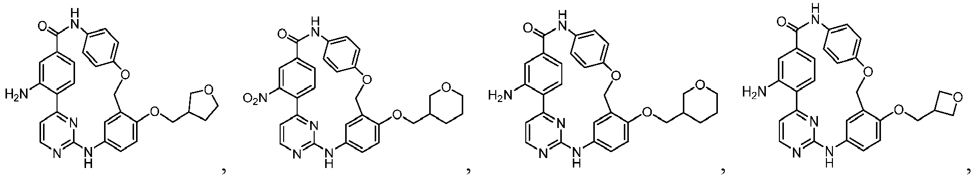

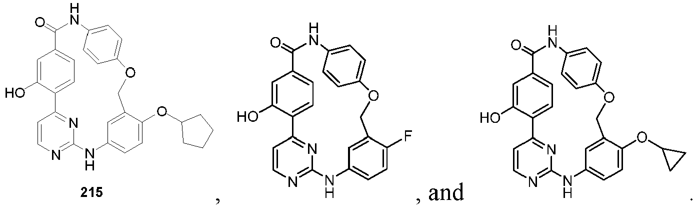

13. A pharmaceutical composition comprising the compound, or the stereoisomer or optical isomer, pharmaceutically acceptable salt, prodrug or solvate thereof of any one of claims 1-12; and a pharmaceutically acceptable carrier.

14. Use of the compound or the stereoisomer or optical isomer, pharmaceutically acceptable salt, prodrug or solvate thereof of any one of claims 1-12 or the pharmaceutical composition of claim 13 for preparing a medicament or a pharmaceutical composition for treating or preventing a disease related to the activity or expression of JAK kinase.

15. A preparation method for the compound or the stereoisomer or optical isomer, pharmaceutically acceptable salt, prodrug or solvate thereof of claim 1 comprising the following step:
in an inert solvent, compound A₈ undergoes ring-forming reaction in the presence of a catalyst to obtain the compound of formula I; 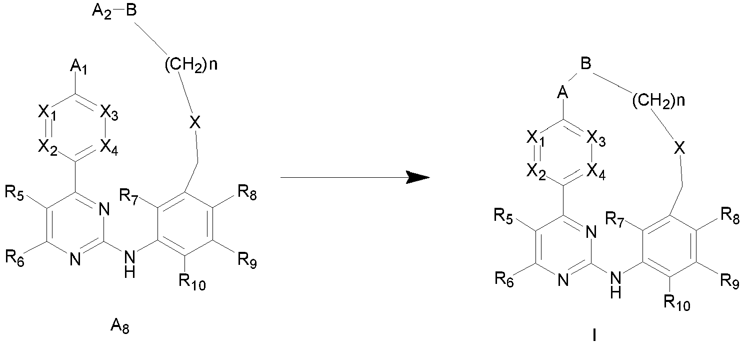 wherein,
A₁ is selected from the group consisting of carboxyl, sulfonic group, CO-O-R", and -CO-NH-R"; wherein, R" is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
A₂ is selected from the group consisting of amino and hydroxyl;
A, B, X₁, X₂, X₃, X₄, R₅, R₆, R₇, R₈, R₉, R₁₀, X and n are as defined in claim 1.
